# EUROPEAN PATENT SPECIFICATION

(11) **EP 2 863 906 B1**
(45) Date of publication and mention of the grant of the patent: **18.03.2020**
(21) Application number: 13807102.2
(22) Date of filing: 21.06.2013
(51) Int. Cl.: A61K 31/295, A61P 7/06, A61P 13/12, A61K 9/28

(54) **USE OF FERRIC CITRATE IN THE TREATMENT OF CHRONIC KIDNEY DISEASE PATIENTS**
VERWENDUNG VON EISENCITRAT BEI DER BEHANDLUNG VON PATIENTEN MIT CHRONISCHEN NIERENERKRANKUNGEN
UTILISATION DE CITRATE FERRIQUE POUR TRAITER LES PATIENTS ATTEINTS D'UNE MALADIE RÉNALE CHRONIQUE

(30) Priority: 21.06.2012 US 201261662565 P; 28.01.2013 US 201361757229 P; 15.03.2013 US 201361801050 P; 15.03.2013 US 201361800618 P
(43) Date of publication of application: 29.04.2015
(73) Proprietor: Keryx Biopharmaceuticals, Inc., Cambridge, MA 02142 (US)
(72) Inventor: PORADOSU, Enrique, Brookline, Massachusetts 02446 (US); BENTSUR, Ron, Tenafly, New Jersey 07670 (US); OLIVIERO III, James F., New York, NY 10013 (US)
(74) Representative: Jones Day
(86) International application number: PCT/US2013/047134
(87) International publication number: WO 2013/192565

(56) References cited:
- WO-A1-98/26776
- WO-A1-2011/011541
- US-A1- 2009 186 939
- YOKOYAMA KEITARO ET AL: "Effect of oral JTT-751 (ferric citrate) on hyperphosphatemia in hemodialysis patients: results of a randomized, double-blind, placebo-controlled trial.", AMERICAN JOURNAL OF NEPHROLOGY 2012, vol. 36, no. 5, 2012, pages 478-487, XP008176600, ISSN: 1421-9670
- Wu-Chang Yang: "An open-label, crossover study of a new phosphate-binding agent in haemodialysis patients: ferric citrate", Nephrol. Dial. Transplant., 1 January 2002 (2002-01-01), pages 265-270, XP055023585, Retrieved from the Internet: URL:http://ndt.oxfordjournals.org/content/ 17/2/265.full.pdf#page=1&view=FitH [retrieved on 2012-04-02]
- M. SIKA ET AL.: "A Dose-Ranging And Efficacy Phase 3 Trial Of Ferric Citrate (FC) As A Phosphate Binder In Dialysis Patients", AM. J. KIDNEY DIS., vol. 57, no. 4, 2011, page A89, XP002740671,
- C. BOND ET AL.: "A Meta-Aanalysis Of Ferric Citrate For Hyperphosphatemia: The Effect odf an Oral Iron-Containing Phospahte Binder on Serum Ferritin and Saturated Transferrin in Hemodialysis Patients", NEPHROLOGY DIALYSIS TRANSPLANTATION, vol. 27, no. S2, May 2012 (2012-05), page ii490, XP002740672,
- T. CHRISTOPHER ET AL.: "Ferric Citrate: an Iron-Based Oral Phosphate Binder", RIDNEY RES. CLIN. PRACT., vol. 31, 20 June 2012 (2012-06-20), page A38, XP002740673,
- K. UMANATH ET AL.: "Rationale and study design of a three-period, 58-week trial of ferric citrate as a phosphate binder in patients with ESDR on dialysis", HEMODIALYSIS INTERNATIONAL, vol. 17, 15 June 2012 (2012-06-15), pages 67-74, XP002740674,
- WANG LI ET AL: "PUB324: The effects of oral vs venous iron supplement in treatment of iron deficiency of maintained patients with anemia", JOURNAL OF THE AMERICAN SOCIETY OF NEPHROL; MEETING OF THE AMERICAN SOCIETY OF NEPHROLOGY RENAL WEEK, WILLIAMS AND WILKINS, BALTIMORE, MD, US; SAN DIEGO, CA, USA, vol. 14, no. Abstracts Issue, 1 November 2003 (2003-11-01), pages 842A-843A, XP008176607, ISSN: 1046-6673

## Description

### FIELD

Methods and compositions disclosed herein relate generally to the use of ferric citrate to treat chronic kidney disease (CKD) patients.

### BACKGROUND

Chronic kidney disease (CKD) is a gradual and progressive loss of the ability of the kidneys to excrete wastes, concentrate urine, and conserve electrolytes. The U.S. National Kidney Foundation defines chronic kidney disease according to the presence or absence of kidney damage and the level of kidney function, regardless of the type (clinical diagnosis) of kidney disease. The primary measure of kidney function is glomerular filtration rate (GFR), which is often estimated as creatinine clearance from serum and urine creatinine concentrations. Chronic kidney disease or failure is defined as having a GFR less than 60 ml/min for three months or more. The U.S. National Kidney Foundation has suggested a five stage classification of renal dysfunction based on GFR:

| **Stages of renal dysfunction (adapted from National Kidney Foundation-K/DOQI)** | | | |
|---|---|---|---|
| **Stage** | **Description** | **Creatinine Clearance (∼GFR: ml/min/1.73 m²)** | **Metabolic consequences** |
| 1 | Normal or increased GFR-People at increased risk or with early renal damage | >90 | - |
| 2 | Early renal insufficiency | 60-89 | Concentration of parathyroid hormone starts to rise (GFR∼60-80) |
| 3 | Moderate renal failure (chronic renal failure) | 30-59 | Decrease in calcium absorption (GFR<50) |
| | | | Lipoprotein activity falls |
| | | | Malnutrition |
| | | | Onset of left ventricular hypertrophy |
| | | | Onset of anemia |
| 4 | Severe renal failure | 15-29 | Triglyceride concentrations start to rise |
| | | | Hyperphosphatemia |
| | | | Metabolic acidosis Tendency to hyperkalemia |
| 5 | End stage renal disease (Uremia) | <15 | Azotaemia develops |

As indicated in the table above, stage 1 is the least severe and stage 5, or ESRD, the most severe. In the early stages of CKD, *e.g*. stages 1-4, dialysis is typically not required. Therefore, patients experiencing the earlier stages of CKD are described as having non-dialysis dependent chronic kidney disease. Such patients are also commonly referred to as non-dialysis chronic kidney disease (ND-CKD) patients. Anemia typically first appears in CKD Stage 3 when the GFR is less than 60 cc/min, long before dialysis is necessary, although anemia may appear at any stage of CKD. At stage 5, a patient may require dialysis treatment several times per week. Once the degeneration process of the kidney begins, the kidney functions in CKD deteriorate irreversibly toward end stage renal disease (ESRD, stage 5). Patients suffering from ESRD cannot survive without dialysis or kidney transplantation.

According to the U.S. National Kidney Foundation, approximately 26 million American adults have CKD and millions of others are at increased risk. Patients experiencing the earlier stages of CKD typically incur increased medical costs of U.S. $14,000 to U.S. $22,000 per patient per year, compared to the age-matched, non-CKD general population. However, there is growing evidence that some of the increased costs and adverse outcomes associated with CKD can be prevented or delayed by preventive measures, early detection, and early treatment.

Iron deficiency and anemia are common complications of CKD, including ESRD. Anemia is the clinical manifestation of a decrease in circulating red blood cell mass and usually is detected by low blood hemoglobin concentration. The properly functioning kidney produces erythropoietin, a hormone that stimulates proliferation and differentiation of red blood cell precursors, which ultimately leads to erythropoiesis (red blood cell production). In the CKD kidney, erythropoietin production is often impaired, leading to erythropoietin deficiency and the concomitant deficiency in erythropoiesis. Anemia is associated with adverse cardiovascular outcomes, ESRD, mortality and diminished quality of life (Macdougall, Curr Med Res Opin (2010) 26:473-482). The prevalence of anemia in CKD increases as kidney function decreases. Approximately 50% of non-dialysis chronic kidney disease patients are anemic, and by the time CKD patients start dialysis, up to 70% are anemic (Macdougall, *supra,* and McClellan et al., Curr Med Res Opin (2004) 20:1501-1510).

Iron deficiency is a significant contributor to anemia in CKD patients. The estimated prevalence ranges from 25 to 70% (Hsu, et al., J Am Soc Nephrol (2002) 13: 2783-2786; Gotloib et al., J Nephrol (2006) 19: 161-167; Mafra, et al., J Ren Nutr (2002) 12: 38-41; Kalantar-Zadeh, et al., Am J Kidney Dis (1995) 26: 292-299; and Post, et al., Int Urol Nephrol (2006) 38: 719-723). The causes include decreased intake or absorption of iron, iron sequestration as a result of inflammation, blood loss, and increased iron use for red blood cell production in response to erythropoiesis stimulating agents (ESAs) (Fishbane, et al., Am J Kidney Dis (1997) 29: 319-333; Kooistra, et al., Nephrol Dial Transplant (1998) 13: 82-88; and Akmal, et al., Clin Nephrol (1994) 42: 198-202). Depending on CKD stage, 20-70% of CKD patients exhibit low iron indices (Quinbi et al., Nephrol Dial Transplant (2011) 26:1599-1607). More than 1 million CKD stage 3 or 4 patients in the U.S. are estimated to suffer from iron deficiency. The presence of either low iron stores ("absolute" iron deficiency) or inadequate iron available to meet the demand for erythropoiesis ("functional" iron deficiency) correlates significantly with reduced hemoglobin levels in CKD patients. Iron deficiency can arise from any one or more factors including, for example, insufficient iron from food intake, increased iron utilization, poor gastrointestinal iron absorption, and generalized malabsorption due to renal failure and bacterial overgrowth, and gastrointestinal bleeding (Macdougall, *supra*).

The current standard of care for anemia and/or iron deficiency in CKD patients is administration of erythropoiesis-stimulating agents (ESAs) and/or iron supplementation. The National Kidney Foundation Kidney Disease Outcomes Quality Initiative guidelines recommend either oral or intravenous iron for patients who have CKD stages 1 to 5 and are not on dialysis (*see* "Using iron agents: KDOQI clinical practice guidelines and clinical practice recommendations for anemia in chronic kidney disease," Am J Kidney Dis (2006) 47: S58-S70). The ferric form of iron (also known as iron(III) or Fe³⁺) has long been known to have poor bioavailability when administered orally. Therefore, oral formulations for iron supplementation in CKD patients typically contain the ferrous form of iron (also known as iron(II) or Fe²⁺). Several ferrous oral iron preparations are available for treatment including ferrous gluconate, ferrous fumarate, and ferrous sulfate. The most common oral iron supplement is ferrous sulfate, which can be given up to three times daily in order to provide an adequate dose for treating iron-deficient CKD patients. However, in some CKD patients, oral iron is poorly tolerated because of adverse side effects, or is ineffective in maintaining adequate body stores of iron. Side effects typically include gastrointestinal problems, such as diarrhea, nausea, bloating and abdominal discomfort. Additionally, because of the frequency in which they are typically given, oral ferrous forms pose a tablet burden on patients and have significant negative gastrointestinal side effects, which lead to non-compliance with oral treatment regimens (Mehdi *et al., supra*).

An alternative is to administer intravenous iron to CKD patients. Some studies have shown that intravenous iron formulations are more effective than either oral ferric iron supplements or oral ferrous iron supplements for treating iron deficiency and/or anemia in CKD patients (Mehdi *et al., supra*). Effective intravenous formulations for the treatment of CKD patients include ferric carboxymaltose, ferumoxytol, ferric gluconate, iron sucrose, and iron dextran.

Wang Li et al. (Journal of American Society of Nephrol; Meeting of the American Society of nephrology renal week, Williams and Wilkins, Baltimore, MD, US; San Diego, CA, USA, vol. 14, no. Abstracts Issue, 1 November 2003, pages 842A-843A) describe that venous iron supplement (ferric citrate) acts more effectively than oral iron supplement (ferrous sulfate) in correcting renal anemia, while erythropoietin is used at the same time.

However, intravenous iron is associated with short-term risks such as anaphylaxis and death, as well as with long-term toxicity, including the development of atherosclerosis, infection, and increased mortality (Quinibi Arzneimittelforschung (2010) 60:399-412). Further, many CKD clinics, particularly community sites, are ill-equipped to administer intravenous iron because they lack the infrastructure of a dialysis center. This has left a majority of CKD iron-deficient patients without intravenous iron treatment.

Wu-Chang Yang et al. (Nephrol. Dial. Transplant., 1 January 2002, pages 265-270) describe an open-label, crossover study of ferric citrate as a phosphate-binding agent for the treatment for hyperphosphatemia in hemodialysis patients that shows that ferric citrate lowers the serum phosphate concentration in hemodialysis patients.
Yokoyama et al. (American Journal of Nephrology 2012, vol. 36, no. 5, 2012, pages 478-487) describe a phase II clinical study of ferric citrate hydrate that demonstrated efficacy and safety at doses between 1.5 g and 6 g daily as a phosphate-binding drug for treating hyperphosphatemia in hemodialysis patients.
Christopher et al. (Kidney Res. Clin. Pract., vol. 31, 20 June 2012 (2012-06-20), page A38) describe the construction of a cost offset model suggesting that there would be cost savings with ferric citrate use through reduced use of erythropoiesis-stimulating agents (ESAs) and of iron.

Thus, there is need to develop improved methods for treatment of CKD patients.

### SUMMARY

Certain aspects of the disclosure provide clinically safe and effective phosphate binders that can be used to reduce and/or control serum phosphorus levels, increase serum bicarbonate levels, improve one or more iron storage parameters (e.g., increase serum ferritin levels, increase transferrin saturation (TSAT), increase hemoglobin concentration) increase iron absorption, maintain iron stores, treat iron deficiency, treat anemia, reduce the need for IV iron and/or reduce the need for erythropoiesis-stimulating agents (ESAs) in CKD patients, including non-dialysis CKD (ND-CKD) patients and end state renal disease (ESRD) patients. In certain aspects, the phosphate binder is clinically safe and effective for long term administration to CKD patients, for example up to and including at least 56 weeks of continuous administration.

In accordance with certain embodiments of the disclosure, a candidate for administrative marketing approval as a phosphate binder is the ferric citrate disclosed herein (also known as KRX-0502 (ferric citrate), see Example 1). Pre-clinical studies have demonstrated the ability of the ferric citrate disclosed herein to bind dietary phosphorus, to decrease intestinal absorption of dietary phosphorus and to reduce serum phosphate levels (Mathew, et al., J Am Soc Nephrol (2006) 17: 357A; Voormolen, et al., Nephrol Dial Transplant (2007) 22: 2909-2916; and Tonelli et al., Circulation (2005) 112: 2627-2633). Four clinical studies of the ferric citrate disclosed herein (*e.g*., KRX-0502 (ferric citrate)) in patients with ESRD have been conducted and reported to the U.S. Food and Drug Administration as part of the KRX-0502 (ferric citrate) Investigational New Drug (IND) submission. One of those studies, a Phase 3 long term study (described herein), has confirmed that the ferric citrate disclosed herein (also known as KRX-0502) demonstrates a highly statistically significant change in serum phosphorus versus placebo over a four-week Efficacy Assessment Period and can increase ferritin and transferrin saturation (TSAT) and reduce the use of intravenous iron and erythropoiesis-stimulating agents in ESRD patients when compared to active control agents over a 52-week Safety Assessment Period.

In accordance with the present disclosure, it has been discovered that the ferric citrate disclosed herein can be used as a clinically safe and effective phosphate binder to control and/or reduce serum phosphorus levels, increase serum bicarbonate levels, improve one or more iron storage parameters (e.g., increase serum ferritin levels, increase transferrin saturation (TSAT), increase hemoglobin concentration, increase iron absorption), maintain iron stores, treat iron deficiency, treat anemia, reduce the need for IV iron and/or reduce the need for erythropoiesis-stimulating agents (ESAs) in CKD patients, including non-dialysis CKD (ND-CKD) patients and end state renal disease (ESRD) patients.
Provided herein is a ferric citrate tablet for use in a method for increasing or maintaining the hemoglobin concentration in a non-dialysis chronic kidney disease (CKD) human patient with anemia, wherein the method comprises orally administering one or more tablets to the patient per day, wherein each tablet comprises 1 g of ferric citrate.
In some embodiments, the administration of the one or more ferric citrate tablets results in a mean increase in hemoglobin concentration of greater than 0.4 g/dL in the patient. In some embodiments, the need for intravenous iron supplementation or the need for erythropoiesis-stimulating agents is reduced in the patient.
In some embodiments, one to twelve ferric citrate tablets are administered to the patient per day.
In some embodiments, each tablet comprises approximately 80% to approximately 90% by weight of ferric citrate, approximately 70% to approximately 92% by weight of ferric citrate, approximately 80% to approximately 92% by weight of ferric citrate, approximately 85% to approximately 92% by weight of ferric citrate, or approximately 90% to approximately 92% by weight of ferric citrate. In some embodiments, each tablet comprises approximately 4.5% to approximately 30% by weight of pregelatinized starch. In some embodiments, each tablet comprises approximately 0.5% to approximately 3% by weight of a lubricant. In a preferred embodiment, the lubricant is calcium stearate. In some embodiments, the water content measured by LOD % is less than 10% water w/w.
In some embodiments, at least 80% of the ferric citrate is dissolved in less than or equal to 60 minutes as measured by test method USP <711>.
In some embodiments, each tablet consists of
a) a core consisting of 80.0 to 90.0 % w/w ferric citrate, 8.0 to 15.0 % w/w pregelatinized starch, and 1.0 to 3.0 % w/w calcium stearate; and
b) a coating.
In some embodiments, the administration of the one or more ferric citrate tablets per day results in a mean increase in hemoglobin concentration of greater than 0.5 g/dL or of 0.4 to 1 g/dL.
In some embodiments, the ferric citrate is a complex of iron(III) and citric acid with a molar ratio of iron (III) to citric acid from 1:0.69 to 1:0.87.
In some embodiments, the anemia is iron deficiency anemia.

In a one aspect, the present disclosure provides ferric citrate for use in methods of reducing and/or controlling serum phosphorus in a patient in need thereof. The methods comprise orally administering ferric citrate to a CKD patient, e.g., an end-stage renal disease patient, at a dose of ferric iron ranging from 210 mg - 2,520 mg, wherein the ferric citrate provides a mean reduction in serum phosphorus of 2.00 - 2.50 mg/dl. The ferric citrate can be administered in a 1 gram tablet dosage form, each dosage form comprising 210 mg of ferric iron. The patient is administered up to 18 tablet dosage forms per day. In some examples, the patient is administered 6 tablet dosage forms per day. The ferric citrate may be administered within 1 hour of the ingestion of a meal or snack by the patient. In some examples, the patient was treated with thrice-weekly hemodialysis or with peritoneal dialysis for at least 3 months prior to administration of the ferric citrate. The ferric citrate has a BET active surface area greater than about 16 m²/g. In some examples, the BET active surface area ranges from about 16 m²/g to about 20 m²/g. In some examples, the BET active surface area ranges from about 27.99 m²/g to about 32.34 m²/g. In some examples, the BET active surface area is selected from 27.99 m²/g, 28.87 m²/g and 32.34 m²/g.

The ferric citrate can have an intrinsic dissolution rate of 1.88 - 4.0 mg/cm²/min.

In another aspect, the present disclosure provides ferric citrate for use in methods of reducing serum phosphorus in a patient in need thereof. The methods comprise orally administering ferric citrate to a CKD patient, e.g., an end-stage renal disease patient, at a dose of ferric iron ranging from 210 mg - 2,520 mg, wherein the ferric citrate provides: a mean reduction in serum phosphorus selected from 1.90, 1.91, 1.92, 1.93, 1.94, 1.95, 1.96, 1.97, 1.98, 1.99, 2.00, 2.01, 2.02, 2.03, 2.04, 2.05, 2.06, 2.07, 2.08, 2.09 and 2.10 mg/dl when administered for a period of 12 weeks; a mean reduction in serum phosphorus selected from 2.10, 2.11, 2.12, 2.13, 2.14, 2.15, 2.16, 2.17, 2.18, 2.19, 2.20, 2.21, 2.22, 2.23, 2.24 and 2.25 mg/dl when administered for a period of 24 weeks; a mean reduction in serum phosphorus selected from 2.10, 2.11, 2.12, 2.13, 2.14, 2.15, 2.16, 2.17, 2.18, 2.19 and 2.20 mg/dl when administered for a period of 36 weeks; a mean reduction in serum phosphorus selected from 1.95, 1.96, 1.97, 1.98, 1.99, 2.00, 2.01, 2.02, 2.03, 2.04, 2.05, 2.06, 2.07, 2.08, 2.09, 2.10, 2.11, 2.12, 2.13, 2.14 and 2.15 mg/dl when administered for a period of 48 weeks; and a mean reduction in serum phosphorus selected from 1.95, 1.96, 1.97, 1.98, 1.99, 2.00, 2.01, 2.02, 2.03, 2.04, 2.05, 2.06, 2.07, 2.08, 2.09, 2.10, 2.11, 2.12, 2.13, 2.14, 2.15, 2.16, 2.17, 2.18, 2.19, 2.20, 2.21, 2.22, 2.23, 2.24, 2.25, 2.26, 2.27, 2.28, 2.29 and 2.30 mg/dl when administered for a period of 52 weeks. The ferric citrate provides a mean reduction in serum phosphorus of 2.00 mg/dl when administered for a period of 12 weeks. The ferric citrate provides a mean reduction in serum phosphorus of 2.20 mg/dl when administered for a period of 24 weeks.

The ferric citrate provides a mean reduction in serum phosphorus of 2.20 mg/dl when administered for a period of 36 weeks. The ferric citrate provides a mean reduction in serum phosphorus of 2.10 mg/dl when administered for a period of 48 weeks. The ferric citrate provides a mean reduction in serum phosphorus of 2.10 mg/dl when administered for a period of 52 weeks.

In yet another aspect, the present disclosure provides ferric citrate for use in methods of increasing serum bicarbonate in a patient in need thereof. The methods comprise orally administering ferric citrate to a CKD patient, e.g., an end-stage renal disease patient, at a dose of ferric iron ranging from 210 mg - 2,520 mg, wherein the ferric citrate provides an increase in serum bicarbonate selected from 0.70, 0.71, 0.72, 0.73, 0.74, 0.75, 0.76, 0.77, 0.78, 0.79 and 0.80 mEq/L when administered for a period of at least 52 weeks. The ferric citrate provides a mean increase in serum bicarbonate concentration of 0.71 mEq/L.

The ferric citrate can be administered in a 1 gram tablet dosage form, each dosage form comprising 210 mg of ferric iron. The patient is administered up to 18 tablet dosage forms per day. In some examples, the patient is administered 6 tablet dosage forms per day. The ferric citrate is administered within 1 hour of the ingestion of a meal or snack by the patient. In some examples, the patient was treated with thrice-weekly hemodialysis or with peritoneal dialysis for at least 3 months prior to administration of the ferric citrate. The ferric citrate has a BET active surface area greater than about 16 m²/g. In some examples, the BET active surface area ranges from about 16 m²/g to about 20 m²/g. In some examples, the BET active surface area ranges from about 27.99 m²/g to about 32.34 m²/g. In some examples, the BET active surface area is selected from 27.99 m²/g, 28.87 m²/g and 32.34 m²/g. The ferric citrate can have an intrinsic dissolution rate of 1.88 - 4.0 mg/cm²/min.

In yet another aspect, the present disclosure provides ferric citrate for use in methods of maintaining iron stores in a patient in need thereof. The methods comprise orally administering ferric citrate to a CKD patient, e.g., a non-dialysis chronic kidney disease patient or an end stage renal disease patient, in an amount ranging from about 1 g to about 18 g per day. The ferric citrate in administered in a 1 gram tablet dosage form. The patient is administered up to 18 tablet dosage forms per day. The ferric citrate has a BET active surface area greater than about 16 m²/g. In some examples, the BET active surface area ranges from about 16 m²/g to about 20 m²/g. In some examples, the BET active surface area ranges from about 27.99 m²/g to about 32.34 m²/g. In some examples, the BET active surface area is selected from 27.99 m²/g, 28.87 m²/g and 32.34 m²/g. The ferric citrate has an intrinsic dissolution rate of 1.88 - 4.0 mg/cm²/min.

In yet another aspect, the present disclosure provides ferric citrate for use in methods of improving one or more iron storage parameters in a patient in need thereof. The methods comprise orally administering ferric citrate to a CKD patient, e.g., a non-dialysis chronic kidney disease patient or an end stage renal disease patient, in an amount ranging from about 1 g to about 18 g per day. The at least one iron storage parameter may be selected from serum ferritin levels, transferrin saturation (TSAT), hemoglobin concentration, hematocrit, total iron-binding capacity, iron absorption levels, serum iron levels, liver iron levels, spleen iron levels, and combinations thereof. The the ferric citrate in administered in a 1 gram tablet dosage form. The patient is administered up to 18 tablet dosage forms per day. The ferric citrate has a BET active surface area greater than about 16 m²/g. In some examples, the BET active surface area ranges from about 16 m²/g to about 20 m²/g. In some examples, the BET active surface area ranges from about 27.99 m²/g to about 32.34 m²/g. In some examples, the BET active surface area is selected from 27.99 m²/g, 28.87 m²/g and 32.34 m²/g. The ferric citrate can have an intrinsic dissolution rate of 1.88 - 4.0 mg/cm²/min.

Disclosed herein is that the at least one iron storage parameter is hematocrit, and improving comprises increasing the hematocrit of the patient. In some examples, the at least one iron storage parameter is hemoglobin concentration, and improving comprises increasing the hemoglobin concentration of the patient. In yet other examples, the at least one iron storage parameter is total iron-binding capacity, and improving comprises decreasing the total iron-binding capacity of the patient. In yet other examples, the at least one iron storage parameter is transferrin saturation, and improving comprises increasing the transferrin saturation of the patient. In yet other examples, the at least one iron storage parameter is serum iron levels, and improving comprises increasing the serum iron levels of the patient. In yet other examples, the at least one iron storage parameter is liver iron levels, and improving comprises increasing the liver iron levels of the patient. In yet other examples, the at least one iron storage parameter is spleen iron levels, and improving comprises increasing the spleen iron levels of the patient. In yet other examples, the at least one iron storage parameter is serum ferritin levels, and improving comprises increasing the serum ferritin levels of the patient.

Disclosed herein is that at least one iron storage parameter is serum ferritin levels, and the present disclosure provides ferric citrate for use in methods of increasing serum ferritin in a patient in need thereof. The methods comprise orally administering ferric citrate to a CKD patient, e.g., an end-stage renal disease patient at a dose of ferric iron ranging from 210 mg - 2,520 mg, wherein the ferric citrate provides a mean increase in serum ferritin in the patient selected from 150 - 310, 151 - 309, 152 - 308, 153 - 307, 154 - 306, 155 - 306, 155 - 305, 155 - 304, 155 - 303 and 155 - 302 ng/ml when administered for a period of at least 52 weeks. The ferric citrate provides a mean increase in serum ferritin of 150 - 305 ng/ml. The ferric citrate can be administered in a 1 gram tablet dosage form, each dosage form comprising 210 mg of ferric iron. The patient is administered up to 18 tablet dosage forms per day. In some examples, the patient is administered 6 tablet dosage forms per day. The ferric citrate may be administered within 1 hour of the ingestion of a meal or snack by the patient. In some examples, the patient was treated with thrice-weekly hemodialysis or with peritoneal dialysis for at least 3 months prior to administration of the ferric citrate. The ferric citrate has a BET active surface area greater than about 16 m²/g. In some examples, the BET active surface area ranges from about 16 m²/g to about 20 m²/g. In some examples, the BET active surface area ranges from about 27.99 m²/g to about 32.34 m²/g. In some examples, the BET active surface area is selected from 27.99 m²/g, 28.87 m²/g and 32.34 m²/g. The ferric citrate can have an intrinsic dissolution rate of 1.88 - 4.0 mg/cm²/min.

Disclosed herein is that the at least one iron storage parameter is transferrin saturation (TSAT), and the present disclosure provides ferric citrate for use in methods of increasing transferrin saturation (TSAT) in a patient in need thereof. The methods comprise orally administering ferric citrate to an a CKD patient, e.g., an end stage renal disease patient, at a dose of ferric iron ranging from 210 mg - 2,520 mg, wherein the ferric citrate provides a mean increase in TSAT of 5 - 10 % when administered for a period of at least 52 weeks. In some embodiments, the ferric citrate provides a mean increase in transferrin saturation (TSAT) in the patient of 6 - 9 %. The ferric citrate provides a mean increase in transferrin saturation (TSAT) in the patient of 8%. The ferric citrate can be administered in a 1 gram tablet dosage form, each dosage form comprising 210 mg of ferric iron. The patient is administered up to 18 tablet dosage forms per day. In some examples, the patient is administered 6 tablet dosage forms per day. The ferric citrate may be administered within 1 hour of the ingestion of a meal or snack by the patient. The ferric citrate has a BET active surface area greater than about 16 m²/g. In some examples, the BET active surface area ranges from about 16 m²/g to about 20 m²/g. In some examples, the BET active surface area ranges from about 27.99 m²/g to about 32.34 m²/g. In some examples, the BET active surface area is selected from 27.99 m²/g, 28.87 m²/g and 32.34 m²/g. The ferric citrate can have an intrinsic dissolution rate of 1.88 - 4.0 mg/cm²/min.

In one embodiment, the at least one iron storage parameter is hemoglobin concentration, and the present disclosure provides ferric citrate for use in methods of increasing hemoglobin concentration in a patient in need thereof. In some embodiments, the methods comprise orally administering ferric citrate to a CKD patient, e.g., an end-stage renal disease patient, at a dose of ferric iron ranging from 210 mg - 2,520 mg, wherein the ferric citrate provides a mean increase in hemoglobin concentration in the patient of 0.3 - 0.6 g/dl when administered for a period of at least 52 weeks. In some embodiments, the ferric citrate provides a mean increase in hemoglobin concentration in the patient of 0.3 - 0.5 g/dl. In some embodiments, the ferric citrate provides a mean increase in hemoglobin concentration of 0.4 g/dl. In some embodiments, the ferric citrate is administered in a 1 gram tablet dosage form, each dosage form comprising 210 mg of ferric iron. In some embodiments, the patient is administered up to 18 tablet dosage forms per day. In some embodiments, the patient is administered 6 tablet dosage forms per day. In some embodiments, the ferric citrate is administered within 1 hour of the ingestion of a meal or snack by the patient. In some embodiments, the ferric citrate has a BET active surface area greater than about 16 m²/g. In some embodiments, the BET active surface area ranges from about 16 m²/g to about 20 m²/g. In some embodiments, the BET active surface area ranges from about 27.99 m²/g to about 32.34 m²/g. In some embodiments, the BET active surface area is selected from 27.99 m²/g, 28.87 m²/g and 32.34 m²/g. In some embodiments, the ferric citrate has an intrinsic dissolution rate of 1.88 - 4.0 mg/cm²/min.

In yet another aspect, the present disclosure provides ferric citrate for use in methods of increasing iron absorption in a patient in need thereof. The methods comprise orally administering ferric citrate to a CKD patient, e.g., a non-dialysis chronic kidney disease patient or an end stage renal disease patient, in an amount ranging from about 1 g to about 18 g per day. The ferric citrate is administered in a 1 gram tablet dosage form. The patient is administered up to 18 tablet dosage forms per day. The ferric citrate has a BET active surface area greater than about 16 m²/g. In some examples, the BET active surface area ranges from about 16 m²/g to about 20 m²/g. In some examples, the BET active surface area ranges from about 27.99 m²/g to about 32.34 m²/g. In some examples, the BET active surface area is selected from 27.99 m²/g, 28.87 m²/g and 32.34 m²/g. The ferric citrate has an intrinsic dissolution rate of 1.88 - 4.0 mg/cm²/min.

In yet another aspect, the present disclosure provides ferric citrate use in methods of treating iron deficiency in a patient in need thereof. The methods comprise orally administering ferric citrate to a CKD patient, e.g., a non-dialysis chronic kidney disease patient or an end stage renal disease patient, in an amount ranging from about 1 g to about 18 g per day. The iron deficiency is anemia. The treatment provides a hemoglobin level in the patient that is at or above a level selected from 12.0 g/dl and 7.4 mmol/L. Further, the treatment provides a hemoglobin level in the patient that is at or above a level selected from 13.0 g/dl and 8.1 mmol/L.

The treatment provides a hemoglobin level in the patient that is at or above a level selected from 6.8 mmol/L, 7.1 mmol/L, 7.4 mmol/L, and 8.1 mmol/L. The treatment provides a hemoglobin level in the patient that is at or above a level selected from 11.0 g/dl, 11.5 g/dl, 12.0 g/dl, and 13.0 g/dl. The treatment reduces at least one symptom of iron deficiency selected from fatigue, dizziness, pallor, hair loss, irritability, weakness, pica, brittle or grooved nails, Plummer-Vinson syndrome, impaired immune function, pagophagia, restless legs syndrome and combinations thereof. The ferric citrate in administered in a 1 gram tablet dosage form. The patient is administered up to 18 tablet dosage forms per day. ferric citrate has a BET active surface area greater than about 16 m²/g. In some examples, the BET active surface area ranges from about 16 m²/g to about 20 m²/g. In some examples, the BET active surface area ranges from about 27.99 m²/g to about 32.34 m²/g. In some examples, the BET active surface area is selected from 27.99 m²/g, 28.87 m²/g and 32.34 m²/g. The ferric citrate can have intrinsic dissolution rate of 1.88 - 4.0 mg/cm²/min.

In yet another aspect, the present disclosure provides ferric citrate for use in methods of reducing intravenous (IV) iron use in a CKD patient, e.g., an end-stage renal disease patient. The methods comprise orally administering ferric citrate to the patient at a dose of ferric iron ranging from 210 mg - 2,520 mg, wherein the ferric citrate reduces the need for the end-stage renal disease patient to be administered IV iron by an amount selected from 50, 51, 52, 53, 54, 55, 56, 57, 58, 59 and 60 % when administered for a period of at least 52 weeks. In some examples, the ferric citrate provides a mean reduction in average cumulative IV iron intake selected from 51.0, 51.1, 51.2, 51.3, 51.4, 51.5, 51.6, 51.7, 51.9 and 52.0 %. Preferably, the ferric citrate provides a mean reduction in average cumulative IV iron intake of 51.6 %. The ferric citrate is administered in a 1 gram tablet dosage form, each dosage form comprising 210 mg of ferric iron. The patient is administered up to 18 tablet dosage forms per day. In some examples, the patient is administered 6 tablet dosage forms per day. In some examples, the ferric citrate may be administered within 1 hour of the ingestion of a meal or snack by the patient. In some examples, the patient was treated with thrice-weekly hemodialysis or with peritoneal dialysis for at least 3 months prior to administration of the ferric citrate. The ferric citrate has a BET active surface area greater than about 16 m²/g. In some examples, the BET active surface area ranges from about 16 m²/g to about 20 m²/g. In some examples, the BET active surface area ranges from about 27.99 m²/g to about 32.34 m²/g. In some examples, the BET active surface area is selected from 27.99 m²/g, 28.87 m²/g and 32.34 m²/g. The ferric citrate can have an intrinsic dissolution rate of 1.88 - 4.0 mg/cm²/min.

In yet another aspect, the present disclosure provides ferric citrate for use in methods of reducing use of erythropoiesis-stimulating agents (ESAs) in a CKD patient, e.g., an end-stage renal disease patient. The methods comprise orally administering ferric citrate to the patient at a dose of ferric iron ranging from 210 mg - 2,520 mg, wherein the ferric citrate reduces the need for the patient to be administered one or more ESAs by an amount selected from 20, 21, 22, 23, 24, 25, 26, 27, 28, 29 and 30 % when administered for a period of at least 52 weeks. In some examples, the ferric citrate provides a decrease in median ESA intake selected from 27.0, 27.1, 27.2, 27.3, 27.4, 27.5, 27.6, 27.7, 27.9 and 28.0 %. In some examples, the ferric citrate provides a mean reduction in average cumulative IV iron intake of 27.1 %. The ferric citrate can be administered in a 1 gram tablet dosage form, each dosage form comprising 210 mg of ferric iron. The patient is administered up to 18 tablet dosage forms per day. In some examples, the patient is administered 6 tablet dosage forms per day. The ferric citrate may be administered within 1 hour of the ingestion of a meal or snack by the patient. In some examples, the patient was treated with thrice-weekly hemodialysis or with peritoneal dialysis for at least 3 months prior to administration of the ferric citrate. The ferric citrate has a BET active surface area greater than about 16 m²/g. In some examples, the BET active surface area ranges from about 16 m²/g to about 20 m²/g. In some examples, the BET active surface area ranges from about 27.99 m²/g to about 32.34 m²/g. In some examples, the BET active surface area is selected from 27.99 m²/g, 28.87 m²/g and 32.34 m²/g. The ferric citrate can have an intrinsic dissolution rate of 1.88 - 4.0 mg/cm²/min.

### DETAILED DESCRIPTION

In some aspects, the present disclosure provides methods of using a ferric citrate to reduce and/or control serum phosphorus levels, increase serum bicarbonate levels, improve one or more iron storage parameters (e.g., increase serum ferritin levels, increase transferrin saturation (TSAT), increase hemoglobin concentration), increase iron absorption, maintain iron stores, treat iron deficiency, treat anemia, reduce the need for IV iron and/or reduce the need for erythropoiesis-stimulating agents (ESAs) in chronic kidney disease (CKD) patients. In each instance, the methods comprise administering ferric citrate to a CKD patient, including a non-dialysis CKD (ND-CKD) patient as well as an end stage renal disease (ESRD) patient. In some aspects, the administration of ferric citrate occurs over a long period of time including, for example, up to and including 52 weeks. In some embodiments, the administration of ferric citrate occurs over a period up to and including 56 weeks.

In each of these disclosed methods, ferric citrate may be administered to the CKD patient over a period of time that is at least 52 weeks and, in some embodiments, up to and including 56 weeks or longer. Additionally, in each of these methods the ferric citrate may be administered to the CKD patient orally, in a 1 g tablet, or caplet, dosage form that contains 210 mg of ferric iron. Up to 18 tablets, or caplets, may be administered over the course of a day.

The present disclosure also provides pharmaceutical compositions, which may also be an iron supplement, which may be administered to CKD patients. The compositions/iron supplements comprise ferric citrate as well as other pharmaceutically acceptable ingredients, as described below. The compositions/iron supplements are formulated to provide iron to CKD patients, and the amount of iron provided by the compositions/iron supplements is sufficient to increase iron absorption, improve one or more iron storage parameters, treat iron deficiency and/or treat anemia in CKD patients. The compositions/iron supplements may be provided in any number of forms, as described below. In particular, the compositions/iron supplements may be provided as oral tablet dosage forms.

Reference is now made in detail to certain embodiments of ferric citrate, dosage forms, compositions, methods of synthesis and methods of use. The disclosed embodiments are not intended to be limiting of the claims. To the contrary, the claims are intended to cover all alternatives, modifications, and equivalents.

### Therapeutic Uses of Ferric Citrate

As set forth in greater detail below, disclosed herein is ferric citrate for use in methods and dosage forms that can be used to reduce and/or control serum phosphorus levels, increase serum bicarbonate levels, improve one or more iron storage parameters (e.g., increase serum ferritin levels, increase transferrin saturation (TSAT), increase hemoglobin concentration) increase iron absorption, maintain iron stores, treat iron deficiency, treat anemia, reduce the need for IV iron and/or reduce the need for erythropoiesis-stimulating agents (ESAs) in CKD patients, including non-dialysis CKD (ND-CKD) patients and end state renal disease (ESRD) patients.

Therefore, in various aspects, the ferric citrate disclosed herein may be administered to CKD patients to reduce and/or control serum phosphorus. In various aspects, the ferric citrate disclosed herein may be administered to CKD patients to increase serum bicarbonate. In various aspects, the ferric citrate disclosed herein may be administered to CKD patients to improve one or more iron storage parameters, including to increase serum ferritin, to increase transferrin saturation (TSAT), and to increase hemoglobin concentration. It is disclosed that the ferric citrate disclosed herein may be administered to CKD patients to increase iron absorption. It is disclosed that the ferric citrate disclosed herein may be administered to CKD patients to maintain iron stores. It is disclosed that the ferric citrate disclosed herein may be administered to CKD patients to treat iron deficiency. In various aspects, the ferric citrate disclosed herein may be administered to CKD patients to treat anemia. It is disclosed that the ferric citrate disclosed herein may be administered to CKD patients to reduce the need for IV iron and/or erythropoiesis-stimulating agents (ESAs).

Ferric citrate for use in methods of treating CKD patients is also disclosed. In various aspects, the present disclosure provides ferric citrate for use in methods of reducing and/or controlling serum phosphorus, the methods comprising orally administering ferric citrate to a CKD patient, wherein the ferric citrate provides a reduction in serum phosphorus. In various aspects, the present disclosure provides ferric citrate for use in methods of increasing serum bicarbonate, the methods comprising orally administering ferric citrate to a CKD patient, wherein the ferric citrate provides an increase in serum bicarbonate. In various aspects, the present disclosure provides ferric citrate for use in methods of improving one or more iron storage parameters, the methods comprising orally administering ferric citrate to a CKD patient, wherein the ferric citrate provides improvement in one or more iron storage parameters. In various aspects, the present disclosure provides ferric citrate for use in methods of increasing serum ferritin, the methods comprising orally administering ferric citrate to a CKD patient, wherein the ferric citrate provides an increase in serum ferritin. In various aspects, the present disclosure provides ferric citrate for use in methods of increasing transferrin saturation (TSAT), the methods comprising orally administering ferric citrate to a CKD patient, wherein the ferric citrate provides an increase in TSAT. In various aspects, the present disclosure provides ferric citrate for use in methods of increasing hemoglobin concentration, the methods comprising orally administering ferric citrate to a CKD patient, wherein the ferric citrate provides an increase in hemoglobin concentration. In various aspects, the present disclosure provides ferric citrate for use in methods of increasing iron absorption, the methods comprising orally administering ferric citrate to a CKD patient, wherein the ferric citrate provides an increase in iron absorption. In various aspects, the present disclosure provides ferric citrate for use in methods of maintaining iron stores, the methods comprising orally administering ferric citrate to a CKD patient, wherein the ferric citrate provides for maintenance of iron stores. In various aspects, the present disclosure provides ferric citrate for use in methods of treating iron deficiency, the methods comprising orally administering ferric citrate to a CKD patient, wherein the ferric citrate provides treatment of iron deficiency. In various aspects, the present disclosure provides ferric citrate for use in methods of treating anemia, the methods comprising orally administering ferric citrate to a CKD patient, wherein the ferric citrate provides for treatment of anemia. In various aspects, the present disclosure provides ferric citrate for use in methods of reducing intravenous (IV) iron use in a CKD patient, the methods comprising orally administering ferric citrate to CKD patient, wherein the ferric citrate reduces the need for the CKD to be administered IV iron. In various aspects, the present disclosure provides ferric citrate for use in methods of reducing use of erythropoiesis-stimulating agents (ESAs) in CKD patient, the methods comprising orally administering ferric citrate to the CKD patient, wherein the ferric citrate reduces the need for the CKD patient to be administered one or more ESAs when administered. In each of the methods, the ferric citrate may be administered for a period of time up to and including 52 weeks, including up to and including 56 weeks.

### Chronic Kidney Disease Patients

In various aspects, the ferric citrate disclosed herein is administered to any chronic kidney disease (CKD) patients to treat any of the conditions and disorders associated with CKD, such as described herein. All individuals with a glomerular filtration rate (GFR) <60 ml/min/1.73 m² for 3 months are classified as having CKD, irrespective of the presence or absence of kidney damage. Those individuals with CKD who require either dialysis or kidney transplantation are typically referred to as end-stage renal disease (ESRD) patients. Therefore, a patient is traditionally classified as an ESRD patient when he or she reaches the conclusion of the non-dialysis dependent, earlier stages, of CKD. Prior to then, those patients are referred to as non-dialysis dependent CKD patients. However, patients with an advanced stage of CKD, such as stage 5, who have not yet started dialysis or who have not been recommended for transplantation are also typically referred to as non-dialysis dependent CKD patients.

Non-dialysis CKD (ND-CKD) patients are those who have been diagnosed with an early stage of chronic kidney disease and who have not yet been medically directed to undergo dialysis. As noted above, the U.S. National Kidney Foundation has defined 5 stages of chronic kidney disease. Typically, patients progress through stages 1 through 4 before dialysis is medically necessary.

As used herein, ND-CKD is intended to cover all patients who have been diagnosed with chronic kidney disease but who are not undergoing dialysis during the administration of ferric citrate. Such patients can include, for example, patients who have never been subjected to dialysis and, in some embodiments, patients who have been subjected to dialysis but who are not undergoing dialysis during the administration of ferric citrate.

In various aspects, ESRD patients are typically those who have been diagnosed with a late stage of chronic kidney disease. In some instances the phrase "end-stage renal disease" is used to indicate the fifth stage of CKD. Therefore, as used herein, an ESRD patient is a patient who has an advanced stage of CKD, such as stage 5, and who has begun either hemodialysis or peritoneal dialysis and/or who has been recommended for kidney transplantation by a health care provider.

In some embodiments, CKD patients display one or more of the following characteristics: a serum phosphorus level between 2.5 mg/dL and 8.0 mg/dL; a serum phosphorus level greater than or equal to 6.0 mg/dL when removed from a phosphate binder; are taking 3 to 18 pills/day of calcium acetate, calcium carbonate, lanthanum carbonate, sevelamer (carbonate or hydrochloride or equivalent sevelamer powder), any other agent serving as a phosphate binder, or a combination of any of the foregoing; have a serum ferritin level that is less than 1000 micrograms/L; have a transferrin saturation level (TSAT) that is less than 50% at screening; have a life expectancy of more than 1 year; or a combination of any of the foregoing.

In addition, CKD patients may be taking phosphorus binding agents other than ferric citrate, though this is not required. The CKD patients can be mammals and, in some embodiments, are humans. In some embodiments, CKD patients are female or male of any age and/or weight. In some embodiments, CKD patients are males or non-pregnant, non-breastfeeding females who are at least 18 years of age and have been on thrice-weekly hemodialysis and/or peritoneal dialysis for at least 3 months.

### Serum Phosphorus

Phosphate is critical for a vast array of cellular processes. It is one of the major components of the skeleton and an integral component of the nucleic acids that make up DNA and RNA. In addition, the phosphate bonds of adenosine triphosphate (ATP) carry the energy required for all cellular functions. Phosphate functions as a buffer in bone, serum, and urine and the addition and/or deletion of phosphate groups to/from enzymes and proteins are common mechanisms for the regulation of their activity. Given the breadth of influence phosphate has, its homeostasis is understandably a highly regulated process.

Patients with CKD typically demonstrate elevated levels of serum phosphate. In non-CKD patients, normal serum phosphate levels should be between 0.81 mmol/L and 1.45 mmol/L. In a CKD patient, however, serum phosphate levels are typically markedly increased as kidney function is lost and the body loses its ability to excrete phosphate through the urine. This means that CKD patients typically experience hyperphosphatemia, which is an electrolyte disturbance in which there is an abnormally elevated level of phosphate in the blood. Hyperphosphatemia develops in the majority of CKD patients and is typically associated with progression of secondary hyperparathyroidism and renal osteodystrophy. In addition, hyperphosphatemia has recently been associated with increased cardiovascular mortality among dialysis patients. Adequate control of serum phosphorus is crucial in the clinical management of CKD patients to attenuate the progression of secondary hyperparathyroidism and to reduce the risk of vascular calcification and cardiovascular mortality. Typical measures taken to control serum phosphate levels in CKD patients include dietary phosphorus restriction, dialysis, and oral phosphate binders. Unfortunately, dietary restriction has limited effect in advanced stages of CKD, such as ESRD. Therefore, oral phosphate binders are necessary to limit dietary absorption of phosphorus in CKD patients.

CKD patients treated according to the methods disclosed herein may experience an improvement in serum phosphate levels. In some embodiments, CKD patients treated according to the methods disclosed herein experience a decrease in serum phosphate levels. The present disclosure provides ferric citrate for use in methods of reducing serum phosphorus in a CKD patient, the methods comprising orally administering ferric citrate to CKD patient, e.g., an end-stage renal disease patient or non-dialysis chronic kidney disease patient, wherein the ferric citrate provides a reduction in serum phosphorus in the patient. The present disclosure provides ferric citrate for use in methods for treatment of hyperphosphatemia in a CKD patient, the methods comprising orally administering ferric citrate to CKD patient, e.g., an end-stage renal disease patient or non-dialysis chronic kidney disease patient, wherein the ferric citrate provides a reduction in serum phosphorus in the patient. The present disclosure provides ferric citrate for use in methods of reducing serum phosphorus, the methods comprising orally administering ferric citrate to an end-stage renal disease patient at a dose of ferric iron ranging from 210 mg - 2,520 mg, wherein the ferric citrate provides a reduction in serum phosphorus in the patient. The ferric citrate is administered for a period of 12 weeks. In some examples for a period of 24 weeks, for a period of 36 weeks, for a period of 48 weeks, for a period of 52 weeks, and in some examples for a period of up to and including 56 weeks. In some examples for a period of 53 weeks. some examples for a period of 54 weeks, in some examples for a period of 55 weeks. In some examples for a period of 56 weeks.

In some embodiments, the ferric citrate provides a mean reduction in serum phosphorus from 1.00 - 3.00 mg/dl. In some embodiments, the ferric citrate provides a mean reduction in serum phosphorus from 1.10 - 2.90 mg/dl. In some embodiments, the ferric citrate provides a mean reduction in serum phosphorus from 1.20 - 2.80 mg/dl. In some embodiments, the ferric citrate provides a mean reduction in serum phosphorus from 1.30 - 2.70 mg/dl. In some embodiments, the ferric citrate provides a mean reduction in serum phosphorus from 1.40 - 2.60 mg/dl. In some embodiments, the ferric citrate provides a mean reduction in serum phosphorus from 1.50 - 2.50 mg/dl. In some embodiments, the ferric citrate provides a mean reduction in serum phosphorus from 1.60 - 2.40 mg/dl. In some embodiments, the ferric citrate provides a mean reduction in serum phosphorus from 1.70 - 2.30 mg/dl. In some embodiments, the ferric citrate provides a mean reduction in serum phosphorus from 1.80 - 2.20 mg/dl. In some embodiments, the ferric citrate provides a mean reduction in serum phosphorus from 1.90 - 2.10 mg/dl. The above ranges are disclosed in this format for purposes of efficiency, and any of the above ranges can be combined with any method, formulation, or combination thereof.

In some embodiments, the ferric citrate provides a mean reduction in serum phosphorus of from 1.00 - 1.25 mg/dl, 1.00 - 1.50 mg/dl. In some embodiments, the ferric citrate provides a mean reduction in serum phosphorus of from 1.00 - 1.75 mg/dl. In some embodiments, the ferric citrate provides a mean reduction in serum phosphorus of from 1.00 - 2.00 mg/dl. In some embodiments, the ferric citrate provides a mean reduction in serum phosphorus selected from 2.00 - 2.25 mg/dl. In some embodiments, the ferric citrate provides a mean reduction in serum phosphorus selected from 2.00 - 2.50 mg/dl. In some embodiments, the ferric citrate provides a mean reduction in serum phosphorus selected from 2.00 - 2.75 mg/dl. In some embodiments, the ferric citrate provides a mean reduction in serum phosphorus selected from 2.00 - 3.00 mg/dl. In some embodiments, the ferric citrate provides a mean reduction in serum phosphorus selected from 1.00 - 2.25 mg/dl. In some embodiments, the ferric citrate provides a mean reduction in serum phosphorus selected from 1.00 - 2.50 mg/dl. In some embodiments, the ferric citrate provides a mean reduction in serum phosphorus selected from 1.00 - 2.75 mg/dl. In some embodiments, the ferric citrate provides a mean reduction in serum phosphorus selected from 1.00 - 3.00 mg/dl. In some embodiments, the ferric citrate provides a mean reduction in serum phosphorus of 2.00 - 2.50 mg/dl. The above ranges are disclosed in this format for purposes of efficiency, and any of the above ranges can be combined with any method, formulation, or combination thereof.

In some embodiments, the ferric citrate provides a mean reduction in serum phosphorus that is greater than 1.00. In some embodiments, the ferric citrate provides a mean reduction in serum phosphorus that is greater than 1.10. In some embodiments, the ferric citrate provides a mean reduction in serum phosphorus that is selected from greater than greater than 1.20. In some embodiments, the ferric citrate provides a mean reduction in serum phosphorus that is greater than 1.30. In some embodiments, the ferric citrate provides a mean reduction in serum phosphorus that is greater than 1.40. In some embodiments, the ferric citrate provides a mean reduction in serum phosphorus that is greater than 1.50. In some embodiments, the ferric citrate provides a mean reduction in serum phosphorus that is greater than 1.60. In some embodiments, the ferric citrate provides a mean reduction in serum phosphorus that is greater than 1.70. In some embodiments, the ferric citrate provides a mean reduction in serum phosphorus that is greater than 1.80. In some embodiments, the ferric citrate provides a mean reduction in serum phosphorus that is greater than 1.90. In some embodiments, the ferric citrate provides a mean reduction in serum phosphorus that is greater than 2.00. In some embodiments, the ferric citrate provides a mean reduction in serum phosphorus that is greater than 2.10. In some embodiments, the ferric citrate provides a mean reduction in serum phosphorus that is greater than 2.20. In some embodiments, the ferric citrate provides a mean reduction in serum phosphorus that is greater than 2.30. In some embodiments, the ferric citrate provides a mean reduction in serum phosphorus that is greater than 2.40. In some embodiments, the ferric citrate provides a mean reduction in serum phosphorus that is greater than 2.50. In some embodiments, the ferric citrate provides a mean reduction in serum phosphorus that is greater than 2.60. In some embodiments, the ferric citrate provides a mean reduction in serum phosphorus that is greater than 2.70. In some embodiments, the ferric citrate provides a mean reduction in serum phosphorus that is greater than 2.80. In some embodiments, the ferric citrate provides a mean reduction in serum phosphorus that is greater than 2.90 mg/dl. The above boundaries are disclosed in this format for purposes of efficiency, and any of the above boundaries can be combined with any method, formulation, lower boundary as disclosed below, or combination thereof.

In some embodiments, the ferric citrate provides a mean reduction in serum phosphorus that is less than 3.00 mg/dl. In some embodiments, the ferric citrate provides a mean reduction in serum phosphorus that is less than 2.90 mg/dl. In some embodiments, the ferric citrate provides a mean reduction in serum phosphorus that is less than 2.80 mg/dl. In some embodiments, the ferric citrate provides a mean reduction in serum phosphorus that is less than 2.70 mg/dl. In some embodiments, the ferric citrate provides a mean reduction in serum phosphorus that is less than 2.60 mg/dl. In some embodiments, the ferric citrate provides a mean reduction in serum phosphorus that is less than 2.50 mg/dl. In some embodiments, the ferric citrate provides a mean reduction in serum phosphorus that is less than 2.40 mg/dl. In some embodiments, the ferric citrate provides a mean reduction in serum phosphorus that is less than 2.30 mg/dl. In some embodiments, the ferric citrate provides a mean reduction in serum phosphorus that is less than 2.20 mg/dl. In some embodiments, the ferric citrate provides a mean reduction in serum phosphorus that is less than 2.10 mg/dl. In some embodiments, the ferric citrate provides a mean reduction in serum phosphorus that is less than 2.00 mg/dl. In some embodiments, the ferric citrate provides a mean reduction in serum phosphorus that is less than 1.90 mg/dl. In some embodiments, the ferric citrate provides a mean reduction in serum phosphorus that is less than 1.80 mg/dl. In some embodiments, the ferric citrate provides a mean reduction in serum phosphorus that is less than 1.70 mg/dl. In some embodiments, the ferric citrate provides a mean reduction in serum phosphorus that is less than 1.60 mg/dl. In some embodiments, the ferric citrate provides a mean reduction in serum phosphorus that is less than 1.50 mg/dl. In some embodiments, the ferric citrate provides a mean reduction in serum phosphorus that is less than 1.40 mg/dl. In some embodiments, the ferric citrate provides a mean reduction in serum phosphorus that is less than 1.30 mg/dl. In some embodiments, the ferric citrate provides a mean reduction in serum phosphorus that is less than 1.20 mg/dl. In some embodiments, the ferric citrate provides a mean reduction in serum phosphorus that is less than 1.10 mg/dl. The above boundaries are disclosed in this format for purposes of efficiency, and any of the above boundaries can be combined with any method, formulation, upper boundary disclosed above, or combination thereof.

In some embodiments, the ferric citrate provides a mean reduction in serum phosphorus of one of about 1.90, 1.91, 1.92, 1.93, 1.94, 1.95, 1.96, 1.97, 1.98, 1.99, 2.00, 2.01, 2.02, 2.03, 2.04, 2.05, 2.06, 2.07, 2.08, 2.09 and 2.10 mg/dl when administered for a period of 12 weeks. In some embodiments, the ferric citrate provides a mean reduction in serum phosphorus of about 2.00 mg/dl when administered for a period of 12 weeks. In some embodiments, the ferric citrate provides a mean reduction in serum phosphorus of one of about 2.10, 2.11, 2.12, 2.13, 2.14, 2.15, 2.16, 2.17, 2.18, 2.19, 2.20, 2.21, 2.22, 2.23, 2.24 and 2.25 mg/dl when administered for a period of 24 weeks. In some embodiments, the ferric citrate provides a mean reduction in serum phosphorus of about 2.20 mg/dl when administered for a period of 24 weeks. In some embodiments, the ferric citrate provides a mean reduction in serum phosphorus of one of about 2.10, 2.11, 2.12, 2.13, 2.14, 2.15, 2.16, 2.17, 2.18, 2.19 and 2.20 mg/dl when administered for a period of 36 weeks. In some embodiments, the ferric citrate provides a mean reduction in serum phosphorus of about 2.20 mg/dl when administered for a period of 36 weeks. In some embodiments, the ferric citrate provides a mean reduction in serum phosphorus of one 1.95 mg/dl, 1.96, 1.97, 1.98, 1.99, 2.00, 2.01, 2.02, 2.03, 2.04, 2.05, 2.06, 2.07, 2.08, 2.09, 2.10, 2.11, 2.12, 2.13, 2.14 and 2.15 mg/dl when administered for a period of 48 weeks. In some embodiments, the ferric citrate provides a mean reduction in serum phosphorus of about 2.10 mg/dl when administered for a period of 48 weeks. In some embodiments, the ferric citrate provides a mean reduction in serum phosphorus of one of about 1.95 mg/dl. , 1.96, 1.97, 1.98, 1.99, 2.00, 2.01, 2.02, 2.03, 2.04, 2.05, 2.06, 2.07, 2.08, 2.09, 2.10, 2.11, 2.12, 2.13, 2.14, 2.15, 2.16, 2.17, 2.18, 2.19, 2.20, 2.21, 2.22, 2.23, 2.24, 2.25, 2.26, 2.27, 2.28, 2.29 and 2.30 mg/dl when administered for a period of 52 weeks. In some embodiments, the ferric citrate provides a mean reduction in serum phosphorus of about 2.10 mg/dl when administered for a period of 52 weeks. In some embodiments, the ferric citrate provides a mean reduction in serum phosphorus of one of about .20, 0.21, 0.22, 0.23, 0.24, 0.25, 0.26, 0.27, 0.28, 0.29, 0.30, 0.31, 0.32, 0.33, 0.34 and 0.35 mg/dl when administered for a period of 56 weeks, as measured from a baseline of 52 weeks. In some embodiments, the ferric citrate provides a mean reduction in serum phosphorus of 0.30 mg/dl when administered for a period of 56 weeks, as measured from a baseline of 52 weeks.

In some embodiments, the ferric citrate provides a mean reduction in serum phosphorus selected from 20 - 35 %. In some embodiments, the ferric citrate provides a mean reduction in serum phosphorus selected from 20 - 35 %, 22 - 33 % and 25 - 30 %. In some embodiments, the ferric citrate provides a mean reduction in serum phosphorus of 27 - 28.5 %. In some embodiments, the ferric citrate provides a mean reduction in serum phosphorus of 27 - 28.4 %. In some embodiments, the ferric citrate provides a mean reduction in serum phosphorus that is selected from greater than 20, greater than 21, greater than 22, greater than 23, greater than 24, greater than 25, greater than 26, greater than 27, greater than 28, greater than 29, greater than 30, greater than 31, greater than 32, greater than 33 and greater than 34 %. In some embodiments, the ferric citrate provides a mean reduction in serum phosphorus that is selected from less than 35, less than 34, less than 33, less than 32, less than 33, less than 32, less than 31, less than 30, less than 29, less than 28, less than 27, less than 26, less than 25, less than 24, less than 23, less than 22 and less than 21 %.

In some embodiments, the ferric citrate provides a mean reduction in serum phosphorus selected from 1.90, 1.91, 1.92, 1.93, 1.94, 1.95, 1.96, 1.97, 1.98, 1.99, 2.00, 2.01, 2.02, 2.03, 2.04, 2.05, 2.06, 2.07, 2.08, 2.09 and 2.10 mg/dl when administered for a period of 12 weeks. In some embodiments, the ferric citrate provides a mean reduction in serum phosphorus of 2.00 mg/dl when administered for a period of 12 weeks. In some embodiments, the ferric citrate provides a mean reduction in serum phosphorus selected from 2.10, 2.11, 2.12, 2.13, 2.14, 2.15, 2.16, 2.17, 2.18, 2.19, 2.20, 2.21, 2.22, 2.23, 2.24 and 2.25 mg/dl when administered for a period of 24 weeks. In some embodiments, the ferric citrate provides a mean reduction in serum phosphorus of 2.20 mg/dl when administered for a period of 24 weeks. In some embodiments, the ferric citrate provides a mean reduction in serum phosphorus selected from 2.10, 2.11, 2.12, 2.13, 2.14, 2.15, 2.16, 2.17, 2.18, 2.19 and 2.20 mg/dl when administered for a period of 36 weeks. In some embodiments, the ferric citrate provides a mean reduction in serum phosphorus of 2.20 mg/dl when administered for a period of 36 weeks. In some embodiments, the ferric citrate provides a mean reduction in serum phosphorus selected from 1.95, 1.96, 1.97, 1.98, 1.99, 2.00, 2.01, 2.02, 2.03, 2.04, 2.05, 2.06, 2.07, 2.08, 2.09, 2.10, 2.11, 2.12, 2.13, 2.14 and 2.15 mg/dl when administered for a period of 48 weeks. In some embodiments, the ferric citrate provides a mean reduction in serum phosphorus of 2.10 mg/dl when administered for a period of 48 weeks. In some embodiments, the ferric citrate provides a mean reduction in serum phosphorus selected from 1.95, 1.96, 1.97, 1.98, 1.99, 2.00, 2.01, 2.02, 2.03, 2.04, 2.05, 2.06, 2.07, 2.08, 2.09, 2.10, 2.11, 2.12, 2.13, 2.14, 2.15, 2.16, 2.17, 2.18, 2.19, 2.20, 2.21, 2.22, 2.23, 2.24, 2.25, 2.26, 2.27, 2.28, 2.29 and 2.30 mg/dl when administered for a period of 52 weeks. In some embodiments, the ferric citrate provides a mean reduction in serum phosphorus of 2.10 mg/dl when administered for a period of 52 weeks. In some embodiments, the ferric citrate provides a mean reduction in serum phosphorus selected from 0.20, 0.21, 0.22, 0.23, 0.24, 0.25, 0.26, 0.27, 0.28, 0.29, 0.30, 0.31, 0.32, 0.33, 0.34 and 0.35 mg/dl when administered for a period of 56 weeks, as measured from a baseline of 52 weeks. In some embodiments, the ferric citrate provides a mean reduction in serum phosphorus of 0.30 mg/dl when administered for a period of 56 weeks, as measured from a baseline of 52 weeks.

In some embodiments, the ferric citrate provides a mean reduction in serum phosphorus as set forth in Table A:

**Table A:**

| **Mean Serum Phosphorus (mg/dL)** | **Placebo (n=91)** | **Ferric Citrate (n=92)** |
|---|---|---|
| Baseline (Week 52) | 5.3 | 5.2 |
| End of Treatment¹ (Week 56) | 7.2 | 4.9 |
| Change from Baseline at Week 56 | 1.9 | -0.3 |
| Least Squares (LS) Mean Difference from Placebo² p-value² | | -2.3 p<0.0001 |

| | | |
|---|---|---|
| ¹ Last observation carried forward was used for missing data. ² The LS Mean treatment difference and p-value is created via an ANCOVA model with treatment as the fixed effect and baseline as the covariate. | | |

In some embodiments, the ferric citrate provides a mean reduction in serum phosphorus as set forth in Table B:

**Table B:**

| **N=277** | **Baseline** | **Week** | | | | |
|---|---|---|---|---|---|---|
| | | **12** | **24** | **36** | **48** | **52** |
| **Ferric Citrate Mean Serum Phosphorus (mg/dL)¹** | 7.4 | 5.4 | 5.2 | 5.2 | 5.3 | 5.3 |
| Change from Baseline | | -2.0 | -2.2 | -2.2 | -2.1 | -2.1 |
| % Change from Baseline | | -27% | -30% | -30% | -28% | -28% |
| p-value | | <0.0001 | <0.0001 | <0.0001 | <0.0001 | <0.0001 |

| | | | | | | |
|---|---|---|---|---|---|---|
| ¹ Last observation carried forward was used for missing data. | | | | | | |

In some embodiments, CKD patients, such as ESRD patients, treated according to the methods disclosed herein experience maintenance of their serum phosphorus levels such that their serum phosphorus levels remain substantially unchanged during administration of the ferric citrate.

### Serum Bicarbonate

Metabolic acidosis is a condition that occurs in CKD patients when the body produces too much acid and/or when the kidneys are not removing enough acid from the body. If unchecked, metabolic acidosis leads to acidemia, where the blood pH drops to less than 7.35, due to increased production of hydrogen by the body and/or the inability of the body to form bicarbonate (HCO₃-) in the kidney. The consequences of metabolic acidosis in CKD patients can be serious, including coma and death. It is therefore important that CKD patients maintain a normal level of bicarbonate in their bloodstream. For non-CKD patients, a typical measure of serum bicarbonate ranges from 22 mEq/L - 28 mEq/L, or from 22 mmol/L to 28 mmol/L, respectively. In a CKD patient, however, the serum bicarbonate concentration can be greatly reduced as the kidneys lose their ability to produce bicarbonate.

CKD patients treated according to the methods disclosed herein may experience an increase in serum bicarbonate concentration. In some embodiments, CKD patients treated according to the methods disclosed herein experience an increase in serum bicarbonate concentration. The present disclosure provides ferric citrate for use in methods of increasing serum bicarbonate concentration in a CKD patient, such as an ESRD patient or ND-CKD patient, the methods comprising orally administering ferric citrate to a CKD patient, wherein the ferric citrate provides an increase in serum bicarbonate concentration in the patient. The present disclosure provides ferric citrate for use in methods of increasing serum bicarbonate concentration, the methods comprising orally administering ferric citrate to a CKD patient at a dose of ferric iron ranging from 210 mg - 2,520 mg, wherein the ferric citrate provides an increase in serum bicarbonate concentration in the patient. The patient is administered up to 18 tablet dosage forms per day. In some examples, the ferric citrate is administered for a period of 12 weeks, for a period of 36 weeks, for a period of 52 weeks, and in some examples for a period of up to and including 56 weeks.

In some embodiments, the ferric citrate provides a mean increase in serum bicarbonate concentration in the patient of 0.1 - 1.0 mEq/L. In some embodiments, the ferric citrate provides a mean increase in serum bicarbonate concentration in the patient selected from 0.70, 0.71, 0.72, 0.73, 0.74, 0.75, 0.76, 0.77, 0.78, 0.79 and 0.80 mEq/L. In some embodiments, the ferric citrate provides a mean increase in serum bicarbonate concentration in the patient of 0.71 mEq/L.

In some embodiments, the ferric citrate provides a mean increase in serum bicarbonate concentration greater than 0.70 mEq/L. In some embodiments, the ferric citrate provides a mean increase in serum bicarbonate concentration greater than 0.71 mEq/L. In some embodiments, the ferric citrate provides a mean increase in serum bicarbonate concentration greater than 0.72 mEq/L. In some embodiments, the ferric citrate provides a mean increase in serum bicarbonate concentration greater than 0.73 mEq/L. In some embodiments, the ferric citrate provides a mean increase in serum bicarbonate concentration greater than 0.74 mEq/L. In some embodiments, the ferric citrate provides a mean increase in serum bicarbonate concentration greater than 0.75 mEq/L. In some embodiments, the ferric citrate provides a mean increase in serum bicarbonate concentration greater than 0.76 mEq/L. In some embodiments, the ferric citrate provides a mean increase in serum bicarbonate concentration greater than 0.77 mEq/L. In some embodiments, the ferric citrate provides a mean increase in serum bicarbonate concentration greater than 0.78 mEq/L. In some embodiments, the ferric citrate provides a mean increase in serum bicarbonate concentration greater than 0.79 mEq/L. The above boundaries are disclosed in this format for purposes of efficiency, and any of the above boundaries can be combined with any method, formulation, lower boundary as disclosed below, or combination thereof.

In some embodiments, the ferric citrate provides a mean increase in serum bicarbonate concentration less than 0.80 mEq/L. In some embodiments, the ferric citrate provides a mean increase in serum bicarbonate concentration less than 0.79 mEq/L. In some embodiments, the ferric citrate provides a mean increase in serum bicarbonate concentration less than 0.78 mEq/L. In some embodiments, the ferric citrate provides a mean increase in serum bicarbonate concentration less than 0.77 mEq/L. In some embodiments, the ferric citrate provides a mean increase in serum bicarbonate concentration less than 0.76 mEq/L. In some embodiments, the ferric citrate provides a mean increase in serum bicarbonate concentration less than 0.75 mEq/L. In some embodiments, the ferric citrate provides a mean increase in serum bicarbonate concentration less than 0.74 mEq/L. In some embodiments, the ferric citrate provides a mean increase in serum bicarbonate concentration less than 0.73 mEq/L. In some embodiments, the ferric citrate provides a mean increase in serum bicarbonate concentration less than 0.72 mEq/L. The above boundaries are disclosed in this format for purposes of efficiency, and any of the above boundaries can be combined with any method, formulation, upper boundary disclosed above, or combination thereof.

In some embodiments, the ferric citrate provides a mean increase in serum bicarbonate concentration of 0.71 mEq/L when administered for a period of 52 weeks.

In some embodiments, CKD patients, such as ESRD patients, treated according to the methods disclosed herein experience maintenance of their serum bicarbonate concentration such that their serum bicarbonate level remains substantially unchanged during administration of the ferric citrate.

### Iron Storage Parameters

Patients with CKD may demonstrate low or inadequate markers of systemic iron status. This means that CKD patients may not have sufficient iron stored within their bodies to maintain proper iron levels. Most well-nourished, non-CKD people living in industrialized countries have approximately 4 to 5 grams of iron stored within their bodies. About 2.5 g of this iron is contained in hemoglobin, which carries oxygen through the blood. Most of the remaining approximately 1.5 to 2.5 grams of iron is contained in iron binding complexes that are present in all cells, but that are more highly concentrated in bone marrow and organs such as the liver and spleen. The liver's stores of iron are the primary physiologic reserve of iron in the non-CKD body. Of the body's total iron content, about 400 mg is utilized in proteins that use iron for cellular processes such as oxygen storage (myoglobin) or performing energy-producing redox reactions (cytochrome proteins). In addition to stored iron, a small amount of iron, typically about 3 to 4 mg, circulates through the blood plasma bound to a protein called transferrin. Because of its toxicity, free soluble ferrous iron (iron(II) or Fe²⁺) is typically kept at a low concentration in the body.

Iron deficiency first depletes the stored iron in the body. Because most of the iron utilized by the body is required for hemoglobin, iron-deficiency anemia is the primary clinical manifestation of iron deficiency. Oxygen transport to the tissues is so important to human life that severe anemia harms or kills people with CKD, inclusive of ND-CKD patients and ESRD patients, by depriving their organs of oxygen. Iron-deficient CKD patients will suffer, and in some instances may die, from organ damage caused by oxygen depletion well before cells run out of the iron needed for intracellular processes.

There are several markers of systemic iron status that may be measured to determine whether a CKD patient has sufficient iron stores to maintain adequate health. These markers may be of circulating iron stores, iron stored in iron-binding complexes, or both, and are also typically referred to as iron storage parameters. Iron storage parameters can include, for example, hematocrit, hemoglobin concentration (Hb), total iron-binding capacity (TIBC), transferrin saturation (TSAT), serum iron levels, liver iron levels, spleen iron levels, and serum ferritin levels. Of these, the hematocrit, hemoglobin concentration (Hb), total iron-binding capacity (TIBC), transferrin saturation (TSAT) and serum iron levels are commonly known as circulating iron stores. The liver iron levels, spleen iron levels, and serum ferritin levels are commonly referred to as stored iron or iron stored in iron-binding complexes.

The present disclosure provides ferric citrate for use in methods of improving one or more iron storage parameters in a patient in need thereof. The methods comprise orally administering ferric citrate to a CKD patient, e.g., a non-dialysis chronic kidney disease patient or an end stage renal disease patient, in an amount ranging from about 1 g to about 18 g per day. The at least one iron storage parameter may be selected from serum ferritin levels, transferrin saturation (TSAT), hemoglobin concentration, hematocrit, total iron-binding capacity, iron absorption levels, serum iron levels, liver iron levels, spleen iron levels, and combinations thereof. The ferric citrate in administered in a 1 gram tablet dosage form. The patient is administered up to 18 tablet dosage forms per day. In some examples, the ferric citrate is administered for a period of 12 weeks, for a period of 36 weeks, for a period of 52 weeks, and in some examples for a period of up to and including 56 weeks.

It is disclosed that the at least one iron storage parameter is hematocrit, and improving comprises increasing the hematocrit of the patient. In some embodiments, the at least one iron storage parameter is hemoglobin concentration, and improving comprises increasing the hemoglobin concentration of the patient. It is further disclosed that the at least one iron storage parameter is total iron-binding capacity, and improving comprises decreasing the total iron-binding capacity of the patient. It is further disclosed that the at least one iron storage parameter is transferrin saturation, and improving comprises increasing the transferrin saturation of the patient. It is further disclosed that the at least one iron storage parameter is serum iron levels, and improving comprises increasing the serum iron levels of the patient. It is also disclosed that the at least one iron storage parameter is liver iron levels, and improving comprises increasing the liver iron levels of the patient. It is also disclosed that the at least one iron storage parameter is spleen iron levels, and improving comprises increasing the spleen iron levels of the patient. It is also disclosed that the at least one iron storage parameter is serum ferritin levels, and improving comprises increasing the serum ferritin levels of the patient.

### Serum Ferritin

The liver's stores of ferritin are the primary source of stored iron in the body. Ferritin is an intracellular protein that stores iron and releases it in a controlled fashion. Medically, the amount of ferritin present in a blood sample and/or in a sample of liver tissue reflects the amount of iron that is stored in the liver (although ferritin is ubiquitous and can be found in many other tissues within the body in addition to the liver). Ferritin serves to store iron in the liver in a non-toxic form and to transport it to areas where it is required. In non-CKD patients, a normal ferritin blood serum level, sometimes referred to as the reference interval, is usually between 30-300 ng/ml for males, and 15-200 ng/ml for females. In a CKD patient, however, serum ferritin levels are typically markedly reduced as the amount of iron available to be bound by ferritin and stored in the liver is decreased, which occurs as the body loses its ability to absorb and store iron.

In some embodiments, CKD patients treated according to the methods disclosed herein experience an increase in serum ferritin levels. The present disclosure provides ferric citrate for use in methods of increasing serum ferritin in a patient in need thereof, the methods comprising orally administering ferric citrate to an CKD patient, e.g., an ESRD patient or ND-CKD patient, wherein the ferric citrate provides an increase in serum ferritin. The present disclosure provides ferric citrate for use in methods of increasing serum ferritin, the methods comprising orally administering ferric citrate to a CKD patient at a dose of ferric iron ranging from 210 mg - 2,520 mg, wherein the ferric citrate provides an increase in serum ferritin in the patient. In some examples, the ferric citrate is administered for a period of 12 weeks, for a period of 24 weeks, for a period of 36 weeks, for a period of 48 weeks, and in some examples for a period of 52 weeks.

In some embodiments, the ferric citrate provides a mean increase in serum ferritin of 100 - 400 ng/ml. In some embodiments, the ferric citrate provides a mean increase in serum ferritin of 110 - 390 ng/ml. In some embodiments, the ferric citrate provides a mean increase in serum ferritin of 120 - 380 ng/ml. In some embodiments, the ferric citrate provides a mean increase in serum ferritin of 130 - 370 ng/ml. In some embodiments, the ferric citrate provides a mean increase in serum ferritin of about 140 - 360 ng/ml. In some embodiments, the ferric citrate provides a mean increase in serum ferritin of 150 - 350 ng/ml. In some embodiments, the ferric citrate provides a mean increase in serum ferritin of 160 - 340 ng/ml. In some embodiments, the ferric citrate provides a mean increase in serum ferritin of 170 - 330 ng/ml. In some embodiments, the ferric citrate provides a mean increase in serum ferritin of 180 - 320 ng/ml. In some embodiments, the ferric citrate provides a mean increase in serum ferritin of 190 - 310 ng/ml. In some embodiments, the ferric citrate provides a mean increase in serum ferritin of 200 - 300 ng/ml. In some embodiments, the ferric citrate provides a mean increase in serum ferritin of 210 - 290 ng/ml. In some embodiments, the ferric citrate provides a mean increase in serum ferritin of 220 - 280 ng/ml. In some embodiments, the ferric citrate provides a mean increase in serum ferritin of 230 - 270 ng/ml. In some embodiments, the ferric citrate provides a mean increase in serum ferritin of 240 - 260 ng/ml. In some embodiments, the ferric citrate provides a mean increase in serum ferritin of from 100 - 400 ng/ml. In some embodiments, the ferric citrate provides a mean increase in serum ferritin of 100 - 375 ng/ml. In some embodiments, the ferric citrate provides a mean increase in serum ferritin of from 100 - 350 ng/ml. In some embodiments, the ferric citrate provides a mean increase in serum ferritin of from 100 - 325 ng/ml. In some embodiments, the ferric citrate provides a mean increase in serum ferritin of from 100 - 300 ng/ml. In some embodiments, the ferric citrate provides a mean increase in serum ferritin of from 100 - 275 ng/ml. In some embodiments, the ferric citrate provides a mean increase in serum ferritin of from 150 - 310 ng/ml. In some embodiments, the ferric citrate provides a mean increase in serum ferritin of from 151 - 309 ng/ml In some embodiments, the ferric citrate provides a mean increase in serum ferritin of from 152 - 308 ng/ml In some embodiments, the ferric citrate provides a mean increase in serum ferritin of from 153 - 307 ng/ml In some embodiments, the ferric citrate provides a mean increase in serum ferritin of from 154 - 306 ng/ml In some embodiments, the ferric citrate provides a mean increase in serum ferritin of from 155 - 306 ng/ml In some embodiments, the ferric citrate provides a mean increase in serum ferritin of from 155 - 305 ng/ml In some embodiments, the ferric citrate provides a mean increase in serum ferritin of from 155 - 304 ng/ml In some embodiments, the ferric citrate provides a mean increase in serum ferritin of from 155 - 303 ng/ml In some embodiments, the ferric citrate provides a mean increase in serum ferritin of from 155 - 302 ng/ml. In some embodiments, the ferric citrate provides a mean increase in serum ferritin of from 150 - 305 ng/ml. The above ranges are disclosed in this format for purposes of efficiency, and any of the above ranges can be combined with any method, formulation, or combination thereof.

In some embodiments, the ferric citrate provides a mean increase in serum ferritin of 302 ng/ml when administered over a period of 52 weeks.

In some embodiments, the ferric citrate provides a mean increase in serum ferritin that is greater than 100 ng/ml. In some embodiments, the ferric citrate provides a mean increase in serum ferritin that is greater than 110 ng/ml. In some embodiments, the ferric citrate provides a mean increase in serum ferritin that is greater than 120 ng/ml. In some embodiments, the ferric citrate provides a mean increase in serum ferritin that is greater than 130 ng/ml. In some embodiments, the ferric citrate provides a mean increase in serum ferritin that is greater than 140 ng/ml. In some embodiments, the ferric citrate provides a mean increase in serum ferritin that is greater than 150 ng/ml. In some embodiments, the ferric citrate provides a mean increase in serum ferritin that is greater than 160 ng/ml. In some embodiments, the ferric citrate provides a mean increase in serum ferritin that is greater than 170 ng/ml. In some embodiments, the ferric citrate provides a mean increase in serum ferritin that is greater than 180 ng/ml. In some embodiments, the ferric citrate provides a mean increase in serum ferritin that is greater than 190 ng/ml. In some embodiments, the ferric citrate provides a mean increase in serum ferritin that is greater than 200 ng/ml. In some embodiments, the ferric citrate provides a mean increase in serum ferritin that is greater than 210 ng/ml. In some embodiments, the ferric citrate provides a mean increase in serum ferritin that is greater than 220 ng/ml. In some embodiments, the ferric citrate provides a mean increase in serum ferritin that is greater than 230 ng/ml. In some embodiments, the ferric citrate provides a mean increase in serum ferritin that is greater than 240 ng/ml. In some embodiments, the ferric citrate provides a mean increase in serum ferritin that is greater than 250 ng/ml. In some embodiments, the ferric citrate provides a mean increase in serum ferritin that is greater than 260 ng/ml. In some embodiments, the ferric citrate provides a mean increase in serum ferritin that is greater than 270 ng/ml. In some embodiments, the ferric citrate provides a mean increase in serum ferritin that is greater than 280 ng/ml. In some embodiments, the ferric citrate provides a mean increase in serum ferritin that is greater than 290 ng/ml. In some embodiments, the ferric citrate provides a mean increase in serum ferritin that is greater than 300 ng/ml. In some embodiments, the ferric citrate provides a mean increase in serum ferritin that is greater than 310 ng/ml. In some embodiments, the ferric citrate provides a mean increase in serum ferritin that is greater than 320 ng/ml. In some embodiments, the ferric citrate provides a mean increase in serum ferritin that is greater than 330 ng/ml. In some embodiments, the ferric citrate provides a mean increase in serum ferritin that is greater than 340 ng/ml. In some embodiments, the ferric citrate provides a mean increase in serum ferritin that is greater than 350 ng/ml. In some embodiments, the ferric citrate provides a mean increase in serum ferritin that is greater than 360 ng/ml. In some embodiments, the ferric citrate provides a mean increase in serum ferritin that is greater than 370 ng/ml. In some embodiments, the ferric citrate provides a mean increase in serum ferritin that is greater than 380 ng/ml. In some embodiments, the ferric citrate provides a mean increase in serum ferritin that is greater than 390 ng/ml. The above boundaries are disclosed in this format for purposes of efficiency, and any of the above boundaries can be combined with any method, formulation, lower boundary as disclosed below, or combination thereof.

In some embodiments, the ferric citrate provides a mean increase in serum ferritin that is selected from less than 400 ng/ml. In some embodiments, the ferric citrate provides a mean increase in serum ferritin that is less than 390 ng/ml. In some embodiments, the ferric citrate provides a mean increase in serum ferritin that is less than 380 ng/ml. In some embodiments, the ferric citrate provides a mean increase in serum ferritin that is less than 370 ng/ml. In some embodiments, the ferric citrate provides a mean increase in serum ferritin that is less than 360 ng/ml. In some embodiments, the ferric citrate provides a mean increase in serum ferritin that is less than 350 ng/ml. In some embodiments, the ferric citrate provides a mean increase in serum ferritin that is less than 340 ng/ml. In some embodiments, the ferric citrate provides a mean increase in serum ferritin that is less than 330 ng/ml. In some embodiments, the ferric citrate provides a mean increase in serum ferritin that is less than 320 ng/ml. In some embodiments, the ferric citrate provides a mean increase in serum ferritin that is less than 310 ng/ml. In some embodiments, the ferric citrate provides a mean increase in serum ferritin that is less than 300 ng/ml. In some embodiments, the ferric citrate provides a mean increase in serum ferritin that is less than 290 ng/ml. In some embodiments, the ferric citrate provides a mean increase in serum ferritin that is less than 280 ng/ml. In some embodiments, the ferric citrate provides a mean increase in serum ferritin that is less than 270 ng/ml. In some embodiments, the ferric citrate provides a mean increase in serum ferritin that is less than 260 ng/ml. In some embodiments, the ferric citrate provides a mean increase in serum ferritin that is less than 250 ng/ml. In some embodiments, the ferric citrate provides a mean increase in serum ferritin that is less than 240 ng/ml. In some embodiments, the ferric citrate provides a mean increase in serum ferritin that is less than 230 ng/ml. In some embodiments, the ferric citrate provides a mean increase in serum ferritin that is less than 220 ng/ml. In some embodiments, the ferric citrate provides a mean increase in serum ferritin that is less than 210 ng/ml. In some embodiments, the ferric citrate provides a mean increase in serum ferritin that is less than 200 ng/ml. In some embodiments, the ferric citrate provides a mean increase in serum ferritin that is less than 190 ng/ml. In some embodiments, the ferric citrate provides a mean increase in serum ferritin that is less than 180 ng/ml. In some embodiments, the ferric citrate provides a mean increase in serum ferritin that is less than 170 ng/ml. In some embodiments, the ferric citrate provides a mean increase in serum ferritin that is less than 160 ng/ml. In some embodiments, the ferric citrate provides a mean increase in serum ferritin that is less than 150 ng/ml. In some embodiments, the ferric citrate provides a mean increase in serum ferritin that is less than 140 ng/ml. In some embodiments, the ferric citrate provides a mean increase in serum ferritin that is less than 130 ng/ml. In some embodiments, the ferric citrate provides a mean increase in serum ferritin that is less than 120 ng/ml. In some embodiments, the ferric citrate provides a mean increase in serum ferritin that is less than 110 ng/ml. The above boundaries are disclosed in this format for purposes of efficiency, and any of the above boundaries can be combined with any method, formulation, upper boundary as disclosed above, or combination thereof.

In some embodiments, the ferric citrate provides a mean increase in serum ferritin selected from about 280, 281, 282, 283, 284, 285, 286, 287, 288, 289, 290, 291, 292, 293, 294, 295, 296, 297, 298, 299, 300, 301, 302, 303, 304, 305, 306, 307, 308, 309 and 310 mg/dl when administered for a period of 52 weeks. In some embodiments, the ferric citrate provides a mean increase in serum ferritin of 302 mg/dl when administered for a period of 52 weeks.

In some embodiments, the ferric citrate provides a mean increase in serum ferritin from about 1 - 100 %. In some embodiments, the ferric citrate provides a mean increase in serum ferritin from about10 - 90 %. In some embodiments, the ferric citrate provides a mean increase in serum ferritin from about 20 - 80 %. In some embodiments, the ferric citrate provides a mean increase in serum ferritin from about 30 - 70 %. In some embodiments, the ferric citrate provides a mean increase in serum ferritin from about 40 - 60 %.

In some embodiments, the ferric citrate provides a mean increase in serum ferritin selected from 40, 41, 42, 43, 44, 45, 46, 47, 48, 49, 50, 51, 52, 53, 54, 55, 56, 57, 58, 59 and 60 %. In some embodiments, the ferric citrate provides a mean increase in serum ferritin selected from 48.0, 48.1, 48.2, 48.3, 48.4, 48.5, 48.6, 48.7, 48.9, 49.0, 49.1, 49.2, 49.3, 49.4, 49.5, 49.6, 49.7, 49.8, 49.9, 50.0, 50.1, 50.2, 50.3, 50.4, 50.5, 50.6, 50.7, 50.8, 50.9 and 50.8 %. In some embodiments, the ferric citrate provides a mean increase in serum ferritin of 50.8 %. In some embodiments, the ferric citrate provides a mean increase in serum ferritin of 50.8 % when administered over a period of 52 weeks.

In some embodiments, the ferric citrate provides a mean increase in serum ferritin that is greater than 1%. In some embodiments, the ferric citrate provides a mean increase in serum ferritin that is greater than 10%. In some embodiments, the ferric citrate provides a mean increase in serum ferritin that is greater than 20%. In some embodiments, the ferric citrate provides a mean increase in serum ferritin that is greater than 30%. In some embodiments, the ferric citrate provides a mean increase in serum ferritin that is greater than 40%. In some embodiments, the ferric citrate provides a mean increase in serum ferritin that is greater than 50%. In some embodiments, the ferric citrate provides a mean increase in serum ferritin that is greater than 60%. In some embodiments, the ferric citrate provides a mean increase in serum ferritin that is greater than 70%. In some embodiments, the ferric citrate provides a mean increase in serum ferritin that is greater than 80%. In some embodiments, the ferric citrate provides a mean increase in serum ferritin that is greater than 90%. The above boundaries are disclosed in this format for purposes of efficiency, and any of the above boundaries can be combined with any method, formulation, lower boundary as disclosed below, or combination thereof.

In some embodiments, the ferric citrate provides a mean increase in serum ferritin that is less than 100%. In some embodiments, the ferric citrate provides a mean increase in serum ferritin that is less than 90%. In some embodiments, the ferric citrate provides a mean increase in serum ferritin that is less than 80%. In some embodiments, the ferric citrate provides a mean increase in serum ferritin that is less than 70%. In some embodiments, the ferric citrate provides a mean increase in serum ferritin that is less than 60%. In some embodiments, the ferric citrate provides a mean increase in serum ferritin that is less than 50%. In some embodiments, the ferric citrate provides a mean increase in serum ferritin that is less than 40%. In some embodiments, the ferric citrate provides a mean increase in serum ferritin that is less than 30%. In some embodiments, the ferric citrate provides a mean increase in serum ferritin that is less than 20%. In some embodiments, the ferric citrate provides a mean increase in serum ferritin that is less than 10 %. The above boundaries are disclosed in this format for purposes of efficiency, and any of the above boundaries can be combined with any method, formulation, upper boundary disclosed above, or combination thereof.

In some embodiments, the ferric citrate provides a mean increase in serum ferritin selected from 49.0, 49.1, 49.2, 49.3, 49.4, 49.5, 49.6, 39.7, 49.8, 49.9 and 50.0 % when administered for a period of 52 weeks. In some embodiments, the ferric citrate provides a mean increase in serum ferritin of 49.2 % when administered for a period of 52 weeks.

In some embodiments, the ferric citrate provides a mean increase in serum ferritin shown in Table C:

**Table C:**

| **Mean Ferritin (ng/mL)**¹ | **Active Controls (n=134)** | **Ferric Citrate (n=249)** |
|---|---|---|
| Baseline (Day 0) | 616 | 595 |
| Week 12 | 657 | 751 |
| Week 24 | 658 | 847 |
| Week 36 | 636 | 863 |
| Week 48 | 627 | 882 |
| Week 52 | 625 | 897 |
| Change from Baseline at Week 52 | 9 | 302 |
| *% Change from Baseline* | *1.5%* | *50.8%* |
| LS Mean Difference from Active Control Group | | |
| at Week 52² | | 286 |
| p-value² | | p<0.0001 |

| | | |
|---|---|---|
| ¹ Last observation carried forward was used for missing data. ² The LS Mean treatment difference and p-value is created via an ANCOVA model with treatment as the fixed effect and baseline as the covariate. | | |

In some embodiments, CKD patients, such as ESRD patients, treated according to the methods disclosed herein experience maintenance of their serum ferritin levels such that their serum ferritin levels remain substantially unchanged during administration of the ferric citrate.

### Transferrin Saturation (TSAT)

In addition to stored iron, a small amount of iron, typically about 3 to 4 mg, circulates through the blood plasma bound to a protein called transferrin. Therefore, serum iron levels can be represented by the amount of iron circulating in the blood that is bound to the protein transferrin. Transferrin is a glycoprotein produced by the liver that can bind one or two ferric iron (iron(III) or Fe³⁺) ions. It is the most prevalent and dynamic carrier of iron in the blood, and therefore is an essential component of the body's ability to transport stored iron for use throughout the body. Transferrin saturation (or TSAT) is measured as a percentage and is calculated as the ratio of serum iron and total iron-binding capacity, multiplied by 100. This value tells a clinician how much serum iron is actually bound to the total amount of transferrin that is available to bind iron. For instance, a TSAT value of 35% means that 35% of the available iron-binding sites of transferrin in a blood sample is occupied by iron. In a non-CKD patient, typical TSAT values are approximately 15-50% for males and 12-45% for females. In a CKD patient, however, TSAT values are typically markedly reduced as the amount of iron available to be bound by transferrin is decreased, which occurs as the body loses its ability to absorb and store iron.

In some embodiments, CKD patients treated according to the methods disclosed herein experience an increase in TSAT values. The present disclosure provides ferric citrate for use in methods of increasing transferrin saturation (TSAT) in a patient in need thereof, the methods comprising orally administering ferric citrate to CKD patient, e.g., an ESRD patient or a ND-CKD patient, wherein the ferric citrate provides an increase in TSAT in the patient. The present disclosure provides ferric citrate for use in methods of increasing transferrin saturation (TSAT), the methods comprising orally administering ferric citrate to an end-stage renal disease patient at a dose of ferric iron ranging from 210 mg - 2,520 mg, wherein the ferric citrate provides an increase in TSAT in the patient. In some examples the ferric citrate is administered for a period of 12 weeks, for a period of 24 weeks, for a period of 36 weeks, for a period of 48 weeks, and in some examples for a period of 52 weeks.

In some embodiments, the ferric citrate provides a mean increase in transferrin saturation (TSAT) of 1 - 20 %. In some embodiments, the ferric citrate provides a mean increase in transferrin saturation (TSAT) of 1 - 15 %. In some embodiments, the ferric citrate provides a mean increase in transferrin saturation (TSAT) of 1 - 12 %. In some embodiments, the ferric citrate provides a mean increase in transferrin saturation (TSAT) of 5 - 12 %. In some embodiments, the ferric citrate provides a mean increase in transferrin saturation (TSAT) of 5 - 10 %. In some embodiments, the ferric citrate provides a mean increase in transferrin saturation (TSAT) of 6 - 9 %. In some embodiments, the ferric citrate provides a mean increase in transferrin saturation (TSAT) of 8%.

In some embodiments, the ferric citrate provides a mean increase in transferrin saturation (TSAT) greater than 1%. In some embodiments, the ferric citrate provides a mean increase in transferrin saturation (TSAT) greater than 2%. In some embodiments, the ferric citrate provides a mean increase in transferrin saturation (TSAT) greater than 3%. In some embodiments, the ferric citrate provides a mean increase in transferrin saturation (TSAT) greater than 4%. In some embodiments, the ferric citrate provides a mean increase in transferrin saturation (TSAT) greater than 5%. In some embodiments, the ferric citrate provides a mean increase in transferrin saturation (TSAT) greater than 6%. In some embodiments, the ferric citrate provides a mean increase in transferrin saturation (TSAT) greater than 7%. In some embodiments, the ferric citrate provides a mean increase in transferrin saturation (TSAT) greater than 8%. In some embodiments, the ferric citrate provides a mean increase in TSAT greater than 9%. In some embodiments, the ferric citrate provides a mean increase in TSAT greater than 10%. In some embodiments, the ferric citrate provides a mean increase in TSAT greater than 11%. In some embodiments, the ferric citrate provides a mean increase in TSAT greater than 12%. In some embodiments, the ferric citrate provides a mean increase in TSAT greater than 13%. In some embodiments, the ferric citrate provides a mean increase in TSAT greater than 14%. In some embodiments, the ferric citrate provides a mean increase in TSAT greater than 15%. In some embodiments, the ferric citrate provides a mean increase in TSAT greater than 16%. In some embodiments, the ferric citrate provides a mean increase in TSAT greater than 17%. In some embodiments, the ferric citrate provides a mean increase in TSAT greater than 18%. In some embodiments, the ferric citrate provides a mean increase in TSAT greater than 19%. The above boundaries are disclosed in this format for purposes of efficiency, and any of the above ranges can be combined with any method, formulation, lower boundary as disclosed below, or combination thereof.

In some embodiments, the ferric citrate provides a mean increase in transferrin saturation (TSAT) less than 20%. In some embodiments, the ferric citrate provides a mean increase in TSAT less than 19%. In some embodiments, the ferric citrate provides a mean increase in TSAT less than 18%. In some embodiments, the ferric citrate provides a mean increase in TSAT less than 17%. In some embodiments, the ferric citrate provides a mean increase in TSAT less than 16%. In some embodiments, the ferric citrate provides a mean increase in TSAT less than 15%. In some embodiments, the ferric citrate provides a mean increase in TSAT less than 14%. In some embodiments, the ferric citrate provides a mean increase in TSAT less than 13%. In some embodiments, the ferric citrate provides a mean increase in TSAT less than 12%. In some embodiments, the ferric citrate provides a mean increase in TSAT less than 11%. In some embodiments, the ferric citrate provides a mean increase in TSAT less than 10%. In some embodiments, the ferric citrate provides a mean increase in TSAT less than 9%. In some embodiments, the ferric citrate provides a mean increase in TSAT less than 8%. In some embodiments, the ferric citrate provides a mean increase in TSAT less than 7%. In some embodiments, the ferric citrate provides a mean increase in TSAT less than 6%. In some embodiments, the ferric citrate provides a mean increase in TSAT less than 5%. In some embodiments, the ferric citrate provides a mean increase in TSAT less than 4%. In some embodiments, the ferric citrate provides a mean increase in TSAT less than 3 %. In some embodiments, the ferric citrate provides a mean increase in TSAT less than 2 %. The above boundaries are disclosed in this format for purposes of efficiency, and any of the above ranges can be combined with any method, formulation, upper boundary disclosed above, or combination thereof.

In some embodiments, the ferric citrate provides a mean increase in transferrin saturation (TSAT) selected from 5 %, 6 %, 7 %, 8 %, 9 %, 10 %, 11 %, 12 %, 13 %, 14 %, 15 %, 16 %, 17 % and 18 % when administered for a period of 52 weeks. In some embodiments, the ferric citrate provides a mean increase in transferrin saturation (TSAT) of 8 % when administered for a period of 52 weeks.

In some embodiments, the ferric citrate provides a mean increase in transferrin saturation (TSAT) shown in Table D:

**Table D:**

| **Mean TSAT (%)¹** | **Active Controls (n=131)** | **Ferric Citrate (n=244)** |
|---|---|---|
| Baseline (Day 0) | 31 | 31 |
| Week 12 | 31 | 40 |
| Week 24 | 32 | 40 |
| Week 36 | 30 | 40 |
| Week 48 | 29 | 41 |
| Week 52 | 30 | 39 |
| Change from Baseline at Week 52 | -1 | 8 |
| *% Change from Baseline* | *-3.2%* | *25.8%* |
| LS Mean Difference from Active Control Group | | |
| at Week 52² | | 10 |
| p-value² | | p<0.0001 |

| | | |
|---|---|---|
| ¹ Last observation carried forward was used for missing data. ² The LS Mean treatment difference and p-value is created via an ANCOVA model with treatment as the fixed effect and baseline as the covariate. | | |

In some embodiments, CKD patients, such as ESRD patients, treated according to the methods disclosed herein experience maintenance of their TSAT values such that their transferrin saturation (TSAT) value remains substantially unchanged during administration of the ferric citrate.

### Hematocrit

The hematocrit, also referred to as packed cell volume or erythrocyte volume fraction, is the volume percentage of red blood cells in the blood. For non-CKD patients, the hematocrit is typically about 45% of blood volume for men and about 40% of blood volume for women. In CKD patients, however, the hematocrit is often significantly depleted due to poor iron absorption and/or poor iron storage capacity.

The ferric citrate disclosed herein may be administered to CKD patients to increase hematocrit. The exact timing of administration will necessarily vary from patient to patient, depending upon, for example, the severity of CKD experienced by the CKD patient, the level of iron absorption the patient is or is not experiencing, and the judgment of the treating health care professional. The present disclosure provides ferric citrate for use in methods of increasing hematocrit in a patient in need thereof, the methods comprising orally administering ferric citrate to a CKD patient, e.g., an ESRD patient or ND-CKD patient, wherein the ferric citrate provides for an increase in the hematocrit of the patient. The present disclosure provides ferric citrate for use in methods of increasing hematocrit in a CKD patient, the methods comprising orally administering ferric citrate to the patient at a dose of ferric iron ranging from 210 mg - 2,520 mg, wherein the ferric citrate provides for an increase in the hematocrit of the patient. The ferric citrate is administered for a period of 52 weeks. The increase is from 1% to 30%. The increase is from 1% to 20%. The increase is from 1% to 15%, the increase is from 1% to 12%, the increase is from 1% to 10%, the increase is from 1% to 9%, in some embodiments the increase is from 1% to 8%, the increase is from 1% to 7%, the increase is from 1% to 6%, the increase is from 1% to 5%, the increase is from 1% to 4%, the increase is from 1% to 3%, and the increase is from 1% to 2%.

In some embodiments, CKD patients, such as ESRD patients, treated according to the methods disclosed herein experience maintenance of their hematocrit level such that their overall volume of red blood cells in the blood remains substantially unchanged during administration of the ferric citrate.

### Hemoglobin Concentration

Hemoglobin concentration, also referred to as the mean corpuscular hemoglobin concentration or MCHC, is a measure of the concentration of hemoglobin protein in a given volume of packed red blood cells. It is typically calculated by dividing the total amount of hemoglobin protein by the hematocrit. Hemoglobin concentration may also be measured as a mass or weight fraction and presented as a percentage (%). Numerically, however, the mass or molar measure of hemoglobin concentration and the mass or weight fraction (%) are identical, assuming a red blood cell density of 1g/ml and negligible hemoglobin loss in the blood plasma. For non-CKD patients, a typical mass or molar measure of hemoglobin concentration ranges from 32 g/dl - 36 g/dl, or from 4.9 mmol/L to 5.5 mmol/L, respectively. In a CKD patient, however, the hemoglobin concentration can be greatly reduced as the body loses its ability to absorb and store iron.

In some embodiments, CKD patients treated according to the methods disclosed herein experience an increase in hemoglobin concentration. The present disclosure provides ferric citrate for use in methods of increasing hemoglobin concentration in a patient in need thereof, the methods comprising orally administering ferric citrate to a CKD patient, e.g., an ESRD patient or ND-CKD patient, wherein the ferric citrate provides an increase in hemoglobin concentration in the patient. The present disclosure provides ferric citrate for use in methods of increasing hemoglobin concentration, the methods comprising orally administering ferric citrate to a CKD patient at a dose of ferric iron ranging from 210 mg - 2,520 mg, wherein the ferric citrate provides an increase in hemoglobin concentration in the patient. In some examples the ferric citrate is administered for a period of 12 weeks, for a period of 24 weeks, for a period of 36 weeks, for a period of 48 weeks, and in some examples for a period of 52 weeks.

In some embodiments, the ferric citrate provides a mean increase in hemoglobin concentration of 0.1 - 5.0 g/dl. In some embodiments, the ferric citrate provides a mean increase in hemoglobin concentration of 0.1 - 4.0 g/dl. In some embodiments, the ferric citrate provides a mean increase in hemoglobin concentration of 0.1 - 3.0 g/dl. In some embodiments, the ferric citrate provides a mean increase in hemoglobin concentration of 0.1 - 2.0 g/dl. In some embodiments, the ferric citrate provides a mean increase in hemoglobin concentration of 0.1 - 1.0 g/dl. In some embodiments, the ferric citrate provides a mean increase in hemoglobin concentration of 0.2 - 0.9 g/dl. In some embodiments, the ferric citrate provides a mean increase in hemoglobin concentration of 0.3 - 0.8 g/dl. In some embodiments, the ferric citrate provides a mean increase in hemoglobin concentration of 0.3 - 0.7 g/dl. In some embodiments, the ferric citrate provides a mean increase in hemoglobin concentration of 0.3 - 0.6 g/dl. In some embodiments, the ferric citrate provides a mean increase in hemoglobin concentration of 0.3 - 0.5 g/dl. In some embodiments, the ferric citrate provides a mean increase in hemoglobin concentration of 0.4 g/dl.

In some embodiments, the ferric citrate provides a mean increase in hemoglobin concentration greater than 0.1 g/dl. In some embodiments, the ferric citrate provides a mean increase in hemoglobin concentration greater than 0.2 g/dl. In some embodiments, the ferric citrate provides a mean increase in hemoglobin concentration greater than 0.3 g/dl. In some embodiments, the ferric citrate provides a mean increase in hemoglobin concentration greater than 0.4 g/dl. In some embodiments, the ferric citrate provides a mean increase in hemoglobin concentration greater than 0.5 g/dl. In some embodiments, the ferric citrate provides a mean increase in hemoglobin concentration greater than 0.6 g/dl. In some embodiments, the ferric citrate provides a mean increase in hemoglobin concentration greater than 0.7 g/dl. In some embodiments, the ferric citrate provides a mean increase in hemoglobin concentration greater than 0.8 g/dl. In some embodiments, the ferric citrate provides a mean increase in hemoglobin concentration greater than 0.9 g/dl. The above boundaries are disclosed in this format for purposes of efficiency, and any of the above boundaries can be combined with any method, formulation, lower boundary as disclosed below, or combination thereof.

In some embodiments, the ferric citrate provides a mean increase in hemoglobin concentration of less than 1.0 g/dl. In some embodiments, the ferric citrate provides a mean increase in hemoglobin concentration less than 0.9 g/dl. In some embodiments, the ferric citrate provides a mean increase in hemoglobin concentration less than 0.8 g/dl. In some embodiments, the ferric citrate provides a mean increase in hemoglobin concentration less than 0.7 g/dl. In some embodiments, the ferric citrate provides a mean increase in hemoglobin concentration less than 0.6 g/dl. In some embodiments, the ferric citrate provides a mean increase in hemoglobin concentration less than 0.5 g/dl. In some embodiments, the ferric citrate provides a mean increase in hemoglobin concentration less than 0.4 g/dl. In some embodiments, the ferric citrate provides a mean increase in hemoglobin concentration less than 0.3 g/dl. In some embodiments, the ferric citrate provides a mean increase in hemoglobin concentration less than 0.2 g/dl. The above boundaries are disclosed in this format for purposes of efficiency, and any of the above boundaries can be combined with any method, formulation, upper boundary disclosed above, or combination thereof.

In some embodiments, the ferric citrate provides a mean increase in hemoglobin concentration shown in Table E:

**Table E:**

| **Mean Hemoglobin (g/dL)¹** | **Active Controls (n=130)** | **Ferric Citrate (n=244)** |
|---|---|---|
| Baseline (Day 0) | 11.7 | 11.6 |
| Week 52 | 11.1 | 11.4 |
| Change from Baseline at Week 52 | -0.6 | -0.2 |
| LS Mean Difference from Active Control Group | | |
| at Week 52² | | 0.4 |
| p-value² | | p=0.0105 |

| | | |
|---|---|---|
| ¹ Last observation carried forward was used for missing data. ² The LS Mean treatment difference and p-value is created via an ANCOVA model with treatment as the fixed effect and baseline as the covariate. | | |

In some embodiments, CKD patients, such as ESRD patients, treated according to the methods disclosed herein experience maintenance of their hemoglobin concentration such that their hemoglobin level remains substantially unchanged during administration of the ferric citrate.

### Total Iron Binding Capacity (TIBC)

Total iron-binding capacity (TIBC) is a measure of the blood's capacity to bind iron with the protein transferrin. TIBC is typically measured by drawing a blood sample and measuring the maximum amount of iron that the sample can carry. Thus, TIBC indirectly measures transferrin, which is a protein that transports iron in the blood. For non-CKD patients, a typical mass or molar measure of TIBC is in the range of 250-370 µg/dL or 45-66 µmol/L, respectively. In CKD patients, however, the TIBC is typically increased above these levels, as the body must produce more transferrin in an attempt to deliver iron to erythrocyte precursor cells to produce hemoglobin.

In some embodiments, CKD patients treated according to the methods disclosed herein experience a reduction in TIBC. The present disclosure provides ferric citrate for use in methods of reducing TIBC in patient in need thereof, the methods comprising orally administering ferric citrate to a CKD patient, e.g., an ESRD patient or ND-CKD patient, wherein the ferric citrate provides for a reduction in the TIBC of the patient. The present disclosure provides ferric citrate for use in methods of reducing TIBC in a CKD patient, the methods comprising orally administering ferric citrate to the patient at a dose of ferric iron ranging from 210 mg - 2,520 mg, wherein the ferric citrate provides for a reduction in the TIBC of the patient. The ferric citrate is administered for a period of 52 weeks. In some examples, the reduction is from 0.1% to 30%, the reduction is from 0.1% to 28%, the reduction is from 0.1% to 26%, the reduction is from 0.1% to 25%, the reduction is from 0.1% to 24%, the reduction is from 0.1% to 23%, the reduction is from 0.1% to 22%, the reduction is from 0.1% to 21%, the reduction is from 0.1% to 20%, the reduction is from 0.1% to 15%, the reduction is from 0.1% to 10%, and in some examples the reduction is from 0.1% to 5%.

In some embodiments, CKD patients, such as ESRD patients, treated according to the methods disclosed herein experience maintenance of their TIBC such that their TIBC level remains substantially unchanged during administration of the ferric citrate.

### Iron Absorption

CKD patients may suffer from low or inadequate iron absorption that can lead to other health concerns such as iron depletion and anemia. For humans, the majority of iron absorbed from food or supplements is absorbed in the small intestine, particularly in the duodenum, by specialized enterocyte cells present in the duodenal lining. These cells have specialized transporter molecules that allow them to move iron from the intestinal lumen into the body. To be absorbed, dietary iron must be present as part of a protein, such as heme, or it must be in ferrous (iron(II) or Fe²⁺) form. Enterocytes express a ferric reductase enzyme, Dcytb, which reduces ferric iron (iron(III) or Fe³⁺) to ferrous iron. A divalent metal transporter protein then transports the iron across the enterocyte's cell membrane and into the cell.

In a non-CKD person, the body regulates iron levels by changing the expression level of the proteins relating to one or more of these steps. For example, in response to iron-deficiency anemia, cells may produce more of the Dcytb enzyme and more of the metal transporter protein in order to increase the amount of iron absorbed from the intestinal lumen. In CKD patients, the body's ability to regulate one or more of these steps is impaired, which in turn leads to reduced or inadequate iron absorption.

CKD patients treated according to the methods disclosed herein may experience increased iron absorption. In some embodiments, the iron that is absorbed is provided by the ferric citrate that is administered to the CKD patients; it is the ferric iron ion that is absorbed into the body from the intestinal lumen. Because the ferric citrate is administered orally, the increased iron absorption occurs through the intestine. While not wishing to be bound by any theory, it is believed that the increased iron absorption may be attributable to the presence of citrate in the ferric citrate administered to the CKD patient. Some studies have shown that administration of iron in combination with citrate (the conjugate base of citric acid) serves to significantly increase (*e.g*., by several fold) the amount of iron absorbed from dietary sources (*see, e.g.,* Ballot, et al., Br. J. Nutr. (1987) 57, 331-343; Gillooly, et al., Br. J. Nutr. (1983) 49, 331-342; Zhang, et al., Eur. J. Nutr. (2007) 46, 95-102; and Salovaara, et al., J. Agric. Food Chem. (2002) 50, 6233-6238).

The ferric citrate disclosed herein may be administered to CKD patients to increase iron absorption. The exact timing of administration will necessarily vary from patient to patient, depending upon, for example, the stage of CKD experienced by the CKD patient, the level of iron absorption the patient is or is not experiencing, and the judgment of the treating health care professional. The present disclosure provides ferric citrate for use in methods of increasing iron absorption in an end-stage renal disease patient, the methods comprising orally administering ferric citrate to the patient, wherein the ferric citrate provides for an increase in the amount of iron absorbed by the patient. The present disclosure provides methods of increasing iron absorption in an end-stage renal disease patient, the methods comprising orally administering ferric citrate to the patient at a dose of ferric iron ranging from 210 mg - 2,520 mg, wherein the ferric citrate provides for an increase in the amount of iron absorbed by the patient. The ferric citrate is administered for a period of 52 weeks.

### Iron Deficiency and Anemia

As stated above, most well-nourished, non-CKD people living in industrialized countries have approximately 4 to 5 grams of iron stored within their bodies in some manner (*e.g*., as circulating iron or stored iron or both). A decrease in this amount represents an iron deficiency, which is commonly seen in CKD patients. Symptoms of iron deficiency can occur in CKD patients before the condition has progressed to iron-deficiency anemia. Symptoms of iron deficiency can include, for example, fatigue, dizziness, pallor, hair loss, irritability, weakness, pica, brittle or grooved nails, Plummer-Vinson syndrome (painful atrophy of the mucous membrane covering the tongue, pharynx and esophagus), impaired immune function, pagophagia, and restless legs syndrome, among others.

CKD patients treated according to the methods disclosed herein may experience an improvement in iron deficiency. In some embodiments, CKD patients treated according to the methods disclosed herein experience a decrease in iron deficiency. This decrease may occur as the total amount of iron in the body of the CKD patient is increased through the administration of the ferric citrate disclosed herein. In some embodiments, CKD patients treated according to the methods disclosed herein experience a decrease in one or more symptoms of iron deficiency, wherein the symptoms are selected from fatigue, dizziness, pallor, hair loss, irritability, weakness, pica, brittle or grooved nails, Plummer-Vinson syndrome (painful atrophy of the mucous membrane covering the tongue, pharynx and esophagus), impaired immune function, pagophagia, restless legs syndrome and combinations of the foregoing. In some embodiments, CKD patients treated according to the methods disclosed herein experience the elimination of one or more symptoms of iron deficiency, wherein the symptoms are selected from fatigue, dizziness, pallor, hair loss, irritability, weakness, pica, brittle or grooved nails, Plummer-Vinson syndrome (painful atrophy of the mucous membrane covering the tongue, pharynx and esophagus), impaired immune function, pagophagia, restless legs syndrome and combinations of the foregoing.

In some embodiments, the iron deficiency is anemia. In some embodiments, the iron deficiency is iron-deficiency anemia. Iron-deficiency anemia is characterized by low levels of circulating red blood cells and, in CKD patients, can be caused by insufficient dietary intake, absorption and/or storage of iron. Red blood cells, which contain iron bound in hemoglobin proteins, and are typically not formed when the amount of iron in the body is deficient.

Iron-deficiency anemia is typically characterized by pallor (pale color resulting from reduced oxyhemoglobin in the skin and mucous membranes), fatigue, lightheadedness, and weakness. However, signs of iron-deficiency anemia can vary between CKD patients. Because iron deficiency in CKD patients tends to develop slowly, adaptation to the disease can occur and it can go unrecognized for some time. In some instances, patients with CKD can develop dyspnea (trouble breathing), pica (unusual obsessive food cravings), anxiety often resulting in OCD-type compulsions and obsessions, irritability or sadness, angina, constipation, sleepiness, tinnitus, mouth ulcers, palpitations, hair loss, fainting or feeling faint, depression, breathlessness on exertion, twitching muscles, pale yellow skin, tingling (numbness) or burning sensations, missed menstrual cycle(s), heavy menstrual period(s), slow social development, glossitis (inflammation or infection of the tongue), angular cheilitis (inflammatory lesions at the mouth's corners), koilonychia (spoon-shaped nails) or nails that are weak or brittle, poor appetite, pruritus (generalized itchiness), Plummer-Vinson syndrome (painful atrophy of the mucous membrane covering the tongue, pharynx and esophagus), and restless legs syndrome, among others.

Anemia is typically diagnosed based on a complete blood count measured from a blood sample from a patient. Typically, automatic counters are utilized that report the total number of red blood cells in a sample, the hemoglobin level, and the size of the red blood cells by flow cytometry. However, a stained blood smear on a microscope slide can be examined using a microscope in order to count the total number of red blood cells in a sample and diagnose anemia. In many countries, four parameters (red blood cell count, hemoglobin concentration, mean corpuscular volume and red blood cell distribution width) are measured to determine the presence of anemia. The World Health Organization has set certain threshold values for hemoglobin levels (Hb), such that when an CKD patient's hemoglobin levels fall below those values, a diagnosis of anemia may be made. Those values are: for children 0.5-5.0 yrs of age, Hb = 11.0 g/dL or 6.8 mmol/L; for children 5-12 yrs years of age, Hb = 11.5 g/ dL or 7.1 mmol/L; for teens 12-15 yrs of age, Hb = 12.0 g/ dL or 7.4 mmol/L; for non-pregnant women 15 years of age and older, Hb = 12.0 g/ dL or 7.4 mmol/L; for pregnant women, Hb = 11.0 g/ dL or 6.8 mmol/L; and for men greater than 15 yrs of age, Hb = 13.0 g/ dL or 8.1 mmol/L.

CKD patients treated according to the methods disclosed herein may experience an improvement in anemia. CKD patients treated according to the methods disclosed herein may experience an improvement in iron-deficiency anemia. In some embodiments, CKD patients treated according to the methods disclosed herein experience a decrease in one or more symptoms of anemia or iron-deficiency anemia. In some embodiments, CKD patients treated according to the methods disclosed herein experience the elimination of one or more symptoms of anemia or iron-deficiency anemia. In some embodiments, the one or more symptoms of anemia or iron-deficiency anemia are selected from pallor, fatigue, lightheadedness, weakness, dyspnea, pica, anxiety, irritability or sadness, angina, constipation, sleepiness, tinnitus, mouth ulcers, palpitations, hair loss, fainting or feeling faint, depression, breathlessness on exertion, twitching muscles, pale yellow skin, tingling (numbness) or burning sensations, missed menstrual cycle(s), heavy menstrual period(s), slow social development, glossitis, angular cheilitis, koilonychia, poor appetite, pruritus, Plummer-Vinson syndrome, restless legs syndrome and combinations of the foregoing.

In some embodiments, CKD patients treated according to the methods disclosed herein may experience an improvement in anemia and/or iron-deficiency anemia because hemoglobin levels are raised and/or maintained above a threshold level. In some embodiments, a method of treating anemia in a CKD patient is disclosed, the method comprising orally administering ferric citrate to the CKD patient, wherein the ferric citrate provides a hemoglobin level in the CKD patient that is at or above a level ranging from 11.0 g/dL - 13.0 g/dL, including a level selected from 11.0 g/dL, 11.5 g/dL, 12.0 g/dL, and 13.0 g/dL. In some embodiments, a method of treating anemia in a CKD patient is disclosed, the method comprising orally administering ferric citrate to the CKD patient, wherein the ferric citrate provides a hemoglobin level in the CKD patient that is at or above a level selected from 6.8 mmol/L, 7.1 mmol/L, 7.4 mmol/L, and 8.1 mmol/L. In some embodiments, a method of treating anemia in a male CKD patient is disclosed, the method comprising orally administering ferric citrate to the male CKD patient, wherein the ferric citrate provides a hemoglobin level in the male CKD patient that is at or above a level selected from 13.0 g/dL and 8.1 mmol/L. In some embodiments, a method of treating anemia in a female CKD patient is disclosed, the method comprising orally administering ferric citrate to the female CKD patient, wherein the ferric citrate provides a hemoglobin level in the female CKD patient that is at or above a level selected from 12.0 g/dL and 7.4 mmol/L.

In some embodiments, ferric citrate for use in a method of treating anemia in a CKD patient is disclosed, wherein the ferric citrate provides a hemoglobin level in the CKD patient that is at or above a level ranging from 11.0 g/dL - 13.0 g/dL, including a level selected from 11.0 g/dL, 11.5 g/dL, 12.0 g/dL, and 13.0 g/dL. In some embodiments, ferric citrate for use in a method of treating anemia in a CKD patient is disclosed, wherein the ferric citrate provides a hemoglobin level in the CKD patient that is at or above a level selected from 6.8 mmol/L, 7.1 mmol/L, 7.4 mmol/L, and 8.1 mmol/L. In some embodiments, ferric citrate for use in a method of treating anemia in a male CKD patient is disclosed, wherein the ferric citrate provides a hemoglobin level in the male CKD patient that is at or above a level selected from 13.0 g/dL and 8.1 mmol/L. In some embodiments, ferric citrate for use in a method of treating anemia in a female CKD patient is disclosed, wherein the ferric citrate provides a hemoglobin level in the female CKD patient that is at or above a level selected from 12.0 g/dL and 7.4 mmol/L.

### Intravenous Iron

Patients with CKD may be at risk for, or may suffer from, iron deficiency. Iron deficiency, also referred to as sideropenia or hypoferremia, is a common type of nutritional deficiency, and can occur in a CKD patient as the body loses its ability to absorb iron from the intestinal lumen and/or to store iron for long-term use. When a loss of or decrease in iron in the body is not compensated for by, for example, a sufficient intake of iron from the diet, iron deficiency can develop over time. When a state of iron deficiency is left uncorrected, it can lead to iron-deficiency anemia. Therefore, a direct consequence of untreated, long-term iron deficiency can be iron-deficiency anemia and, in some instances, anemia.

In CKD patients, there are typically three means by which iron-deficiency anemia can be treated. The first approach is by eating foods that are high in iron. If that is insufficient, then a clinician may prescribe oral iron supplements. However, many oral iron supplements cause numerous adverse side effects in CKD patients, which leads to patient non-compliance. In those instances where a CKD patient cannot take oral iron supplements, he or she may have to have intravenous iron supplementation.

Intravenous (IV) iron supplementation is a method of delivering iron by injection with a needle, either through a muscle or into a vein. CKD patients who are receiving IV iron usually do so because they cannot take oral iron. In particular, ESRD patients are on dialysis and often lose blood during dialysis. These patients are usually also taking an erythropoiesis-stimulating agent (ESA - see below) and may need extra iron because of that as well. Intravenous iron is delivered into the CKD patient's vein through a needle that is attached to an IV bag that contains an iron solution. The procedure takes place in a doctor's office or a clinic and may take up to several hours, depending on which treatment the physician has prescribed. The CKD patient usually receives iron injections over the course of several visits until his or her iron levels are correct. In some instances, an CKD patient may require permanent IV iron supplementation.

The side effects of IV iron supplementation include: gastrointestinal pains, including nausea and cramps; problems breathing; skin problems, including rash; chest pain; low blood pressure; and anaphylaxis, among others.

CKD patients treated according to the methods disclosed herein may experience a decrease in the need for IV iron supplementation. In some embodiments, CKD patients treated according to the methods disclosed herein experience a decrease in cumulative IV iron supplementation. The disclosure provides ferric citrate for use in methods of reducing intravenous (IV) iron use in a patient in need thereof, the methods comprising orally administering ferric citrate to a CKD patient, particularly an ESRD patient, wherein the ferric citrate provides for a reduction in IV iron use in the patient. The present disclosure provides ferric citrate for use in methods of reducing intravenous (IV) iron use in an end-stage renal disease patient, the methods comprising orally administering ferric citrate to the patient at a dose of ferric iron ranging from 210 mg - 2,520 mg, wherein the ferric citrate provides for a reduction in IV iron use in the patient. The ferric citrate is administered for a period of 52 weeks.

In some embodiments, the ferric citrate provides a mean reduction in average cumulative IV iron intake from 1 - 100%. In some embodiments, the ferric citrate provides a mean reduction in average cumulative IV iron intake from 10 - 90 %. In some embodiments, the ferric citrate provides a mean reduction in average cumulative IV iron intake from 20 - 80 %. In some embodiments, the ferric citrate provides a mean reduction in average cumulative IV iron intake from 30 - 70 %. The above ranges are disclosed in this format for purposes of efficiency, and any of the above ranges can be combined with any method, formulation, or combination thereof.

In some embodiments, the ferric citrate provides a mean reduction in average cumulative IV iron intake from 40 - 60 %. In some embodiments, the ferric citrate provides a mean reduction in average cumulative IV iron intake selected from 50, 51, 52, 53, 54, 55, 56, 57, 58, 59 and 60 %. In some embodiments, the ferric citrate provides a mean reduction in average cumulative IV iron intake selected from 51.0, 51.1, 51.2, 51.3, 51.4, 51.5, 51.6, 51.7, 51.9 and 52.0 %. In some embodiments, the ferric citrate provides a mean reduction in average cumulative IV iron intake of 51.6 %. In some embodiments, the ferric citrate provides a mean reduction in average cumulative IV iron intake of 51.6 % when administered over a period of 52 weeks.

In some embodiments, the ferric citrate provides a mean reduction in average cumulative IV iron intake that is greater than 10%. In some embodiments, the ferric citrate provides a mean reduction in average cumulative IV iron intake that is greater than 20%. In some embodiments, the ferric citrate provides a mean reduction in average cumulative IV iron intake that is greater than 30%. In some embodiments, the ferric citrate provides a mean reduction in average cumulative IV iron intake that is greater than 40%. In some embodiments, the ferric citrate provides a mean reduction in average cumulative IV iron intake that is greater than 50%.

In some embodiments, the ferric citrate provides a mean reduction in average cumulative IV iron intake that is selected from less than 100%. In some embodiments, the ferric citrate provides a mean reduction in average cumulative IV iron intake that is less than 90%. In some embodiments, the ferric citrate provides a mean reduction in average cumulative IV iron intake that is less than 80%. In some embodiments, the ferric citrate provides a mean reduction in average cumulative IV iron intake that is less than 70%. In some embodiments, the ferric citrate provides a mean reduction in average cumulative IV iron intake that is less than 60%. In some embodiments, the ferric citrate provides a mean reduction in average cumulative IV iron intake that is less than 50%. In some embodiments, the ferric citrate provides a mean reduction in average cumulative IV iron intake that is less than 40%. In some embodiments, the ferric citrate provides a mean reduction in average cumulative IV iron intake that is less than 30%. In some embodiments, the ferric citrate provides a mean reduction in average cumulative IV iron intake that is less than 20%. In some embodiments, the ferric citrate provides a mean reduction in average cumulative IV iron intake that is less than 10 %. The above boundaries are disclosed in this format for purposes of efficiency, and any of the above boundaries can be combined with any method, formulation, upper boundary as disclosed above, or combination thereof.

In some embodiments, the ferric citrate provides a mean reduction in average cumulative IV iron intake that is greater than 60%. In some embodiments, the ferric citrate provides a mean reduction in average cumulative IV iron intake that is greater than 70%. In some embodiments, the ferric citrate provides a mean reduction in average cumulative IV iron intake that is greater than %. In some embodiments, the ferric citrate provides a mean reduction in average cumulative IV iron intake that is greater than 90 %. The above boundaries are disclosed in this format for purposes of efficiency, and any of the above boundaries can be combined with any method, formulation, lower boundary as disclosed below, or combination thereof.

In some embodiments, CKD patients, such as ESRD patients, treated according to the methods disclosed herein experience maintenance of the amount of IV iron supplementation needed such that the total amount of IV iron supplementation received by the CKD patient remains substantially unchanged during administration of the ferric citrate.

### Erythropoiesis-Stimulating Agents

In addition to the means of controlling iron-deficiency anemia in CKD patients set forth above, CKD patient, particularly an ESRD patient, may also take one or more erythropoiesis-stimulating agents (ESAs) in an effort to control anemia. ESAs work by helping the body to produce red blood cells. These red blood cells are then released from the bone marrow into the bloodstream where they help maintain blood iron levels. Erythropoiesis-stimulating agents, commonly abbreviated as ESAs, are agents that are similar in structure and/or function to the cytokine erythropoietin, which stimulates red blood cell production (erythropoeisis) in the body. Typical ESAs, structurally and biologically, are similar to naturally occurring protein erythropoietin. Examples of commercially available ESAs include Erythropoietin (Epo), Epoetin alfa (Procrit/Epogen), Epoetin beta (NeoRecormon), Darbepoetin alfa (Aranesp), and Methoxy polyethylene glycol-epoetin beta (Mircera). The two ESAs presently approved for marketing in the U.S. are Epoetin alfa (Procrit, Epogen), and Darbepoietin alfa (Aranesp).

ESAs are commonly given to ESRD patients. These patients usually have lower hemoglobin levels because they can't produce enough erythropoietin. The side effects that occur most often with ESA use include: high blood pressure; swelling; fever; dizziness; nausea; and pain at the site of the injection, among others. In addition to these side effects, there are several safety issues that result from ESA use. ESAs increase the risk of venous thromboembolism (blood clots in the veins). ESAs can also cause hemoglobin to rise too high, which puts the patient at higher risk for heart attack, stroke, heart failure, and death.

CKD patients treated according to the methods disclosed herein may experience a decrease in the amount of ESAs needed to maintain hemoglobin levels. In some embodiments, CKD patients treated according to the methods disclosed herein experience a decrease in ESA use. The present disclosure provides ferric citrate for use in methods of reducing ESA use in a CKD patient, particularly an ESRD patient, the methods comprising orally administering ferric citrate to the patient, wherein the ferric citrate provides for a reduction in ESA use in the patient. The present disclosure provides ferric citrate for use in methods of reducing ESA use in an end-stage renal disease patient, the methods comprising orally administering ferric citrate to the patient at a dose of ferric iron ranging from 210 mg - 2,520 mg, wherein the ferric citrate provides for a reduction in ESA use in the patient, The ferric citrate is administered for a period of 52 weeks.

In some embodiments, the ferric citrate provides a decrease in median ESA intake is from 1 - 50 %. In some embodiments, the ferric citrate provides a decrease in median ESA intake is from 10 - 40 %. In some embodiments, the ferric citrate provides a decrease in median ESA intake is from 20 - 30 %. In some embodiments, the ferric citrate provides a decrease in median ESA intake selected from 20, 21, 22, 23, 24, 25, 26, 27, 28, 29 and 30 %. In some embodiments, the ferric citrate provides a decrease in median ESA intake selected from 27.0, 27.1, 27.2, 27.3, 27.4, 27.5, 27.6, 27.7, 27.9 and 28.0 %. In some embodiments, the ferric citrate provides a decrease in median ESA intake of 27.1 %. In some embodiments, the ferric citrate provides a decrease in median ESA intake of 27.1 % when administered over a period of 52 weeks.

In some embodiments, the ferric citrate provides a mean reduction in average cumulative IV iron intake that is greater than 20%. In some embodiments, the ferric citrate provides a mean reduction in average cumulative IV iron intake that is greater than 21%. In some embodiments, the ferric citrate provides a mean reduction in average cumulative IV iron intake that is greater than 22%. In some embodiments, the ferric citrate provides a mean reduction in average cumulative IV iron intake that is greater than 23%. In some embodiments, the ferric citrate provides a mean reduction in average cumulative IV iron intake that is greater than 24%. In some embodiments, the ferric citrate provides a mean reduction in average cumulative IV iron intake that is greater than 25%. In some embodiments, the ferric citrate provides a mean reduction in average cumulative IV iron intake that is greater than 26%. In some embodiments, the ferric citrate provides a mean reduction in average cumulative IV iron intake that is greater than 27%. In some embodiments, the ferric citrate provides a mean reduction in average cumulative IV iron intake that is greater than 28 %. In some embodiments, the ferric citrate provides a mean reduction in average cumulative IV iron intake that is greater than 29 %. The above boundaries are disclosed in this format for purposes of efficiency, and any of the above boundaries can be combined with any method, formulation, lower boundary as disclosed below, or combination thereof.

In some embodiments, the ferric citrate provides a mean reduction in average cumulative IV iron intake that is less than 30%. In some embodiments, the ferric citrate provides a mean reduction in average cumulative IV iron intake that is less than 29%. In some embodiments, the ferric citrate provides a mean reduction in average cumulative IV iron intake that is less than 28%. In some embodiments, the ferric citrate provides a mean reduction in average cumulative IV iron intake that is less than 27%. In some embodiments, the ferric citrate provides a mean reduction in average cumulative IV iron intake that is less than 26%. In some embodiments, the ferric citrate provides a mean reduction in average cumulative IV iron intake that is less than 25%. In some embodiments, the ferric citrate provides a mean reduction in average cumulative IV iron intake that is less than 24%. In some embodiments, the ferric citrate provides a mean reduction in average cumulative IV iron intake that is less than 23%. In some embodiments, the ferric citrate provides a mean reduction in average cumulative IV iron intake that is less than 22%. In some embodiments, the ferric citrate provides a mean reduction in average cumulative IV iron intake that is less than 21 %. The above boundaries are disclosed in this format for purposes of efficiency, and any of the above boundaries can be combined with any method, formulation, upper boundary as disclosed above, or combination thereof.

In some embodiments, CKD patients, particularly ESRD patients, treated according to the methods disclosed herein experience maintenance of the amount of ESAs needed to maintain hemoglobin levels such that the total amount of ESA use by the patient remains substantially unchanged during administration of the ferric citrate.

### Oral Iron Supplement

In some embodiments, the present disclosure provides an oral iron supplement comprising ferric citrate in an amount effective to increase iron absorption in CKD patients. In some embodiments, the present disclosure provides an oral iron supplement comprising ferric citrate in an amount effective to maintain iron stores in CKD patients. In some embodiments, the present disclosure provides an oral iron supplement comprising ferric citrate in an amount effective to improve one or more iron storage parameters in CKD patients. In some embodiments, the one or more iron storage parameters are selected from hematocrit, hemoglobin concentration (Hb), total iron-binding capacity (TIBC), transferrin saturation (TSAT), serum iron levels, liver iron levels, spleen iron levels, and serum ferritin levels. In some embodiments, the present disclosure provides an oral iron supplement comprising ferric citrate in an amount effective to treat iron deficiency in CKD patients. In some embodiments, the present disclosure provides an oral iron supplement comprising ferric citrate in an amount effective to treat anemia in CKD patients.

In some embodiments, the present disclosure provides an oral iron supplement comprising ferric citrate having a dose of ferric iron of 210 mg. In some embodiments, the oral iron supplements comprising ferric citrate can be administered so that the dose of ferric iron ranges from 210 mg - 2,520 mg.

The present disclosure provides ferric citrate for use in the manufacture of an oral iron supplement to increase iron absorption in CKD patients. The present disclosure provides ferric citrate for use in the manufacture of an oral iron supplement to maintain iron stores in CKD patients. The present disclosure provides ferric citrate for use in the manufacture of an oral iron supplement to improve one or more iron storage parameters in CKD patients. The one or more iron storage parameters are selected from hematocrit, hemoglobin concentration (Hb), total iron-binding capacity (TIBC), transferrin saturation (TSAT), serum iron levels, liver iron levels, spleen iron levels, and serum ferritin levels. The present disclosure provides ferric citrate for use in the manufacture of an oral iron supplement to treat iron deficiency in CKD patients. In some embodiments, the present disclosure provides ferric citrate for use in the manufacture of an oral iron supplement to treat anemia in CKD patients.

In some embodiments, the present disclosure provides ferric citrate for use in the manufacture of an oral iron supplement comprising a dose of ferric iron of 210 mg.

### Ferric Citrate

In various aspects, the present disclosure relates to the use of ferric citrate to reduce and/or control serum phosphorus levels, increase serum bicarbonate levels, improve one or more iron storage parameters (e.g., increase serum ferritin levels, increase transferrin saturation (TSAT), increase hemoglobin concentration) increase iron absorption, maintain iron stores, treat iron deficiency, treat anemia, reduce the need for IV iron and/or reduce the need for erythropoiesis-stimulating agents (ESAs) in CKD patients. In various aspects, the present disclosure relates to the use of pharmaceutical compositions comprising ferric citrate and a pharmaceutically acceptable binder to reduce and/or control serum phosphorus levels, increase serum bicarbonate levels, improve one or more iron storage parameters (e.g., increase serum ferritin levels, increase transferrin saturation (TSAT), increase hemoglobin concentration) increase iron absorption, maintain iron stores, treat iron deficiency, treat anemia, reduce the need for IV iron and/or reduce the need for erythropoiesis-stimulating agents (ESAs) in CKD patients.

Therefore, disclosed herein are preparations of ferric citrate and pharmaceutical compositions comprising the ferric citrate. In various embodiments, the ferric citrate preparations, and the pharmaceutical compositions comprising the ferric citrate preparations, meet certain dissolution, tableting and disintegration standards. In various aspects, the pharmaceutical compositions can include ferric citrate as the active ingredient and a binder. The pharmaceutical compositions also can include a lubricant and/or a disintegrant (which, in some embodiments, can be the same as the binder).

Certain embodiments of the ferric citrate preparations disclosed for use herein are also disclosed in U.S. Patent Nos. 7,767,851, 8,093,423, 8,299,298 and 8,338,642, and PCT Publication Nos. WO 2004/074444, WO 2007/022435, WO 2007/089571, WO 2007/089577 and WO 2011/011541. Certain embodiments of the ferric citrate preparations, however, are unique to this disclosure. The ferric citrate preparations disclosed herein display an enhanced BET active surface area compared to commercially available or chemical grade forms of ferric citrate. BET theory explains the physical adsorption of gas molecules onto a solid surface. The theory serves as the basis for the measurement of the specific surface area of a material. This theory allows the calculation of surface areas of materials in a very accurate manner and is thus capable of distinguishing differences between separate preparations of what would otherwise appear to be the same material. For example, activated carbon is a form of carbon that has been processed to make it extremely porous and thus to have a very large surface area. Activated carbon has been experimentally determined, using calculations derived from BET theory, to have a surface area of around 3000 m² g⁻¹. This surface area is significantly higher than the active surface areas of other preparations of carbon even though they are made of the same material.

In some embodiments, the ferric citrate preparations disclosed herein have a BET active surface area exceeding 16 m²/g. In some embodiments, the high purity ferric citrate preparations disclosed herein have a BET active surface area exceeding 20 m²/g. In some embodiments, the high purity ferric citrate preparations disclosed herein have a BET active surface area exceeding 25 m²/g. In some embodiments, the high purity ferric citrate preparations disclosed herein have a BET active surface area exceeding 30 m²/g. In some embodiments, the high purity ferric citrate preparations disclosed herein have a BET active surface area exceeding 35 m²/g. In some embodiments, the high purity ferric citrate preparations disclosed herein have a BET active surface area exceeding 40 m²/g. In some embodiments, the high purity ferric citrate preparations disclosed herein have a BET active surface area exceeding 45 m²/g. In some embodiments, the high purity ferric citrate preparations disclosed herein have a BET active surface area exceeding 50 m²/g. In some embodiments, the ferric citrate preparations disclosed herein have a BET active surface area ranging from 16.17 m²/g to 19.85 m²/g. In some embodiments, the ferric citrate preparations disclosed herein have a BET active surface area selected from 16.17 m²/g and 19.85 m²/g. In some embodiments, the ferric citrate preparations disclosed herein have a BET active surface area exceeding 27 m²/g. In some embodiments, the ferric citrate preparations disclosed herein have a BET active surface area ranging from 27.99 m²/g to 32.34 m²/g. In some embodiments, the ferric citrate preparations disclosed herein have a BET active surface area ranging from 28.5 m²/g to 31.5 m²/g. In some embodiments, the ferric citrate preparations disclosed herein have a BET active surface area selected from 27.99 m²/g, 28.87 m²/g and 32.34 m²/g. In some embodiments, the ferric citrate preparations disclosed herein have a BET active surface area selected from 28.5 m²/g, 29.1 m²/g, 30.6 m²/g and 31.5 m²/g. This is in sharp contrast to other preparations of ferric citrate such as chemical-grade preparations that are known and commercially available as of the filing date of this disclosure. Commercial grade preparations of ferric citrate have BET active surface areas that are substantially lower than the ferric citrate preparation of the present disclosure. Therefore, the ferric citrate preparations disclosed herein have a significantly larger surface area available for adsorption or chemical reactions, making the preparations of ferric citrate disclosed herein substantially more reactive than commercial preparations.

The BET active surface areas determined for five ferric citrate preparations produced by the methods disclosed in PCT Publication No. WO2004/074444 have been determined. Those BET active surface areas are displayed in Table 1, below, compared to the BET active surface area of commercial-grade preparations of ferric citrate:

**Table 1. BET active surface areas of various forms of ferric citrate**

| **Sample** | **Mean Dissolution Rates (mg/cm2/min)** | **BET Active Surface Area** |
|---|---|---|
| RFS-12-1 (sigma / commercially available) | 0.76 | 0.61 |
| RFS-12-2 (sigma / commercially available) | | |
| STM-134-1 (reference material 1) | 2.47 | 16.17 |
| STM-134-2 (reference material 2) | | |
| STM-182-1 (lab-scale 500 g batch 1) | 2.61 | 19.85 |
| STM-182-2 (lab-scale 500 g batch 2) | | |

The BET active surface areas determined for five ferric citrate preparations produced by the methods disclosed in PCT Publication No. WO2011/011541 have been determined. Those BET active surface areas are displayed in Table 2, below, compared to the BET active surface area of commercial-grade preparations of ferric citrate:

**Table 2. BET active surface areas**

| **Sample** | **BET Active Surface Area (m²/g)** |
|---|---|
| RFS-12-1 (sigma / commercially available) | 0.61 |
| RFS-12-2 (sigma / commercially available) | |
| Sample #10-1 (Pre-granulation(API+ProSolv))¹ | 27.99 |
| Sample #10-2 (Pre-granulation(API+ProSolv))² | 32.34 |
| Sample #11-1 (Pre-granulation(API+ProSolv))³ | 28.87 |
| Sample #11-2 (Pre-granulation(API+ProSolv))⁴ | |
| Sample #11-3 (Pre-granulation(API+ProSolv))⁵ | |

The BET active surface areas for four additional ferric citrate preparations produced by methods disclosed herein have also been determined. Those BET active surface areas are displayed in Table 3, below, compared to the BET active surface area of commercial-grade preparations of ferric citrate:
¹ From Example 10 of PCT Publication No. WO 2011/011541.
² From Example 10 of PCT Publication No. WO 2011/011541.
³ From Example 11 of PCT Publication No. WO 2011/011541.
⁴ From Example 11 of PCT Publication No. WO 2011/011541.
⁵ From Example 11 of PCT Publication No. WO 2011/011541.

**Table 3. BET active surface areas**

| **Sample** | **BET Active Surface Area (m²/g)** |
|---|---|
| RFS-12-1 (sigma / commercially available) | 0.61 |
| RFS-12-2 (sigma / commercially available) | |
| Batch No. 35102 | 30.6 |
| Batch No. 35103 | 29.1 |
| Batch No. 35105 | 31.5 |
| Batch No. 35106 | 28.5 |

The BET active surface areas of the embodiments of ferric citrate preparations disclosed in Tables 1, 2 and 3 are thus significantly higher than those of commercial grade ferric citrate.

Table 4 illustrates the assay content of ferric iron of the ferric citrate disclosed herein. The assay content of ferric iron represents the amount of ferric iron in each of the preparations of ferric citrate shown in Table 4. In some embodiments, the assay content of ferric iron is greater than or exceeds about 20% w/w. In some embodiments, the assay content of ferric iron is 21.2% w/w. In some embodiments, the assay content of ferric iron is 22.1% w/w. In some embodiments, the assay content of ferric iron is 22.4% w/w. In some embodiments, the assay content of ferric iron is between 21% w/w and 23% w/w.

**Table 4: Ferric Iron Content**

| **Batch** | **Material balance** | **+ Water** | **Revised Mat Bal. (mat bal+water)** | **Impurity Content** | **% Fe(III)** |
|---|---|---|---|---|---|
| A | 94.60 | 1.9 | 96.50 | 3.5 | 21.2 |
| B | 94.40 | 2.1 | 96.50 | 3.5 | 21.2 |
| C | 93.40 | 2.0 | 95.40 | 4.6 | 22.4 |
| D | 92.90 | 2.2 | 95.10 | 4.9 | 22.1 |

The ferric citrate disclosed herein is a complex of iron(III) and citric acid. In some aspects, the molar ratio of iron (III) to citric acid is from 1: 0.70 to 1: 0.78. In some aspects, the molar ratio of iron (III) to citric acid is from 1: 0.69 to 1: 0.87. In some aspects, the molar ratio of iron (III) to citric acid is from 1: 0.75 to 1: 1.10. In some aspects, the molar ratio of iron (III) to citric acid is from 1: 0.78 to 1: 0.95. In some aspects, the molar ratio of iron (III) to citric acid is from 1: 0.80 to 1: 0.92. In some aspects, the molar ratio of iron (III) to citric acid is from 1: 0.81 to 1: 0.91. In some aspects, the molar ratio of iron (III) to citric acid is from 1: 0.75 to 1: 1.15. In some aspects, the molar ratio of iron (III) to citric acid is from 1: 0.80 to 1: 1.10.

In some aspects, the molar ratio of iron (III) to water is from 1: 0.32 to 1: 0.42. In some aspects, the molar ratio of iron (III) to water is from 1: 0.32 to 1: 0.46. In some aspects, the molar ratio of iron (III) to water is from 1: 1.8 to 1: 3.2. In some aspects, the molar ratio of iron (III) to water is from 1: 1.8 to 1: 3.2. In some aspects, the molar ratio of iron (III) to water is from 1: 2.4 to 1: 3.1. In some aspects, the molar ratio of iron (III) to water is from 1: 2.7 to 1: 3.1.

The ferric citrate preparations disclosed herein are more soluble compared to commercially available or chemical grade forms of ferric citrate. In dissolution testing, the percentage of ferric citrate of the present disclosure dissolved within 5 minutes is 91% or more, within 15 minutes is 96% or more, within 30 minutes is 96% or more and within 60 minutes is 95% or more in dissolution testing conducted on the ferric citrate preparations in USP <711> vessels using Apparatus II. Table 5 illustrates dissolution testing data for four exemplary batches of ferric citrate according to the present disclosure. The particular standard used for the dissolution testing establishes a baseline of 100 so to the extent that a batch may have a dissolution greater than 100%, it is a dissolution rate relative to that standard.

**Table 5. Dissolution testing data**

| Batch | 5 minutes | 15 minutes | 30 minutes | 60 minutes |
|---|---|---|---|---|
| A | 101% | 102% | 101% | 101% |
| B | 101% | 102% | 102% | 102% |
| C | 97% | 97% | 97% | 97% |
| D | 91% | 96% | 96% | 95% |

Thus, in some embodiments, the percentage of ferric citrate dissolved within 15 minutes is 80% or more in dissolution testing conducted in USP <711> vessels using Apparatus II. In some embodiments, the percentage of ferric citrate dissolved within 15 minutes is 85% or more in dissolution testing conducted in USP <711> vessels using Apparatus II. In some embodiments, the percentage of ferric citrate dissolved within 15 minutes is 90% or more in dissolution testing conducted in USP <711> vessels using Apparatus II. In some embodiments, the percentage of ferric citrate dissolved within 15 minutes is 91% or more in dissolution testing conducted in USP <711> vessels using Apparatus II. In some embodiments, the percentage of ferric citrate dissolved within 15 minutes is 95% or more in dissolution testing conducted in USP <711> vessels using Apparatus II. In some embodiments, the percentage of ferric citrate dissolved within 15 minutes is 96% or more in dissolution testing conducted in USP <711> vessels using Apparatus II. In some embodiments, the percentage of ferric citrate dissolved within 15 minutes is 97% or more in dissolution testing conducted in USP <711> vessels using Apparatus II. In some embodiments, the percentage of ferric citrate dissolved within 15 minutes is 100% or more in dissolution testing conducted in USP <711> vessels using Apparatus II.

The ferric citrate preparations disclosed herein are more soluble compared to commercially available or chemical grade forms of ferric citrate. This increase in solubility of the ferric citrate preparations disclosed herein is believed to be a result of the unique, significantly large active surface area of the ferric citrate preparations disclosed herein. The intrinsic dissolution rate is defined as the dissolution rate of pure substances under the condition of constant surface area. The intrinsic dissolution rate and bioavailability of a drug substance is influenced by its solid state properties including: crystallinity, amorphism, polymorphism, hydration, solvation, particle size and particle surface area. The measured intrinsic dissolution rate is dependent on these solid-state properties and is typically determined by exposing a constant surface area of a material to an appropriate dissolution medium while maintaining constant temperature, stirring rate, and pH.

In some embodiments, the ferric citrate preparations disclosed herein have an intrinsic dissolution rate of greater than 2.28 mg/cm²/min. In some embodiments, the ferric citrate preparations disclosed herein have an intrinsic dissolution rate exceeding 2.28 mg/cm²/min. In some embodiments, the ferric citrate preparations disclosed herein have an intrinsic dissolution rate of 2.99 mg/cm²/min. In some embodiments, the ferric citrate preparations disclosed herein have an intrinsic dissolution rate ranging from 2.28 mg/cm²/min to 2.99 mg/cm²/min. In some embodiments, the ferric citrate preparations disclosed herein have an intrinsic dissolution rate selected from 2.28 mg/cm²/min and 2.99 mg/cm²/min. This is in sharp contrast to other preparations of ferric citrate such as chemical-grade preparations that are known and commercially available. Commercial grade preparations of ferric citrate have an intrinsic dissolution rate that is substantially lower than the ferric citrate preparation of the present disclosure. Therefore, the ferric citrate preparations disclosed herein have a significantly higher intrinsic dissolution rate, making the preparations of ferric citrate disclosed herein substantially more soluble than commercial preparations.

The intrinsic dissolution rate was determined for a preparation of ferric citrate produced according to the present disclosure. The mean intrinsic dissolution rate is displayed in Table 6, below, compared to the dissolution rate of a commercial-grade preparation of ferric citrate:

**Table 6. Intrinsic Dissolution Rates**

| **Sample** | **Mean Intrinsic Dissolution Rates (mg/cm²/min)** |
|---|---|
| RFS-12 (sigma/commercially available) | 0.83 |
| High Purity Ferric Citrate | 2.64 |

The intrinsic dissolution rate of the ferric citrate preparation disclosed in Table 6 is thus significantly higher than that of commercial grade ferric citrate.

### Methods of Manufacture

Exemplary methods of manufacture of preparations of ferric citrate provided by this disclosure are disclosed in U.S. Patent Nos. 7,767,851, 8,093,423, 8,299,298 and 8,338,642, and PCT Publication Nos. WO 2004/074444, WO 2007/022435, WO 2007/089571, WO 2007/089577 and WO 2011/011541.

### Modes of Administration

The ferric citrate disclosed herein may be advantageously used in human medicine. As disclosed herein, the ferric citrate disclosed herein is useful to reduce and/or control serum phosphorus levels, increase serum bicarbonate levels, improve one or more iron storage parameters (e.g., increase serum ferritin levels, increase transferrin saturation (TSAT), increase hemoglobin concentration) increase iron absorption, maintain iron stores, treat iron deficiency, treat anemia, reduce the need for IV iron and/or reduce the need for erythropoiesis-stimulating agents (ESAs) in CKD patients. The ferric citrate disclosed herein may also be advantageously used as an iron supplement. In various aspects, the ferric citrate disclosed herein can be administered orally. In some embodiments, the ferric citrate is administered in an oral dosage form. In some embodiments, the ferric citrate is administered in an oral tablet dosage form. In some embodiments, the tablet is in the form of a caplet.

When used to treat the above diseases and/or conditions, or when used as an iron supplement, the ferric citrate disclosed herein may be administered or applied singly, or in combination with other agents. The ferric citrate disclosed herein may also be administered or applied singly or in combination with other pharmaceutically active agents, including other agents known to reduce and/or control serum phosphorus levels, increase serum bicarbonate levels, improve one or more iron storage parameters (e.g., increase serum ferritin levels, increase transferrin saturation (TSAT), increase hemoglobin concentration) increase iron absorption, maintain iron stores, treat iron deficiency, treat anemia, reduce the need for IV iron and/or reduce the need for erythropoiesis-stimulating agents (ESAs) in CKD patients.

Ferric citrate for use in methods of treatment is disclosed above and include orally administering ferric citrate to the patient at a dose of ferric iron ranging from 210 mg - 2,520 mg. The ferric citrate disclosed herein can therefore be administered orally. In various aspects, the ferric citrate disclosed herein may be administered in an oral tablet dosage form that comprises 1 gram of ferric citrate and a dose of ferric iron of about 210 mg.

The ferric citrate disclosed herein serves to enhance the absorption of iron from the intestinal lumen and to enhance/maintain the storage of iron after absorption. It is believed that the enhanced absorption and storage of iron may be due to the presence of citrate in the ferric citrate administered to the CKD patient. While not wishing to be bound by any theory, some studies have shown that administration of iron in combination with citrate (the conjugate base of citric acid) serves to significantly increase (*e.g*., by several fold) the amount of iron absorbed from dietary sources (*see, e.g*., Ballot, et al., Br. J. Nutr. (1987) 57, 331-343; Gillooly, et al., Br. J. Nutr. (1983) 49, 331-342; Zhang, et al., Eur. J. Nutr. (2007) 46, 95-102; and Salovaara, et al., J. Agric. Food Chem. (2002) 50, 6233-6238).

The ferric citrate disclosed herein can be administered in some embodiments once per day, in some embodiments twice per day, in some embodiments three times per day, and in some embodiments more than twice per day. In various aspects, the ferric citrate may be administered in the form of a daily dose that is split up during the course of a single day. By way of example, a single daily dose of ferric citrate may be 6 grams and that 6 grams may be spread out over the course of the day such that 2 grams is taken in the morning, 2 grams in the afternoon, and the final 2 grams in the evening, for a total of 6 grams over the course of a day.

The ferric citrate disclosed herein can be used to reduce and/or control serum phosphorus levels, increase serum bicarbonate levels, improve one or more iron storage parameters (e.g., increase serum ferritin levels, increase transferrin saturation (TSAT), increase hemoglobin concentration) increase iron absorption, maintain iron stores, treat iron deficiency, treat anemia, reduce the need for IV iron and/or reduce the need for erythropoiesis-stimulating agents (ESAs) in CKD patients, while also reducing adverse drug effects associated with known forms of oral iron supplements (such as ferrous iron-containing supplements) and/or IV iron supplements.

### Pharmaceutical Compositions and Iron Supplements

Disclosed herein are ferric citrate-containing pharmaceutical compositions comprising the ferric citrate preparations disclosed herein and a binder. In some embodiments, the pharmaceutical compositions can be provided to CKD patients as iron supplements. In some embodiments, the pharmaceutical compositions can be provided to CKD patients as phosphate binders and/or to reduce and/or control serum phosphorus levels, increase serum bicarbonate levels, improve one or more iron storage parameters (e.g., increase serum ferritin levels, increase transferrin saturation (TSAT), increase hemoglobin concentration) increase iron absorption, maintain iron stores, treat iron deficiency, treat anemia, reduce the need for IV iron and/or reduce the need for erythropoiesis-stimulating agents (ESAs) in CKD patients. In various embodiments, the pharmaceutical compositions meet certain dissolution, tableting and/or disintegration standards. In various aspects, the pharmaceutical compositions can include ferric citrate as the active ingredient and a binder. The pharmaceutical compositions also can include a lubricant and/or a disintegrant (which, in some embodiments, can be the same as the binder). In some embodiments, the pharmaceutical compositions are oral tablet dosage forms.

Certain embodiments of the pharmaceutical compositions and oral tablet dosage forms provided by this disclosure are disclosed in PCT Publication No. WO 2011/011541. Other embodiments, however, are unique to this disclosure.

### Oral Tablet Dosage Forms and Oral Iron Supplements

In one aspect, the pharmaceutical compositions are tablets that include ferric citrate and a binder. As is used herein, a "tablet" is a material produced by compression force, such as with a tableting machine. In other embodiments the tablets can include ferric citrate, a binder, a lubricant and a disintegrant. In some embodiments, a single tablet comprises 1 gram of ferric citrate having a 210 mg dose of ferric iron. In some embodiments, the tablets can be used to reduce and/or control serum phosphorus levels, increase serum bicarbonate levels, improve one or more iron storage parameters (e.g., increase serum ferritin levels, increase transferrin saturation (TSAT), increase hemoglobin concentration) increase iron absorption, maintain iron stores, treat iron deficiency, treat anemia, reduce the need for IV iron and/or reduce the need for erythropoiesis-stimulating agents (ESAs) in CKD patients. In some embodiments, the tablets can be administered to CKD patients as oral iron supplements.

In some embodiments, the tablets and/or oral iron supplements can be characterized as highly drug loaded with the ferric citrate present in the tablets and/or oral iron supplements at values of greater than approximately 65% by weight of the formulation, greater than approximately 70% by weight of the formulation, greater than approximately 75% by weight of the formulation, greater than approximately 80% by weight of the formulation, greater than approximately 85% by weight of the formulation, greater than approximately 90% by weight of the formulation and as high as approximately 92% of the formulation. Intermediate values such as approximately 80% by weight ferric citrate, approximately 85% by weight ferric citrate and approximately 90% by weight ferric citrate also can be used in the ferric citrate tablets and/or oral iron supplements. The characteristics of the tablets and/or oral iron supplements produced at these highly loaded weight percentages are controlled by variables such as binder, binder amount, disintegrant, disintegrant amount, formulation method used (e.g., granulation, direct compression), tableting parameters, etc. Thus if a tablet and/or oral iron supplement is made and it has a slight amount of lamination or capping, by varying one or more of the above variables, the lamination or capping can be corrected.

In various embodiments, the tablets and/or oral iron supplements contains one or more components selected from among one or more binders, one or more lubricants, and one or more disintegrants.

The binder can be any binder known in the art. Without limitation, examples of the binder can include one or more of hydroxypropyl cellulose (HPC), hydroxypropylmethyl cellulose (HPMC), sodium alginate, alginic acid, guar gum, acacia gum, xanthan gum, carbolpol, cellulose gum (carboxy methyl cellulose), ethyl cellulose, maltodextrin, PVP/VA, povidone, microcrystalline cellulose, starch, partially or fully pregelatinized starch, and methyl cellulose. The maltodextrin, PVP/VA, and methyl cellulose function as immediate release binders when used in the ferric citrate tablets and/or oral iron supplements.

It also should be understood that combinations of binders can be used to control and vary the effect of the binder. For example, a binder system can be made up of hydroxypropyl cellulose and polyvinyl pyrrolidone (povidone) with or without microcrystalline cellulose. One or both of the hydroxypropyl cellulose and povidone can be replaced with pregelatinized starch.

In various aspects, the tablets and/or oral iron supplements can include a lubricant. As an example of a lubricant for the ferric citrate tablets and/or oral iron supplements, magnesium stearate, calcium stearate, sodium stearyl fumarate and combinations can be used. Other suitable lubricants include one or more of polyethylene glycol (molecular weight above 3350), sodium lauryl sulfate, talc, mineral oil, leucine, and poloxamer.

In various aspects, the tablets and/or oral iron supplements can include a disintegrant. The disintegrant can be included in the tablets and/or oral iron supplements. The disintegrant can be the same as or different from the binder. By way of example and not limitation, microcrystalline cellulose has both binder and disintegrant properties and microcrystalline cellulose can be used as the sole binder/disintegrant in the tablets and/or oral iron supplements. Examples of other suitable disintegrants include croscarmellose sodium, crospovidone, sodium starch glycolate, and starch.

The binder can be present in the tablets and/or oral iron supplements in an amount ranging from approximately 4.5% by weight to approximately 30% by weight. The disintegrant can be present in the tablets and/or oral iron supplements in an amount ranging from approximately 1.5% by weight to approximately 15% by weight. In various embodiments, some non-starch disintegrants are often used at lower weight percents, e.g., as low as 0.25% and thus the disintegrant present in the tablets and/or oral iron supplements can be as low as 0.25% in some conditions.

The lubricant can be present in the tablets and/or oral iron supplements in an amount ranging from approximately 0.5% by weight to approximately 3% by weight. It should be understood that some components, such as microcrystalline cellulose, can function with both disintegrant and binder properties.

The weight of individual tablets and/or oral iron supplements can depend upon the final dosage to be produced; e.g. 125mg, 250mg, 500mg, 667mg, 750mg and 1,000mg of ferric citrate. In some embodiments, the tablets comprise 1 gram of ferric citrate and therefore a dose of 210 mg of ferric iron.

In various embodiments, tablets and/or oral iron supplements are coated to a weight gain of approximately 2% to 5% using an Opadry suspension or equivalent in a perforated pan coater. Calcium stearate and Opadry purple can be replaced with or used with a different lubricant or coating system, respectively.

In other variations, the tablets and/or oral iron supplements have reduced water content. In one embodiment, the water content of the tablet, as measured by LOD %, is less than 20%. In another embodiment, the water content of the tablet, as measured by LOD %, is less than 19%. In another embodiment, the water content of the tablet, as measured by LOD %, is less than 18%. In another embodiment, the water content of the tablet, as measured by LOD %, is less than 17%. In another embodiment, the water content of the tablet, as measured by LOD %, is less than 16%. In another embodiment, the water content of the tablet, as measured by LOD %, is less than 15%. In another embodiment, the water content of the tablet, as measured by LOD %, is less than 14%. In another embodiment, the water content of the tablet, as measured by LOD %, is less than 13%. In another embodiment, the water content of the tablet, as measured by LOD % is less than 12%. In another embodiment, the water content as measured by LOD % is less than 11%. In another embodiment, the water content as measured by LOD % is less than 10%. In another embodiment, the water content of the tablet, as measured by LOD %, is less than 9%. In another embodiment, the water content of the tablet, as measured by LOD %, is less than 8%. In another embodiment, the water content of the tablet, as measured by LOD %, is less than 7%. In another embodiment, the water content of the tablet, as measured by LOD %, is less than 6%. In another embodiment, the water content of the tablet, as measured by LOD %, is less than 5%.

LOD (loss on drying) is a method of thermogravimetric moisture determination. In thermogravimetric processes, the moisture of a material includes substances that volatilize during warming, and therefore contribute to the material's loss of mass. Alongside water this may also include alcohol or decomposition products. When using thermogravimetric measurement methods (drying using infrared, halogen, microwaves or ovens) no distinction is made between water and other volatile components.

In some embodiments, the tablets and/or oral iron supplements comprise an amount of ferric citrate selected from approximately 1000 mg, approximately 667 mg, approximately 500 mg, approximately 250 mg and approximately 125 mg. In some embodiments, the tablets and/or oral iron supplements comprise 1 gram (1000mg) of ferric citrate. In some embodiments, the tablets and/or oral iron supplements comprise 1 gram of ferric citrate containing approximately 210 mg of ferric iron.

In some embodiments, the tablets and/or oral iron supplements comprise 1.3 grams of ferric citrate. In some embodiments, the tablets and/or oral iron supplements comprise 1.5 grams of ferric citrate. In some embodiments, the tablets and/or oral iron supplements comprise 1.6 grams of ferric citrate. In some embodiments, the tablets and/or oral iron supplements comprise an amount of ferric citrate selected from 100mg, 125mg, 150mg, 175mg, 200mg, 225mg, 250mg, 275mg, 300mg, 325mg, 350mg, 375mg, 400mg, 425mg, 450mg, 475mg, 500mg, 525mg, 550mg, 575mg, 600mg, 625mg, 650mg, 675mg, 700mg, 725mg, 750mg, 775mg, 800mg, 825mg, 850mg, 875mg, 900mg, 925mg, 950mg, 975mg, 1000mg, 1025mg, 1050mg, 1075mg, 1100mg, 1125mg, 1150mg, 1175mg, 1200mg, 1225mg, 1250mg, 1275mg, 1300mg, 1325mg, 1350mg, 1375mg, 1400mg, 1425mg, 1450mg, 1475mg, 1500mg, 1525mg, 1550mg, 1575mg, 1600mg, 1625mg, 1650mg, 1675mg, 1700mg, 1725mg, 1750mg, 1775mg, 1800mg, 1825mg, 1850mg, 1875mg, 1900mg, 1925mg, 1950mg, 1975mg and 2000mg.

In some embodiments, the tablets and/or oral iron supplements comprise between approximately 65 wt% and 92 wt% ferric citrate; between approximately 4.5 wt% and 30 wt% binder; and between 0.5 wt% and 3 wt% lubricant. In some embodiments, the lubricant is selected from one or more of magnesium stearate, calcium stearate, and sodium stearyl fumarate.

In some embodiments, the tablets and/or oral iron supplements comprise 65 % by weight to 92 % by weight of ferric citrate and 4.5 % by weight to 30 % by weight of a binder, wherein the mean surface area to mass ratio of said tablet is equal to or greater than 1 m² per gram, and wherein the LOD % water of the tablet is less than 20% water w/w. In some embodiments, the mean surface area to mass ratio of the tablets and/or oral iron supplements can be equal to or greater than 5 m² per gram. In some embodiments, the mean surface area to mass ratio of the tablets and/or oral iron supplements is equal to or greater than 10 m² per gram. In some embodiments, the tablets and/or oral iron supplements comprise at least 70 weight percent ferric citrate. In some embodiments, the tablets and/or oral iron supplements comprise at least 80 weight percent ferric citrate. In some embodiments, the tablets and/or oral iron supplements comprise at least 90 weight percent ferric citrate. In some embodiments, the binder comprises one or more of hydroxypropyl cellulose (HPC), hydroxypropylmethyl cellulose (HPMC), sodium alginate, alginic acid, guar gum, acacia gum, xanthan gum, carbolpol, cellulose gum (carboxymethyl cellulose), ethyl cellulose, maltodextrin, PVP/VA, povidone, microcrystalline cellulose, starch (partially or fully pregelatinized starch) and methyl cellulose. In some embodiments, the LOD % water of the tablets and/or oral iron supplements is less than 15% water w/w. In some embodiments, the LOD % water of the tablets and/or oral iron supplements is less than 10% water w/w. In some embodiments, the tablets and/or oral iron supplements further comprise a disintegrant selected from one or more of microcrystalline cellulose, croscarmellose sodium, crospovidone, sodium starch glycolate, and starch. In some embodiments, the tablets and/or oral iron supplements further comprise a lubricant selected from one or more of magnesium stearate, calcium stearate, and sodium stearyl fumarate. In some embodiments, the tablets and/or oral iron supplements comprise between 0.5% and 3% lubricant. In some embodiments, the binder comprises pregelatinized starch. In some embodiments, the lubricant comprises calcium stearate and sodium stearyl fumarate. In some embodiments, at least 80% of the ferric citrate in the tablets and/or oral iron supplements is dissolved in a time less than or equal to 60 minutes as measured by test method USP <711>. In some embodiments, the tablets and/or oral iron supplements comprise approximately 1000 mg of ferric citrate. In some embodiments, the tablets and/or oral iron supplements comprise approximately 667 mg of ferric citrate. In some embodiments, the tablets and/or oral iron supplements comprise approximately 500 mg of ferric citrate.

Table 7 provides a formulation for a ferric citrate tablet and/or oral iron supplement according to one embodiment of the present disclosure:

**Table 7. Formulation for a Ferric Citrate Tablet and/or Oral Iron Supplement**

| **Material Description** | **Theoretical kg/Batch** | **% w/w** |
|---|---|---|
| Ferric Citrate | 14.89 | 87.6 |
| Pregelatinized Starch | 1.70 | 10.0 |
| Calcium Stearate | 0.406 | 2.4 |
| Purified Water | 15.30* | N/A* |
| **Core Tablet Total** | 17.00 | 100.0 |
| Opadry Purple 03K100000 | 0.51 | 15.0 |
| Purified Water | 2.89* | 85.0* |
| **Coated Tablet Total** | 17.5 | 100.0 |

| | | |
|---|---|---|
| * - Purified water is removed during a drying phase in the manufacturing process | | |

Table 8 provides a formulation for a ferric citrate tablet and/or oral iron supplement according to one embodiment of the present disclosure:

**Table 8:**

| **Material Description** | **Target kg/Batch** | **Theoretical 100 kg/Lot** | **% w/w Individual** | **% w/w Coated Tablet** |
|---|---|---|---|---|
| Ferric Citrate | 14.9 | 80.0 - 90.0 | 80.0 - 90.0 | 76.2 - 88.2 |
| Pregelatinized Starch | 1.7 | 8.0 - 15.0 | 8.0 - 15.0 | 7.6 - 14.7 |
| Calcium Stearate (1) | 0.4 | 1.0 - 3.0 | 1.0 - 3.0 | 0.9 - 2.9 |
| OR - Sodium Stearyl Fumarate (1) | 0.4 | 2.0 - 3.0 | 2.0 - 3.0 | 1.9 - 2.9 |
| Purified Water | 15.3* | 72.0-135.0* | * | * |
| **Core Tablet Total** | 17.0 | 100.0 | 100.0 | N/A* |
| Opadry Purple | 0.9 | 5.3 | 15.0 | 2.0 - 5.0 |
| Purified Water | 5.1* | 30.0* | 85.0* | N/A* |
| **Coated Tablet Total** | 17.5 to 17.9 | 35.3 | 100.0 | 100.0 |

| | | | | |
|---|---|---|---|---|
| (1) - use either calcium stearate or sodium stearyl fumarate as lubricant * - Purified water is removed | | | | |

Table 9 provides a formulation for a ferric citrate tablet and/or oral iron supplement according to one embodiment of the present disclosure:

**Table 9:**

| **Material Description** | **Target kg/Batch** | **% w/w Individual** |
|---|---|---|
| Ferric Citrate | 14.89 | 87.6 |
| Pregelatinized Starch | 1.70 | 10.0 |
| Calcium Stearate (1) | 0.406 | 2.4 |
| Purified Water | 15.30 | N/A |
| **Core Tablet Total** | 17.00 | 100.0 |
| Opadry Purple | 0.51 | 15.0 |
| Purified Water | 2.89 | 85.0 |
| **Coated Tablet Total** | 17.5 | 100.0 |

Table 10 provides a formulation for a ferric citrate tablet and/or oral iron supplement according to one embodiment of the present disclosure:

**Table 10:**

| **Material / Component** | **Formula Composition % w/w** |
|---|---|
| Ferric Citrate | 70.0 to 99.0 |
| Starch | 0.0 to 30.0 |
| Microcrystalline Cellulose | 0.0 to 30.0 |
| Polyvinylpyrrolidone | 0.0 to 30.0 |
| Calcium Stearate | 0.0 to 3.0 |
| Sodium Stearyl Fumarate | 0.0 to 3.0 |
| Purified Water | N/A* |
| **Core Caplet Total** | 100.0 |
| Film coating | 0.0 to 5.0 |
| Purified Water | N/A* |
| **Coated Caplet Total** | 100.0 |

| | |
|---|---|
| * The purified water is removed. | |

Table 11 provides a formulation for a ferric citrate tablet and/or oral iron supplement according to one embodiment of the present disclosure:

**Table 11:**

| **Material** | **Weight mg ± 10%** |
|---|---|
| Ferric Citrate | 1,500 |
| Starch | 150 |
| Microcrystalline Celluose | 0 |
| Polyvinylpyrrolidone | 0 |
| Calcium Stearate | 16 |
| Sodium Stearyl Fumarate | 0 |
| Purified Water | N/A* |
| **Core Caplet Total** - **mg** | 1,666 |
| Film coating | 50 |
| Purified Water | N/A* |
| **Coated Caplet Total** - **mg** | 1,766 |

| | |
|---|---|
| * The purified water is removed. | |

### Dosing

The tablets and/or oral iron supplements disclosed herein can be made to accommodate a number of doses of ferric citrate. The weight of individual tablets and/or oral iron supplements can depend upon the final dosage to be produced; e.g., 125mg, 250mg, 500mg, 667mg, 750mg and 1,000mg of ferric citrate per tablet. In various aspects, the ferric citrate is provided in a tablet dosage form comprising 1 gram of ferric citrate containing approximately 210 mg of ferric iron. The number of tablets and/or oral iron supplements administered can be adjusted to conform to the desired amount of ferric citrate to be administered. For example, if a CKD patient is directed to take 4 grams of ferric citrate daily in a single dose, the CKD patient may take 4 tablets and/or oral iron supplements, each comprising 1 gram of ferric citrate, or may take 8 tablets and/or oral iron supplements, each comprising 500mg of ferric citrate.

In some embodiments, a daily dose of ferric citrate administered to CKD patients can be from 1 gram - 18 grams, at a dose of ferric iron ranging from 210 mg - 3,780 mg. In some embodiments, one or more tablets comprising 1 gram of ferric citrate, each tablet having a dose of ferric iron of 210 mg, is/are administered to reduce and/or control serum phosphorus levels, increase serum bicarbonate levels, improve one or more iron storage parameters (e.g., increase serum ferritin levels, increase transferrin saturation (TSAT), increase hemoglobin concentration) increase iron absorption, maintain iron stores, treat iron deficiency, treat anemia, reduce the need for IV iron and/or reduce the need for erythropoiesis-stimulating agents (ESAs) in CKD patients.

In some embodiments, the ferric citrate is administered at a daily dose of 1 tablet per day, the tablet comprising 1 gram of ferric citrate containing 210 mg of ferric iron, for a total daily dose of 1 gram of ferric citrate and 210 mg ferric iron. In some embodiments, the ferric citrate is administered at a daily dose of 2 tablets per day, each tablet comprising 1 gram of ferric citrate containing 210 mg of ferric iron, for a total daily dose of 2 grams of ferric citrate and 420 mg ferric iron. In some embodiments, the ferric citrate is administered at a daily dose of 3 tablets per day, each tablet comprising 1 gram of ferric citrate containing 210 mg of ferric iron, for a total daily dose of 3 grams of ferric citrate and 630 mg ferric iron. In some embodiments, the ferric citrate is administered at a daily dose of 4 tablets per day, each tablet comprising 1 gram of ferric citrate containing 210 mg of ferric iron, for a total daily dose of 4 grams of ferric citrate and 840 mg ferric iron. In some embodiments, the ferric citrate is administered at a daily dose of 5 tablets per day, each tablet comprising 1 gram of ferric citrate containing 210 mg of ferric iron, for a total daily dose of 5 grams of ferric citrate and 1,050 mg ferric iron. In some embodiments, the ferric citrate is administered at a daily dose of 6 tablets per day, each tablet comprising 1 gram of ferric citrate containing 210 mg of ferric iron, for a total daily dose of 6 grams of ferric citrate and 1,260 mg ferric iron. In some embodiments, the ferric citrate is administered at a daily dose of 7 tablets per day, each tablet comprising 1 gram of ferric citrate containing 210 mg of ferric iron, for a total daily dose of 7 grams of ferric citrate and 1,470 mg ferric iron. In some embodiments, the ferric citrate is administered at a daily dose of 8 tablets per day, each tablet comprising 1 gram of ferric citrate containing 210 mg of ferric iron, for a total daily dose of 8 grams of ferric citrate and 1,680 mg ferric iron. In some embodiments, the ferric citrate is administered at a daily dose of 9 tablets per day, each tablet comprising 1 gram of ferric citrate containing 210 mg of ferric iron, for a total daily dose of 9 grams of ferric citrate and 1,890 mg ferric iron. In some embodiments, the ferric citrate is administered at a daily dose of 10 tablets per day, each tablet comprising 1 gram of ferric citrate containing 210 mg of ferric iron, for a total daily dose of 10 grams of ferric citrate and 2,100 mg ferric iron. In some embodiments, the ferric citrate is administered at a daily dose of 11 tablets per day, each tablet comprising 1 gram of ferric citrate containing 210 mg of ferric iron, for a total daily dose of 11 grams of ferric citrate and 2,310 mg ferric iron. In some embodiments, the ferric citrate is administered at a daily dose of 12 tablets per day, each tablet comprising 1 gram of ferric citrate containing 210 mg of ferric iron, for a total daily dose of 12 grams of ferric citrate and 2,520 mg ferric iron. In some embodiments, the ferric citrate is administered at a daily dose of 13 tablets per day, each tablet comprising 1 gram of ferric citrate containing 210 mg of ferric iron, for a total daily dose of 13 grams of ferric citrate and 2,730 mg ferric iron. In some embodiments, the ferric citrate is administered at a daily dose of 14 tablets per day, each tablet comprising 1 gram of ferric citrate containing 210 mg of ferric iron, for a total daily dose of 14 grams of ferric citrate and 2,940 mg ferric iron. In some embodiments, the ferric citrate is administered at a daily dose of 15 tablets per day, each tablet comprising 1 gram of ferric citrate containing 210 mg of ferric iron, for a total daily dose of 15 grams of ferric citrate and 3,150 mg ferric iron. In some embodiments, the ferric citrate is administered at a daily dose of 16 tablets per day, each tablet comprising 1 gram of ferric citrate containing 210 mg of ferric iron, for a total daily dose of 16 grams of ferric citrate and 3,360 mg ferric iron. In some embodiments, the ferric citrate is administered at a daily dose of 17 tablets per day, each tablet comprising 1 gram of ferric citrate containing 210 mg of ferric iron, for a total daily dose of 17 grams of ferric citrate and 3,570 mg ferric iron. In some embodiments, the ferric citrate is administered at a daily dose of 18 tablets per day, each tablet comprising 1 gram of ferric citrate containing 210 mg of ferric iron, for a total daily dose of 18 grams of ferric citrate and 3,780 mg ferric iron.

### EXAMPLES

The following example describes in detail the use of the ferric citrate disclosed herein. It will be apparent to those skilled in the art that many modifications, both to materials and methods, may be practiced without departing from the scope of the disclosure.

### Example 1 (Reference example)

### A Three-Period, 58-Week Trial Of Ferric Citrate As A Phosphate Binder In Patients With End-Stage Renal Disease (ESRD) On Dialysis

The primary objectives of this trial were as follows:
1. To determine the long-term safety over 52 weeks of up to twelve (12) caplets/day of KRX-0502 (ferric citrate) in patients with end-stage renal disease undergoing either hemodialysis or peritoneal dialysis.
2. To determine the efficacy of KRX-0502 (ferric citrate) in a four-week, randomized, open-label, placebo-controlled Efficacy Assessment Period.

### Study Rationale

Previous clinical trials have demonstrated the ability of ferric citrate to lower serum phosphorus levels in patients with ESRD who are on thrice-weekly hemodialysis. These trials used a maximum of approximately 12 g/day of ferric citrate for four weeks.

This clinical trial determined the long-term safety of ferric citrate in controlling and managing serum phosphorus levels over a 56-week treatment period when compared to an active control for 52 weeks in the Safety Assessment Period and to placebo in a randomized, open-label, placebo-controlled four-week Efficacy Assessment Period.

### Study Design

This trial was a three-period, multicenter, safety and efficacy clinical trial. The first period was a two-week washout (the Washout Period), the second period was a 52-week randomized, open-label, active control safety assessment (the Safety Assessment Period), and the third period was a four-week, randomized, open-label, placebo-controlled, efficacy assessment (the Efficacy Assessment Period) in only patients randomized to treatment with ferric citrate during the Safety Assessment Period.

Period 1 (Washout Period). Patients were washed out from their current phosphate binder for up to approximately two weeks. Only patients who achieve a serum phosphorus ≥6.0 mg/dL during the Washout Period were moved into the Safety Assessment Period. Patients who did not achieve a serum phosphorus ≥6.0 mg/dL during washout were screen failures.

Period 2 (Safety Assessment Period). Following washout, patients were randomized 2:1 to either the ferric citrate group or an active-control group of either calcium acetate, sevelamer carbonate, or any combination of calcium acetate and sevelamer carbonate at the discretion of the PI and/or patient. Both ferric citrate and the active-control medications were provided by the sponsor. Patients were followed on their randomized assignment for safety assessments over 52 weeks. If a patient was ≥ 80% compliant with 12 caplets/day of ferric citrate or 12 pills/day of calcium acetate and/or sevelamer carbonate at least 2 visits in a row, and had a serum phosphorus > 8.0 mg/dL, the patient was considered a treatment failure and stopped study drug but continued to complete all trial visits. The ferric citrate or active-control drug was stopped and the patient returned to the care of their primary nephrologist, but continued to be followed for all trial visits and outcomes.

Period 3 (Efficacy Assessment Period). Following the Safety Assessment Period, those patients randomized to treatment with ferric citrate entered a four-week, randomized, open-label, placebo-controlled Efficacy Assessment Period. Patients entering the Efficacy Assessment Period were re-randomized 1:1 to treatment with ferric citrate or placebo.

A Dietician provided a study-supplied list of Vitamin D-rich foods to the patient either during the Washout Period or at the Randomization Visit and instructed the patient to keep their diet consistent in Vitamin D-rich food throughout the trial as much as possible. Within 30 days before the start of the Efficacy Assessment Period, the Dietician again reviewed the list of Vitamin D-rich foods with the patient and reminded the patient to try to keep their diet consistent in terms of Vitamin D-rich foods until the end of the trial, if possible. The Dietician was blinded as to assignment to ferric citrate or placebo during the Efficacy Assessment Period.

Laboratory measurements were conducted throughout the study to assess safety and efficacy. The dose and specific IV iron preparation administered (if necessary) were at the discretion of the PI. Oral iron therapy was not permitted. Calcium-containing drugs were not permitted if given within two hours of food ingestion (calcium-containing drugs were permitted two hours or more prior to or following food ingestion or at bedtime for the purpose of raising the serum calcium). No Vitamin C supplements were permitted. Patients were allowed to take daily water soluble vitamins that include a small amount of Vitamin C (e.g., Centrum, Nephrocaps, Renaphro), but those patients were instructed to take them two hours or more prior to or following food ingestion or at bedtime. IV iron therapy was not permitted if the ferritin level is > 1000 micrograms/L or the TSAT is > 30%. If it was deemed in the patient's best interest to receive IV iron outside these parameters, the Clinical Coordinating Center (CCC) was consulted, and when approved and documented, was not considered a protocol exception.

### Study Duration

The duration of the trial was approximately 18 to 24 months, with approximately six to eight months allocated for patient Screening, Washout Period, and Randomization, 12 months for the Safety Assessment Period, and one (1) month for the Efficacy Assessment Period.

### Study Population

ESRD patients on thrice-weekly hemodialysis or on peritoneal dialysis for at least three months prior to the Screening Visit (Visit 0) who were currently taking ≥3 and ≤18 pills/day of calcium acetate, calcium carbonate, lanthanum carbonate, and/or sevelamer (carbonate or hydrochloride or sevelamer powder equivalent to sevelamer tablets), or any other agent serving as a phosphate binder, or any combination of these agents were eligible for enrollment. It was anticipated that there would be approximately 20 to 40 centers in the United States and approximately 5 to 10 centers in Israel. Up to approximately 775 patients were screened to randomize approximately 350 patients to the ferric citrate group or active-control group. Each of approximately 25 to 50 sites were asked to randomize no more than approximately 35 patients.

### Inclusion criteria:

- Males or non-pregnant, non-breast-feeding females
- Age ≥ 18 years
- On thrice-weekly hemodialysis or on peritoneal dialysis for at least the previous three months prior to Screening Visit (Visit 0)
- Serum phosphorus levels ≥2.5 mg/dL and ≤8.0 mg/dL at Screening Visit (Visit 0)
- Serum phosphorus ≥6.0 mg/dL during the Washout Period (Visits 2 or 3)
- Taking 3 to 18 pills/day of calcium acetate, calcium carbonate, lanthanum carbonate, and/or sevelamer (carbonate or hydrochloride or equivalent sevelamer powder) or any other agent serving as a phosphate binder, or any combination of these agents as reported by the patient at Screening Visit (Visit 0)
- Serum ferritin <1000 micrograms/L and TSAT < 50% at the Screening Visit (Visit 0)
- Willing to be discontinued from current phosphate binder and randomized to ferric citrate or active-control group
- Willing and able to give informed consent
- Life expectancy >1 year

### Exclusion Criteria:

- Parathyroidectomy within six months prior to Screening Visit (Visit 0)
- Actively symptomatic gastrointestinal bleeding or inflammatory bowel disease
- Serum phosphorus levels ≥10.0 mg/dL documented in all of the three monthly laboratories (done routinely in the dialysis unit) in the 3 months prior to the Screening Visit (Visit 0)
- History of malignancy in the last five years (treated cervical or non-melanomatous skin cancer may be permitted if approved by the CCC)
- Absolute requirement for oral iron therapy
- Absolute requirement for Vitamin C (multivitamins [Nephrocaps, Renaphro, etc.] allowed)
- Absolute requirement for calcium-, magnesium-, or aluminum-containing drugs with meals
- Intolerance to oral iron-containing products
- Intolerance to orally administered calcium acetate and sevelamer carbonate

### Study Drug

KRX-0502 (ferric citrate) was the drug under investigation in this study. The drug was administered as caplets, each caplet comprising 1 gram (1,000 mg) of ferric citrate containing approximately 210 mg of ferric iron.

### Study Drug Administration

The target goal for serum phosphorus was 3.5 to 5.5 mg/dL.

Ferric citrate, active control, and placebo were considered study drugs. Eligible patients with a serum phosphorus level ≥6.0mg/dL after the Washout Period were randomized in a 2:1 ratio to the ferric citrate group or the active-control group. For patients randomized to ferric citrate, the starting dose was 6 caplets/day. For patients randomized to the active-control group, the starting dose of phosphate binder was the last dose that was administered immediately prior to the start of the Washout Period (if the patient remained on the same phosphate binder) or at the discretion of the PI, guided by the package insert, if the patient changed binders. However, for patients whose previous dose of phosphate binder exceeded 12 pills/day, if randomized to the active-control group, their starting dose of active-control drug was at the discretion of the PI, but will not exceed 12 pills/day. Calcium acetate 667 mg capsules and sevelamer carbonate 800 mg tablets were used and were supplied by Keryx Biopharmaceuticals, Inc. (Keryx) for the duration of the trial.

Serum phosphorus and calcium were checked at Visit 5 (Week 1), and every two weeks during the first 12 weeks after Visit 4 (Randomization Visit), and monthly for the rest of the Safety Assessment Period. During the Efficacy Assessment Period, serum phosphorus and calcium were drawn weekly. These values guided study drug administration. While on study drug, the use of other phosphate binders was not permitted. Dose adjustments in ferric citrate were guided by a titration schedule. The titration of calcium acetate and sevelamer carbonate throughout the 52-week Safety Assessment Period were according to the current package inserts for these agents and/or at the discretion of the site PI.

Patients took study drug orally with or within one hour of meals or snacks. Patients were instructed not to take the study drug if greater than one hour has passed since the ingestion of their meals or snacks. The PI or designee at each site dispensed the study drug to the patient and instructed the patient on how to administer it. It was recognized that some patients required a different distribution in pills in a given day due to snacks or missed meals. If the patient was receiving the total number of pills per day required by protocol in any distribution with meals, there was no need for approval by the CCC (for example, a patient on a starting dose of ferric citrate 6 g/day may take 1 caplet with breakfast, 1 with a snack, 2 with lunch, and 2 with dinner).

### Laboratory Assessments

For patients on hemodialysis, blood samples were obtained pre-dialysis on the second or third dialysis session of the week, if possible. For patients who are on hemodialysis who dialyze on Monday, Wednesday or Friday, all blood samples were drawn pre-dialysis on Wednesday or Friday, if possible. For patients who dialyze on Tuesday, Thursday or Saturday, all blood samples were drawn pre-dialysis on Thursday or Saturday, if possible. These collection methods were allowed to be different for sites in Israel. The total amount of blood collected from each patient for trial-related analyses was approximately 15 ml per visit.

For patients who were on peritoneal dialysis, blood samples were collected either at the dialysis unit or the clinic as per the study protocols.

Serum phosphorus and calcium were performed at Screening (Visit 0); weekly during the Washout Period after Visit 1 (Week -2); at Visit 4 (Randomization Visit); at Visits 5 (Week 1), 6 (Week 2), 7 (Week 4), 8 (Week 6), 9 (Week 8), 10 (Week 10), 11 (Week 12), 12 (Week 16), 13 (Week 20), 14 (Week 24), 15 (Week 28), 16 (Week 32), 17 (Week 36), 18 (Week 40), 19 (Week 44), 20 (Week 48), and 21 (Week 52) of the 52-week Safety Assessment Period; and at Visits 22 (Week 53), 23 (Week 54), 24 (Week 55) and 25 (Week 56) of the Efficacy Assessment Period.

Complete Blood Count (CBC) (white blood cell [WBC] count, white blood cell types [WBC differential], red blood cell [RBC] count, hematocrit [HCT], hemoglobin [Hgb], red blood cell indices, platelet [thrombocyte] count) was done at the Randomization Visit (Visit 4); at Visits 11 (Week 12), 14 (Week 24), 17 (Week 36), 20 (Week 48), and 21 (Week 52) of the 52-week Safety Assessment Period; and at Visit 25 (Week 56) of the Efficacy Assessment Period.

Complete Chemistry Profile (sodium, potassium, chloride, blood urea nitrogen (BUN), creatinine, glucose [random], aspartate aminotransferase [AST], alanine aminotransferase [ALT], alkaline phosphate [ALP], total bilirubin, total protein, albumin, and albumin-adjusted calcium) was done at the Randomization Visit (Visit 4); at Visits 11 (Week 12), 14 (Week 24), 17 (Week 36), 20 (Week 48), and 21 (Week 52) of the 52-week Safety Assessment Period; and at Visit 25 (Week 56) of the Efficacy Assessment Period.

Iron studies including serum iron, ferritin, TSAT, and total iron-binding capacity were done at Screening (Visit 0); at the Randomization Visit (Visit 4); at Visits 7 (Week 4), 9 (Week 8), 11 (Week 12), 12 (Week 16), 13 (Week 20), 14 (Week 24), 15 (Week 28), 16 (Week 32), 17 (Week 36), 18 (Week 40), 19 (Week 44), 20 (Week 48), and 21 (Week 52) of the 52-week Safety Assessment Period; and at Visit 25 (Week 56) of the Efficacy Assessment Period.

Intact parathyroid hormone (iPTH) levels were done at the Randomization Visit (Visit 4); at Visits 11 (Week 12), 17 (Week 36), and 21 (Week 52) during the Safety Assessment Period; and at Visit 25 (Week 56) of the Efficacy Assessment Period.

Serum vitamins (25-dihydroxy-vitamin D3, vitamin A, vitamin B-12, vitamin E, vitamin K, and folic acid) were done at the Randomization Visit (Visit 4,); and at Visits 11 (Week 12), 17 (Week 36), and 21 (Week 52) during the Safety Assessment Period.

A lipid profile (total cholesterol, low-density lipoprotein [LDL], high-density lipoprotein [HDL], and triglycerides) was done at the Randomization Visit (Visit 4); at Visits 11 (Week 12), 17 (Week 36), and 21 (Week 52) during the Safety Assessment Period.

Serum aluminum was done at the Randomization Visit (Visit 4) and at Visit 21 (Week 52).

Serum bicarbonate was performed at a local laboratory and was done at the Randomization Visit (Visit 4); at Visits 11 (Week 12), 14 (Week 24), 17 (Week 36), 20 (Week 48) and 21 (Week 52) during the Safety Assessment Period; and at Visit 25 (Week 56) of the Efficacy Assessment Period.

Except for serum bicarbonate, which was collected and measured locally, all labs were performed by Spectra Clinical Research, Rockleigh, NJ, USA.

### Statistical Considerations: Efficacy

Unless otherwise stated, all hypotheses were tested at a 2-sided significance level of 0.05 and the 95% confidence interval was two-sided. All analyses were performed using SAS Version 9.

Prior to the database lock, a detailed Statistical Analysis Plan (SAP) was completed and placed on file. The Data Analysis Plan contained a more comprehensive explanation than described below of the methodology used in the statistical analyses. The Data Analysis Plan also contained the rules and data handling conventions used to perform the analyses, and the procedure used for accounting for missing data.

Summary tabulations displayed the number of observations, mean, standard deviation, median, minimum, maximum, and appropriate percentiles for continuous variables, and the number and percentage by category for categorical data. Summaries present data by treatment arm and overall, if appropriate. The data listings include all available efficacy and safety data.

The efficacy analyses were based on Full Analysis (FA) population that consisted of all patients who took at least one dose of study medication and provided baseline and at least one post-baseline efficacy assessment. The safety analyses were based on safety population that was consistent of all patients who took at least one dose of study medication.

There were two unique and distinct baseline assessments. The baseline for the Safety Assessment Period was the Randomization Visit (Visit 4) and was defined as "Week-0-baseline." The baseline for the Efficacy Assessment Period was the last visit of the Safety Assessment Period (Visit 21, Week 52) and was defined as "study-baseline."

The primary efficacy outcome of this trial was the effect of ferric citrate vs. placebo on the change in serum phosphorus from study-baseline (Visit 21, Week 52) to end of the Efficacy Assessment Period (Visit 25, Week 56). The primary efficacy variable was analyzed via an ANCOVA model with treatment as the fixed effect and study-baseline as the covariate. Between-treatment differences were estimated and two-sided 95% confidence intervals for the differences were presented.

The secondary endpoints for this trial include the following:

### 1. CHANGE FROM BASELINE IN FERRITIN AT WEEK 52

Change from baseline in ferritin at Week 52 as compared to baseline (Visit 4). This variable will be analyzed using LOCF methodology. ANCOVA will be employed. The model will include treatment (fixed effect), and baseline (covariate). A sensitivity analysis will be performed using MMRM method.

### 2. CHANGE FROM BASELINE IN TSAT AT WEEK 52

Change from baseline in TSAT at Week 52 as compared to baseline (Visit 4). This variable will be analyzed using LOCF methodology. ANCOVA will be employed. The model will include treatment (fixed effect), and baseline (covariate). A sensitivity analysis will be performed using MMRM method.

### 3. CUMULATIVE USE OF IV IRON OVER 52 WEEKS

The cumulative IV iron intake from randomization to Week 52 will be compared between treatment groups. This variable will be similarly analyzed as the primary efficacy variable using ANCOVA method. The two-sided 95% confidence intervals of treatment differences for all above comparisons will be presented.

### 4. CUMULATIVE USE OF EPO (ESA) OVER 52 WEEKS

The cumulative EPO (ESA) administrated from randomization to Week 52 will be compared between treatment groups. This variable will be similarly analyzed as the primary efficacy variable using ANCOVA method. The two-sided 95% confidence intervals of treatment differences for all above comparisons will be presented.

Treatment differences between ferric citrate and all active control binders as well as the differences between ferric citrate and sevelamer carbonate as a single agent at Week 12 (Visit 11) in terms of change from Visit-4 baseline in serum phosphorus, phosphorus times calcium product, and in serum calcium will be analyzed. These variables will be analyzed using LOCF methodology. ANCOVA will be employed. The model will include treatment (fixed effect), and Visit-4 baseline (covariate). An analysis using MMRM method will be conducted as a sensitivity analysis. The least square mean estimates of the treatment effects as well as the 2-sided 95% confidence intervals (CI) of the treatment effects will be derived. Non-inferiority will be claimed if the lower-bound of the two-sided 95% confidence interval of the treatment difference is within 20% of least square mean of the control.

### 5. PERCENTAGE OF PATIENTS ACHIEVING PHOSPHORUS GOAL

1. Percentage of patients achieving phosphorus goal (≤5.5mg/dL) at Weeks 12, 24, 36, 48, 52 and 56 - These variables will be analyzed via chi-square tests. Between-treatment differences in the percentages will be estimated and two-sided 95% confidence intervals for the differences will be calculated using normal approximation without continuity correction.
2. Percentage of patients achieving the phosphorus goal (≤5.5mg/dL) at Week 56 for patients remaining on study medication during the four-week Efficacy Assessment Period - These variables will be analyzed via chi-square tests. Between-treatment differences in the percentages will be estimated and two-sided 95% confidence intervals for the differences will be calculated using normal approximation without continuity correction.
3. Percentage of patients obtaining a serum phosphorus ≥ 9.0mg/dL at any time during the four-week Efficacy Assessment Period - These variables will be analyzed via chi-square tests. Between-treatment differences in the percentages will be estimated and two-sided 95% confidence intervals for the differences will be calculated using normal approximation without continuity correction.

### 6. CHANGE IN SERUM PHOSPHORUS CONCENTRATION

1. Change in serum phosphorus concentration at Weeks 12, 24, 36, 48, and 52 as compared to baseline (Visit 4). This variable will be analyzed using LOCF methodology. ANCOVA will be employed. The model will include treatment (fixed effect), and baseline (covariate).

### 7. CHANGE IN OTHER LABORATORY MEASURES

1. Change in serum calcium concentration at Weeks 12, 24, 36, 48, and 52 as compared to baseline (Visit 4). This variable will be analyzed using LOCF methodology. ANCOVA will be employed. The model will include treatment (fixed effect), and baseline (covariate).
2. Change in ferritin, and TSAT at Weeks 12, 24, 36 and 48 as compared to baseline (Visit 4). This variable will be analyzed using LOCF methodology. ANCOVA will be employed. The model will include treatment (fixed effect), and baseline (covariate).
3. Change in serum iron and TIBC at Weeks 12, 24, 36, 48, and 52 as compared to baseline (Visit 4). This variable will be analyzed using LOCF methodology. ANCOVA will be employed. The model will include treatment (fixed effect), and baseline (covariate).
4. Change in Ca x P product at Weeks 12, 24, 36, 48, and 52 as compared to baseline (Visit 4). This variable will be analyzed using LOCF methodology. ANCOVA will be employed. The model will include treatment (fixed effect), and baseline (covariate).
5. Change in iPTH at Weeks 12, 36, 52, and 56 as compared to baseline (Visit 4). This variable will be analyzed using LOCF methodology. ANCOVA will be employed. The model will include treatment (fixed effect), and baseline (covariate).
6. Change in serum 25-dihydroxy-vitamin D3, vitamin A, vitamin B-12, vitamin E, vitamin K and folic acid at Weeks 12, 36, and 52 as compared to baseline (Visit 4). This variable will be analyzed using LOCF methodology. ANCOVA will be employed. The model will include treatment (fixed effect), and baseline (covariate).
7. Change in serum bicarbonate concentration at Weeks 12, 36, and 52 as compared to baseline (Visit 4). This variable will be analyzed using LOCF methodology. ANCOVA will be employed. The model will include treatment (fixed effect), and baseline (covariate).
8. Change in IV iron intake at Weeks 12, 24, 36, 48, and 52 as compared to baseline (Visit 4). This variable will be analyzed using LOCF methodology. ANCOVA will be employed. The model will include treatment (fixed effect), and baseline (covariate).
9. Change in the use of EPO (ESA) administered at Weeks 12, 24, 36, 48, and 52 as compared to baseline (Visit 4). This variable will be analyzed using LOCF methodology. ANCOVA will be employed. The model will include treatment (fixed effect), and baseline (covariate).
10. Change in the use of Vitamin D supplementation (and its analogs) and Sensipar (cinacalcet) at Weeks 12, 24, 36, 48, and 52 as compared to baseline (Visit 4). This variable will be analyzed using LOCF methodology. ANCOVA will be employed. The model will include treatment (fixed effect), and baseline (covariate).
11. Change in LDL, HDL, and triglycerides at Weeks 12, 36, and 52 as compared to baseline (Visit 4). This variable will be analyzed using LOCF methodology. ANCOVA will be employed. The model will include treatment (fixed effect), and baseline (covariate).

### Statistical Considerations: Safety

Safety was assessed by recording and monitoring adverse events, concomitant medication use, physical examinations, and sequential blood by treatment assignment. Rates of adverse events were summarized overall and by organ system class, preferred term, severity, and suspected relationship to study drug by treatment assignment. AEs were summarized for the Washout Period, Safety Assessment Period, and Efficacy Assessment Period separately by treatment assignment. The changes from baseline in laboratory parameters over time were summarized by treatment assignment.

### Statistical Considerations: Power

Approximately 434 patients were randomized in a 2:1 ratio to either ferric citrate (approximately 288 patients) or active-control (approximately 146 patients), to be treated during the Safety Assessment Period. This sample size provided at least 90% power to detect a treatment difference between ferric citrate and placebo at a 5% significance level, assuming that the treatment difference is 1.2 and the common standard deviation is 2.

### Results

Summary of Treatment Differences in Serum Phosphorus, Phosphorus times Calcium Product and Serum Calcium Change from Study-baseline at Week 12 between Ferric Citrate and Sevelamer Carbonate as a Single Agent (ANCOVA Method), Full Analysis Population - shown in Table 12:

**Table 12:**

| **Statistics** | **KRX-0502 in Safety Assessment Period (N=288)** | **Sevelamer Carbonate in Safety Assessment Period (N=73)** | **Treatment Differences [1]** |
|---|---|---|---|
| **Phosphorus (MG/DL)** | | | |
| | | | |
| Baseline | | | |
| N | 277 | 72 | |
| Mean (SD) | 7.39 (1.557) | 7.51 (1.633) | |
| Median | 7.20 | 7.40 | |
| (Min, Max) | (2.7, 12.3) | (4.3, 12.9) | |
| | | | |

| Week 12 | | | |
|---|---|---|---|
| N | 277 | 72 | |
| Mean (SD) | 5.38 (1.374) | 5.23 (1.713) | |
| Median | 5.10 | 5.00 | |
| (Min, Max) | (2.4, 9.9) | (2.5, 14.1) | |

| **Statistics** | **KRX-0502 in Safety Assessment Period (N=288)** | **Sevelamer Carbonate in Safety Assessment Period (N=73)** | **Treatment Differences[1]** |
|---|---|---|---|
| Week 12 Change from Baseline | | | |
| N | 277 | 72 | |
| Mean (SD) | -2.01 (1.887) | -2.28 (2.169) | |
| Median | -2.00 | -2.45 | |
| (Min, Max) | (-7.6, 4.6) | (-8.9, 6.7) | |
| 95% CI | (5.21, 5.55) | (4.89, 5.55) | (-0.21, 0.54) |
| LS Mean (SE) | 5.38 (0.09) | 5.22 (0.17) | 0.16 (0.19) |
| p-value | | | 0.3900 |
| | | | |

| **Product Of Calcium And Phosphorus** | | | |
|---|---|---|---|
| | | | |
| Baseline | | | |
| N | 277 | 72 | |
| Mean (SD) | 65.4075 (15.47697) | 68.0872 (16.29263) | |
| Median | 62.7000 | 66.2700 | |
| (Min, Max) | (25.920, 123.210) | (36.660, 123.840) | |
| | | | |

| Week 12 | | | |
|---|---|---|---|
| N | 277 | 72 | |
| Mean (SD) | 48.8440 (12.93765) | 48.0251 (14.36518) | |
| Median | 47.5000 | 46.2800 | |
| (Min, Max) | (20.440, 92.650) | (22.500, 109.980) | |
| | | | |

| Week 12 Change from Baseline | | | |
|---|---|---|---|
| N | 277 | 72 | |
| Mean (SD) | -16.5635 | -20.0621 (19.17393) | |
| | (16.97535) | | |
| Median | -16.7400 | -19.8500 | |
| (Min, Max) | (-78.660, 42.700) | (-86.200, 46.340) | |
| 95% CI | (47.47, 50.48) | (44.57, 50.48) | (-1.87, 4.77) |
| LS Mean (SE) | 48.97 (0.77) | 47.52 (1.50) | 1.45 (1.69) |
| p-value | | | 0.3903 |
| | | | |

| **Calcium (MG/DL)** | | | |
|---|---|---|---|
| | | | |
| Baseline | | | |
| N | 278 | 72 | |
| Mean (SD) | 8.843 (0.8048) | 9.056 (0.7291) | |
| Median | 8.900 | 9.150 | |
| (Min, Max) | (6.30, 11.10) | (6.70, 10.30) | |
| | | | |

| Week 12 | | | |
|---|---|---|---|
| N | 278 | 72 | |
| Mean (SD) | 9.089 (0.7568) | 9.231 (0.7210) | |
| Median | 9.100 | 9.400 | |
| (Min, Max) | (6.30, 12.00) | (7.00, 10.60) | |
| | | | |

| Week 12 Change from Baseline | | | |
|---|---|---|---|
| N | 278 | 72 | |
| Mean (SD) | 0.245 (0.7486) | 0.175 (0.7509) | |
| Median | 0.200 | 0.100 | |
| (Min, Max) | (-2.80, 3.00) | (-1.50, 2.30) | |
| 95% CI | (9.04, 9.19) | (9.00, 9.29) | (-0.20, 0.13) |
| LS Mean (SE) | 9.11 (0.04) | 9.15 (0.08) | -0.04 (0.08) |
| p-value | | | 0.6765 |

| | | | |
|---|---|---|---|
| ***Note***: [1]. The LS Mean treatment difference and p-value for the change in Serum Phosphorus, Ca x P and Ca is created via an ANCOVA model with treatment as the fixed effect and Day-0 baseline as the covariate. Between-treatment differences are calculated as the LS Mean (KRX-0502) - LS Mean (Control). Only subjects with both a baseline and post baseline observations for the parameter of interest were included. | | | |

Summary of Mean Serum Phosphorus Values at Weeks 12, 24, 36, 48, and 52 and Change from Study-baseline by Treatment (ANCOVA Method), Full Analysis Population - shown in Table 13:

**Table 13:**

| **statistics** | **XRX-0502 in Safety Assessment Period (N=288)** | **Control in Safety Assessment Period (N=146)** | **Treatment Differences[1]** |
|---|---|---|---|
| Day 0 Baseline | | | |
| N | 277 | 144 | |
| Mean (SD) | 7.39 (1.557) | 7.55 (1.750) | |
| Median | 7.20 | 7.40 | |
| (Min, Max) | (2.7, 12.3) | (4.3, 12.9) | |
| | | | |

| Week 12 | | | |
|---|---|---|---|
| N | 277 | 144 | |
| Mean (SD) | 5.38 (1.374) | 5.34 (1.652) | |
| Median | 5.10 | 5.05 | |
| (Min, Max) | (2.4, 9.9) | (2.5, 14.1) | |
| | | | |

| Week 12 Change from Baseline | | | |
|---|---|---|---|
| N | 277 | 144 | |
| Mean (SD) | -2.01 (1.887) | -2.21 (2.086) | |
| Median | -2.00 | -2.25 | |
| (Min, Max) | (-7.6, 4.6) | (-8.9, 6.7) | |
| 95% CI | (5.22, 5.56) | (5.08, 5.56) | (-0.23, 0.36) |
| LS Mean (SE) | 5.39 (0.09) | 5.32 (0.12) | 0.07 (0.15) |
| p-value | | | 0.6594 |
| | | | |

| Week 24 | | | |
|---|---|---|---|
| N | 277 | 144 | |
| Mean (SD) | 5.24 (1.455) | 5.49 (1.536) | |
| Median | 5.10 | 5.30 | |
| (Min, Max) | (1.3, 10.7) | (2.0, 14.1) | |
| | | | |

| Week 24 Change from Baseline | | | |
|---|---|---|---|
| N | 277 | 144 | |
| Mean (SD) | -2.14 (1.844) | -2.06 (2.125) | |
| Median | -2.10 | -2.00 | |
| (Min, Max) | (-7.5, 3.9) | (-8.4, 6.7) | |
| 95% CI | (5.08, 5.43) | (5.23, 5.71) | (-0.51, 0.08) |
| LS Mean (SE) | 5.26 (0.09) | 5.47 (0.12) | -0.21 (0.15) |
| p-value | | | 0.1510 |
| | | | |

| Week 36 | | | |
|---|---|---|---|
| N | 277 | 144 | |
| Mean (SD) | 5.22 (1.348) | 5.32 (1.557) | |
| Median | 5.10 | 5.10 | |
| (Min, Max) | (1.1, 9.5) | (2.2, 14.1) | |
| | | | |

| Week 36 Change from Baseline | | | |
|---|---|---|---|
| N | 277 | 144 | |
| Mean (SD) | -2.16 (1.748) | -2.24 (2.037) | |
| Median | -2.10 | -2.10 | |
| (Min, Max) | (-7.4, 3.2) | (-8.1, 6.7) | |
| 95% CI | (5.08, 5.40) | (5.07, 5.52) | (-0.33, 0.22) |
| LS Mean (SE) | 5.24 (0.08) | 5.29 (0.11) | -0.05 (0.14) |
| p-value | | | 0.7075 |
| | | | |

| Week 48 | | | |
|---|---|---|---|
| N | 277 | 144 | |
| Mean (SD) | 5.32 (1.468) | 5.48 (1.563) | |
| Median | 5.20 | 5.35 | |
| (Min, Max) | (2.2, 10.8) | (2.2, 14.1) | |
| | | | |

| Week 48 Change from Baseline | | | |
|---|---|---|---|
| N | 277 | 144 | |
| Mean (SD) | -2.07 (1.828) | -2.07 (2.036) | |
| Median | -2.10 | -1.90 | |
| (Min, Max) | (-8.4, 4.6) | (-7.8, 6.7) | |
| 95% CI | (5.16, 5.50) | (5.22, 5.69) | (-0.42, 0.17) |
| LS Mean (SE) | 5.33 (0.09) | 5.46 (0.12) | -0.12 (0.15) |
| p-value | | | 0.4086 |
| | | | |

| Week 52 | | | |
|---|---|---|---|
| N | 277 | 144 | |
| Mean (SD) | 5.32 (1.437) | 5.36 (1.572) | |
| Median | 5.20 | 5.10 | |

| **Statistics** | **KRX-0502 in Safety Assessment Period (N=288)** | **Control in Safety Assessment Period (N=146)** | **Treatment Differences[1]** |
|---|---|---|---|
| (Min, Max) | (1.1, 10.7) | (2.6, 14.1) | |
| | | | |

| Week 52 Change from Baseline | | | |
|---|---|---|---|
| N | 277 | 144 | |
| Mean (SD) | -2.06 (1.834) | -2.19 (2.220) | |
| Median | -2.20 | -2.10 | |
| (Min, Max) | (-7.1, 3.7) | (-9.8, 6.7) | |
| 95% CI | (5.16, 5.51) | (5.10, 5.58) | (-0.30, 0.29) |
| LS Mean (SE) | 5.33 (0.09) | 5.34 (0.12) | -0.01 (0.15) |
| p-value | | | 0.9696 |

| | | | |
|---|---|---|---|
| ***Note*:** [1]. The LS Mean treatment difference and p-value for the change in Ferritin is created via an ANCOVA model with treatment as the fixed effect and Day-0 baseline as the covariate. Between-treatment differences are calculated as the LS Mean (KRX-0502) - LS Mean (control). Only subjects with both a baseline and post baseline observations for the parameter of interest were included. | | | |

Summary of Mean Serum Phosphorus Values and Change from Week-52-baseline by Treatment and Visit during the Efficacy Assessment Period (ANCOVA Method), Full Analysis Population - shown in Table 14:

**Table 14:**

| **Statistics** | **XRX-0502 in Efficacy Assessment Period (N=92)** | **Placebo in Efficacy Assessment Period (N=91)** | **Treatment Differences [1]** |
|---|---|---|---|
| | | | |
| Week 52 Baseline | | | |
| N | 85 | 82 | |
| Mean (SD) | 5.16 (1.259) | 5.25 (1.475) | |
| Median | 5.10 | 5.30 | |
| (Min, Max) | (2.2, 8.7) | (1.1, 8.8) | |
| | | | |

| Week 53 | | | |
|---|---|---|---|
| N | 76 | 79 | |
| Mean (SD) | 4.90 (1.152) | 6.66 (1.611) | |
| Median | 4.95 | 6.50 | |
| (Min, Max) | (2.0, 7.7) | (2.4, 10.6) | |
| | | | |

| Week 53 Change from Baseline | | | |
|---|---|---|---|
| N | 76 | 79 | |
| Mean (SD) | -0.31 (1.432) | 1.39 (1.626) | |
| Median | -0.30 | 1.30 | |
| (Min, Max) | (-4.6, 2.9) | (-2.1, 5.5) | |
| 95% CI | (4.62, 5.21) | (6.36, 6.94) | (-2.15, -1.32) |
| LS Mean (SE) | 4.92 (0.15) | 6.65 (0.15) | -1.73 (0.21) |
| p-value | | | <0.0001 |
| | | | |

| Week 54 | | | |
|---|---|---|---|
| N | 84 | 81 | |
| Mean (SD) | 4.78 (1.309) | 6.91 (1.724) | |
| Median | 4.70 | 6.80 | |
| (Min, Max) | (2.1, 8.9) | (3.4, 10.6) | |
| | | | |

| Week 54 Change from Baseline | | | |
|---|---|---|---|
| N | 84 | 81 | |
| Mean (SD) | -0.36 (1.404) | 1.65 (1.847) | |
| Median | -0.40 | 1.60 | |
| (Min, Max) | (-3.9, 3.8) | (-2.3, 6.5) | |
| 95% CI | (4.50, 5.11) | (6.57, 7.20) | (-2.52, -1.64) |
| LS Mean (SE) | 4.80 (0.16) | 6.88 (0.16) | -2.08 (0.22) |
| p-value | | | <0.0001 |
| | | | |

| Week 55 | | | |
|---|---|---|---|
| N | 85 | 82 | |
| Mean (SD) | 4.75 (1.237) | 6.96 (1.808) | |
| Median | 4.60 | 7.00 | |
| (Min, Max) | (2.8, 9.5) | (2.7, 10.6) | |
| | | | |

| Week 55 Change from Baseline | | | |
|---|---|---|---|
| N | 85 | 82 | |
| Mean (SD) | -0.41 (1.444) | 1.71 (1.967) | |
| Median | -0.50 | 1.85 | |
| (Min, Max) | (-3.2, 4.6) | (-2.6, 6.5) | |
| 95% CI | (4.45, 5.08) | (6.62, 7.26) | (-2.63, -1.73) |
| LS Mean (SE) | 4.76 (0.16) | 6.94 (0.16) | -2.18 (0.23) |
| p-value | | | <0.0001 |
| | | | |

| Week 56 | | | |
|---|---|---|---|
| N | 85 | 82 | |
| Mean (SD) | 4.92 (1.323) | 7.24 (1.812) | |
| Median | 4.60 | 7.25 | |
| (Min, Max) | (2.3, 9.5) | (3.0, 10.6) | |
| | | | |

| Week 56 Change from Baseline | | | |
|---|---|---|---|
| N | 85 | 82 | |
| Mean (SD) | -0.23 (1.484) | 1.99 (1.979) | |
| Median | -0.50 | 2.20 | |
| (Min, Max) | (-2.9, 4.6) | (-2.7, 6.5) | |
| 95% CI | (4.62, 5.26) | (6.89, 7.55) | (-2.74, -1.82) |
| LS Mean (SE) | 4.94 (0.16) | 7.22 (0.17) | -2.28 (0.23) |
| p-value | | | <0.0001 |

| | | | |
|---|---|---|---|
| ***Note*:** [1]. The LS Mean treatment difference and p-value for the change in Serum Phosphorus is created via an ANCOVA model with treatment as the fixed effect and Week-52 baseline as the covariate. Between-treatment differences are calculated as the LS Mean (KRX-0502) - LS Mean (Placebo). Only subjects with both a baseline and post baseline observations for the parameter of interest were included. | | | |

Summary of Mean Ferritin at Weeks 12, 24, 36, 48, and 52 and Change from Study-baseline by Treatment (ANCOVA Method), Full Analysis Population - shown in Table 15:

**Table 15:**

| **Statistics** | **XRX-0502 in Safety Assessment Period (N=288)** | **Control in Safety Assessment Period (N=146)** | **Treatment Differences[1]** |
|---|---|---|---|
| Day 0 Baseline | | | |
| N | 249 | 134 | |
| Mean (SD) | 595.00 (293.896) | 615.76 (307.842) | |
| Median | 587.00 | 574.00 | |
| (Min, Max) | (22.0, 1612.0) | (11.0, 1548.0) | |
| | | | |

| Week 12 | | | |
|---|---|---|---|
| N | 243 | 134 | |
| Mean (SD) | 751.19 (379.766) | 656.68 (321.518) | |
| Median | 718.00 | 646.50 | |
| (Min, Max) | (25.0, 2691.0) | (13.0, 1664.0) | |
| | | | |

| Week 12 Change from Baseline | | | |
|---|---|---|---|
| N | 243 | 134 | |
| Mean (SD) | 158.88 (283.314) | 40.92 (273.201) | |
| Median | 123.00 | 26.50 | |
| (Min, Max) | (-882.0, 1660.0) | (-794.0, 920.0) | |
| 95% CI | (723.34, 792.15) | (598.46, 691.14) | (55.22, 170.68) |
| LS Mean (SE) | 757.75 (17.50) | 644.80 (23.57) | 112.95 (29.36) |
| p-value | | | 0.0001 |
| | | | |

| Week 24 | | | |
|---|---|---|---|
| N | 247 | 134 | |

| **Statistics** | **XRX-0502 in Safety Assessment Period (N=288)** | **Control in Safety Assessment Period (N=146)** | **Treatment Differences[1]** |
|---|---|---|---|
| Mean (SD) | 846.90 (414.672) | 658.44 (301.698) | |
| Median | 830.00 | 675.00 | |
| (Min, Max) | (91.0, 2413.0) | (11.0, 1525.0) | |
| | | | |

| Week 24 Change from Baseline | | | |
|---|---|---|---|
| N | 247 | 134 | |
| Mean (SD) | 252.49 (326.299) | 42.68 (291.868) | |
| Median | 220.00 | 35.50 | |
| (Min, Max) | (-628.0, 1594.0) | (-997.0, 757.0) | |
| 95% CI | (814.24, 890.79) | (596.11, 700.06) | (139.87, 269.00) |
| LS Mean (SE) | 852.52 (19.47) | 648.08 (26.43) | 204.43 (32.84) |
| p-value | | | <0.0001 |
| | | | |

| Week 36 | | | |
|---|---|---|---|
| N | 247 | 134 | |
| Mean (SD) | 863.18 (444.094) | 635.96 (326.652) | |
| Median | 818.00 | 612.00 | |
| (Min, Max) | (51.0, 3181.0) | (13.0, 2080.0) | |
| | | | |

| Week 36 Change from Baseline | | | |
|---|---|---|---|
| N | 247 | 134 | |
| Mean (SD) | 268.77 (391.292) | 20.20 (328.820) | |
| Median | 223.00 | 11.00 | |
| (Min, Max) | (-754.0, 2193.0) | (-958.0, 1589.0) | |
| 95% CI | (823.50, 912.72) | (566.30, 687.45) | (165.99, 316.49) |
| LS Mean (SE) | 868.11 (22.69) | 626.87 (30.81) | 241.24 (38.27) |
| p-value | | | <0.0001 |
| | | | |

| Week 48 | | | |
|---|---|---|---|
| N | 247 | 134 | |
| Mean (SD) | 882.10 (461.772) | 626.63 (353.836) | |
| Median | 850.00 | 597.00 | |
| (Min, Max) | (44.0, 3188.0) | (84.0, 1784.0) | |
| | | | |

| Week 48 Change from Baseline | | | |
|---|---|---|---|
| N | 247 | 134 | |
| Mean (SD) | 287.69 (395.752) | 10.87 (352.066) | |
| Median | 233.00 | 13.50 | |
| (Min, Max) | (-667.0, 2032.0) | (-1184.0, 1409.0) | |
| 95% CI | (840.95, 933.86) | (553.76, 679.93) | (192.20, 348.93) |
| LS Mean (SE) | 887.41 (23.63) | 616.85 (32.08) | 270.56 (39.85) |
| p-value | | | <0.0001 |
| | | | |

| Week 52 | | | |
|---|---|---|---|
| N | 249 | 134 | |
| Mean (SD) | 897.12 (485.296) | 625.30 (359.018) | |
| Median | 858.00 | 576.00 | |
| (Min, Max) | (44.0, 3144.0) | (33.0, 1789.0) | |
| | | | |

| Week 52 Change from Baseline | | | |
|---|---|---|---|
| N | 249 | 134 | |
| Mean (SD) | 302.11 (435.183) | 9.54 (360.411) | |
| Median | 224.00 | 21.50 | |
| (Min, Max) | (-785.0, 2032.0) | (-1165.0, 1409.0) | |
| 95% CI | (852.25, 951.66) | (548.54, 684.08) | (201.58, 369.71) |
| LS Mean (SE) | 901.95 (25.28) | 616.31 (34.47) | 285.65 (42.76) |
| p-value | | | <0.0001 |

| | | | |
|---|---|---|---|
| ***Note*:** [1]. The LS Mean treatment difference and p-value for the change in Ferritin is created via an ANCOVA model with treatment as the fixed effect and Day-0 baseline as the covariate. Between-treatment differences are calculated as the LS Mean (KRX-0502) - LS Mean (control). Only subjects with both a baseline and post baseline observations for the parameter of interest were included. | | | |

Summary of Mean TSAT at Weeks 12, 24, 36, 48, and 52 and Change from Study-baseline by Treatment (ANCOVA Method), Full Analysis Population - shown in Table 16:

**Table 16:**

| **Statistics** | **XRX-0502 in Safety Assessment Period (N=288)** | **Control in Safety Assessment Period (N=146)** | **Treatment Differences[1]** |
|---|---|---|---|
| | | | |
| Day 0 Baseline | | | |
| N | 244 | 131 | |
| Mean (SD) | 31.0 (10.99) | 31.0 (11.75) | |
| Median | 29.5 | 29.0 | |
| (Min, Max) | (10, 83) | (10, 73) | |

| Week 12 | | | |
|---|---|---|---|
| N | 238 | 131 | |
| Mean (SD) | 40.2 (16.00) | 31.4 (12.13) | |
| Median | 37.0 | 29.0 | |
| (Min, Max) | (12, 85) | (10, 79) | |

| Week 12 Change from Baseline | | | |
|---|---|---|---|
| N | 238 | 131 | |
| Mean (SD) | 9.2 (17.95) | 0.5 (15.91) | |
| Median | 7.0 | 1.0 | |
| (Min, Max) | (-61, 62) | (-54, 51) | |
| 95% CI | (38.31, 42.03) | (28.92, 33.94) | (5.61, 11.87) |
| LS Mean (SE) | 40.17 (0.95) | 31.43 (1.28) | 8.74 (1.59) |
| p-value | | | <0.0001 |

| Week 24 | | | |
|---|---|---|---|
| N | 242 | 131 | |
| Mean (SD) | 39.9 (15.52) | 31.6 (11.96) | |
| Median | 38.0 | 29.0 | |
| (Min, Max) | (13, 92) | (11, 79) | |

| Week 24 Change from Baseline | | | |
|---|---|---|---|
| N | 242 | 131 | |
| Mean (SD) | 8.9 (17.49) | 0.6 (15.40) | |
| Median | 7.0 | 0.0 | |
| (Min, Max) | (-43, 63) | (-52, 49) | |
| 95% CI | (38.11, 41.70) | (29.18, 34.06) | (5.25, 11.31) |
| LS Mean (SE) | 39.90 (0.91) | 31.62 (1.24) | 8.28 (1.54) |
| p-value | | | <0.0001 |

| Week 36 | | | |
|---|---|---|---|
| N | 242 | 131 | |
| Mean (SD) | 39.8 (15.66) | 30.4 (10.88) | |
| Median | 37.0 | 28.0 | |
| (Min, Max) | (14, 86) | (13, 67) | |

| Week 36 Change from Baseline | | | |
|---|---|---|---|
| N | 242 | 131 | |
| Mean (SD) | 8.8 (17.47) | -0.6 (14.99) | |
| Median | 7.0 | -1.0 | |
| (Min, Max) | (-57, 63) | (-45, 49) | |
| 95% CI | (38.03, 41.57) | (27.95, 32.76) | (6.45, 12.43) |
| LS Mean (SE) | 39.80 (0.90) | 30.36 (1.22) | 9.44 (1.52) |
| p-value | | | <0.0001 |

| Week 48 | | | |
|---|---|---|---|
| N | 242 | 131 | |
| Mean (SD) | 40.6 (16.94) | 29.4 (10.71) | |
| Median | 38.0 | 28.0 | |
| (Min, Max) | (13, 86) | (10, 74) | |

| Week 48 Change from Baseline | | | |
|---|---|---|---|
| N | 242 | 131 | |
| Mean (SD) | 9.6 (19.25) | -1.5 (14.48) | |
| Median | 7.0 | -2.0 | |
| (Min, Max) | (-45, 67) | (-48, 42) | |
| 95% CI | (38.71, 42.49) | (26.85, 32.00) | (7.98, 14.37) |
| LS Mean (SE) | 40.60 (0.96) | 29.43 (1.31) | 11.17 (1.62) |
| p-value | | | <0.0001 |

| Week 52 | | | |
|---|---|---|---|
| N | 244 | 131 | |
| Mean (SD) | 39.4 (16.81) | 29.7 (11.49) | |
| Median | 35.0 | 28.0 | |

| **Statistics** | **KRX-0502 in Safety Assessment Period (N=288)** | **Control in Safety Assessment Period (N=146)** | **Treatment Differences[1]** |
|---|---|---|---|
| (Min, Max) | (7, 88) | (10, 72) | |
| | | | |

| Week 52 Change from Baseline | | | |
|---|---|---|---|
| N | 244 | 131 | |
| Mean (SD) | 8.3 (17.97) | -1.3 (14.94) | |
| Median | 6.0 | 0.0 | |
| (Min, Max) | (-60, 62) | (-53, 43) | |
| 95% CI | (37.48, 41.23) | (27.14, 32.25) | (6.49, 12.83) |
| LS Mean (SE) | 39.35 (0.95) | 29.69 (1.30) | 9.66 (1.61) |
| p-value | | | <0.0001 |

| | | | |
|---|---|---|---|
| ***Note*:** [1]. The LS Mean treatment difference and p-value for the change in Ferritin is created via an ANCOVA model with treatment as the fixed effect and Day-0 baseline as the covariate. Between-treatment differences are calculated as the LS Mean (KRX-0502) - LS Mean (control). Only subjects with both a baseline and post baseline observations for the parameter of interest were included. | | | |

Summary of Mean Hemoglobin at Weeks 12, 24, 36, 48, and 52 and Change from Study-baseline by Treatment (ANCOVA method), Full Analysis Population - shown in Table 17:

**Table 17:**

| **Statistics** | **KRX-0502 in Safety Assessment Period (N=288)** | **Control in Safety Assessment Period (N=146)** | **Treatment Differences[1]** |
|---|---|---|---|
| Day 0 Baseline | | | |
| N | 244 | 130 | |
| Mean (SD) | 11.61 (1.213) | 11.72 (1.265) | |
| Median | 11.45 | 11.70 | |
| (Min, Max) | (8.7, 15.8) | (8.7, 15.7) | |

| Week 12 | | | |
|---|---|---|---|
| N | 231 | 128 | |
| Mean (SD) | 11.82 (1.375) | 11.55 (1.268) | |
| Median | 11.70 | 11.60 | |
| (Min, Max) | (7.5, 17.4) | (6.7, 14.5) | |

| Week 12 Change from Baseline | | | |
|---|---|---|---|
| N | 231 | 128 | |
| Mean (SD) | 0.19 (1.397) | -0.16 (1.522) | |
| Median | 0.10 | -0.05 | |
| (Min, Max) | (-4.6, 4.0) | (-4.3, 3.5) | |
| 95% CI | (11.67, 11.99) | (11.31, 11.75) | (0.03, 0.57) |
| LS Mean (SE) | 11.83 (0.08) | 11.53 (0.11) | 0.30 (0.14) |
| p-value | | | 0.0291 |

| Week 24 | | | |
|---|---|---|---|
| N | 241 | 130 | |
| Mean (SD) | 11.55 (1.401) | 11.47 (1.165) | |
| Median | 11.30 | 11.40 | |
| (Min, Max) | (6.6, 17.3) | (9.2, 15.4) | |

| Week 24 Change from Baseline | | | |
|---|---|---|---|
| N | 241 | 130 | |
| Mean (SD) | -0.08 (1.405) | -0.25 (1.394) | |
| Median | -0.10 | -0.30 | |
| (Min, Max) | (-6.3, 3.8) | (-2.9, 3.5) | |
| 95% CI | (11.41, 11.72) | (11.23, 11.65) | (-0.14, 0.38) |
| LS Mean (SE) | 11.56 (0.08) | 11.44 (0.11) | 0.12 (0.13) |
| p-value | | | 0.3756 |

| Week 36 | | | |
|---|---|---|---|
| N | 241 | 130 | |
| Mean (SD) | 11.54 (1.432) | 11.31 (1.205) | |
| Median | 11.20 | 11.20 | |
| (Min, Max) | (8.6, 17.4) | (8.9, 14.9) | |
| | | | |

| Week 36 Change from Baseline | | | |
|---|---|---|---|
| N | 241 | 130 | |
| Mean (SD) | -0.08 (1.359) | -0.41 (1.577) | |
| Median | -0.10 | -0.50 | |
| (Min, Max) | (-5.1, 3.9) | (-3.8, 4.6) | |
| 95% CI | (11.39, 11.71) | (11.06, 11.50) | (0.00, 0.54) |
| LS Mean (SE) | 11.55 (0.08) | 11.28 (0.11) | 0.27 (0.14) |
| p-value | | | 0.0482 |

| Week 48 | | | |
|---|---|---|---|
| N | 241 | 130 | |
| Mean (SD) | 11.50 (1.502) | 11.25 (1.296) | |
| Median | 11.20 | 11.10 | |
| (Min, Max) | (6.7, 18.2) | (7.9, 16.1) | |

| Week 48 Change from Baseline | | | |
|---|---|---|---|
| N | 241 | 130 | |
| Mean (SD) | -0.12 (1.395) | -0.47 (1.498) | |
| Median | -0.20 | -0.30 | |
| (Min, Max) | (-4.8, 4.9) | (-4.2, 3.5) | |
| 95% CI | (11.35, 11.68) | (10.99, 11.44) | (0.03, 0.58) |
| LS Mean (SE) | 11.52 (0.08) | 11.21 (0.11) | 0.30 (0.14) |
| p-value | | | 0.0322 |

| Week 52 | | | |
|---|---|---|---|
| N | 244 | 130 | |
| Mean (SD) | 11.42 (1.474) | 11.11 (1.403) | |
| Median | 11.20 | 11.00 | |
| (Min, Max) | (8.3, 16.6) | (7.1, 15.3) | |

| Week 52 Change from Baseline | | | |
|---|---|---|---|
| N | 244 | 130 | |
| Mean (SD) | -0.20 (1.326) | -0.61 (1.581) | |
| Median | -0.20 | -0.60 | |
| (Min, Max) | (-3.9, 3.7) | (-4.9, 4.6) | |
| 95% CI | (11.27, 11.60) | (10.85, 11.30) | (0.09, 0.64) |
| LS Mean (SE) | 11.44 (0.08) | 11.07 (0.11) | 0.36 (0.14) |
| p-value | | | 0.0105 |

| | | | |
|---|---|---|---|
| ***Note*:** [1]. The LS Mean treatment difference and p-value for the change in Ferritin is created via an ANCOVA model with treatment as the fixed effect and Day-0 baseline as the covariate. Between-treatment differences are calculated as the LS Mean (KRX-0502) - LS Mean (control). Only subjects with both a baseline and post baseline observations for the parameter of interest were included. | | | |

Summary of Mean Serum Bicarbonate Concentration at Weeks 12, 24, 36, 48 and 52 and Change from Study-baseline by Treatment (ANCOVA Method), Full Analysis Population - shown in Table 18:

**Table 18:**

| **Statistics** | **XRX-0502 in Safety Assessment Period (N=288)** | **Control in Safety Assessment Period (N=146)** | **Treatment Differences[1]** |
|---|---|---|---|
| | | | |
| Day 0 Baseline | | | |
| N | 214 | 117 | |
| Mean (SD) | 23.92 (3.408) | 23.65 (3.393) | |
| Median | 24.00 | 23.00 | |
| (Min, Max) | (13.0, 34.0) | (11.0, 32.0) | |

| Week 12 | | | |
|---|---|---|---|
| N | 190 | 101 | |
| Mean (SD) | 25.63 (3.358) | 26.25 (3.481) | |
| Median | 25.00 | 26.00 | |
| (Min, Max) | (15.0, 36.0) | (16.0, 34.0) | |

| Week 12 Change from Baseline | | | |
|---|---|---|---|
| N | 190 | 101 | |
| Mean (SD) | 1.57 (3.364) | 2.41 (3.813) | |
| Median | 1.05 | 2.00 | |
| (Min, Max) | (-7.0, 13.0) | (-10.0, 14.0) | |
| 95% CI | (25.17, 26.03) | (25.73, 26.91) | (-1.45, 0.01) |
| LS Mean (SE) | 25.60 (0.22) | 26.32 (0.30) | -0.72 (0.37) |
| p-value | | | 0.0522 |

| Week 24 | | | |
|---|---|---|---|
| N | 200 | 113 | |
| Mean (SD) | 25.39 (3.424) | 25.66 (3.953) | |
| Median | 25.45 | 26.00 | |
| (Min, Max) | (16.0, 36.0) | (16.0, 34.0) | |

| Week 24 Change from Baseline | | | |
|---|---|---|---|
| N | 200 | 113 | |
| Mean (SD) | 1.48 (3.499) | 1.99 (3.854) | |
| Median | 1.00 | 2.00 | |
| (Min, Max) | (-13.0, 13.0) | (-6.0, 14.0) | |
| 95% CI | (24.90, 25.79) | (25.15, 26.33) | (-1.13, 0.35) |
| LS Mean (SE) | 25.35 (0.23) | 25.74 (0.30) | -0.39 (0.38) |
| p-value | | | 0.2974 |

| Week 36 | | | |
|---|---|---|---|
| N | 212 | 117 | |
| Mean (SD) | 25.27 (3.152) | 25.29 (3.700) | |
| Median | 25.00 | 25.00 | |
| (Min, Max) | (17.0, 33.0) | (17.0, 36.0) | |

| Week 36 Change from Baseline | | | |
|---|---|---|---|
| N | 212 | 117 | |
| Mean (SD) | 1.36 (3.441) | 1.64 (3.555) | |
| Median | 1.00 | 1.00 | |
| (Min, Max) | (-10.0, 16.0) | (-7.0, 14.0) | |
| 95% CI | (24.82, 25.62) | (24.83, 25.91) | (-0.82, 0.53) |
| LS Mean (SE) | 25.22 (0.20) | 25.37 (0.27) | -0.15 (0.34) |
| p-value | | | 0.6706 |

| Week 48 | | | |
|---|---|---|---|
| N | 212 | 117 | |
| Mean (SD) | 24.81 (3.177) | 25.24 (3.634) | |
| Median | 25.00 | 25.20 | |
| (Min, Max) | (15.0, 33.0) | (15.0, 34.0) | |

| Week 48 Change from Baseline | | | |
|---|---|---|---|
| N | 212 | 117 | |
| Mean (SD) | 0.91 (3.614) | 1.59 (4.081) | |
| Median | 1.00 | 1.00 | |
| (Min, Max) | (-12.0, 14.0) | (-9.0, 14.0) | |
| 95% CI | (24.36, 25.20) | (24.74, 25.87) | (-1.23, 0.18) |
| LS Mean (SE) | 24.78 (0.21) | 25.30 (0.29) | -0.52 (0.36) |
| p-value | | | 0.1458 |

| Week 52 | | | |
|---|---|---|---|
| N | 214 | 117 | |
| Mean (SD) | 24.63 (4.049) | 25.25 (3.871) | |
| Median | 25.00 | 25.00 | |
| (Min, Max) | (-9.0, 33.0) | (15.0, 35.0) | |

| Week 52 Change from Baseline | | | |
|---|---|---|---|
| N | 214 | 117 | |
| Mean (SD) | 0.71 (4.369) | 1.59 (4.668) | |
| Median | 1.00 | 1.00 | |
| (Min, Max) | (-37.0, 15.0) | (-9.0, 14.0) | |
| 95% CI | (24.08, 25.11) | (24.60, 26.00) | (-1.57, 0.16) |
| LS Mean (SE) | 24.60 (0.26) | 25.30 (0.36) | -0.70 (0.44) |
| p-value | | | 0.1117 |

| | | | |
|---|---|---|---|
| ***Note***: [1]. The LS Mean treatment difference and p-value for the change in Ferritin is created via an ANCOVA model with treatment as the fixed effect and Day-0 baseline as the covariate. Between-treatment differences are calculated as the LS Mean (KRX-0502) - LS Mean (control). Only subjects with both a baseline and post baseline observations for the parameter of interest were included. | | | |

Summary of Cumulative IV iron intake to Week 52 by Treatment, Full Analysis Population, Method 1 to Handle Overlapping Doses - shown in Table 19:

**Table 19:**

| **Statistics** | **KRX-0502 in Safety Assessment Period (N=288)** | **Control in Safety Assessment Period (N=146)** | **Treatment Differences[1]** |
|---|---|---|---|
| Average Daily IV iron intake based on the Cumulative IV iron intake to week 52 (Visit 4 - 21) [2,3] | | | |
| N | 278 | 138 | |
| Mean (SD) | 2.96 (4.260) | 4.86 (4.374) | |
| Median | 1.86 | 3.84 | |
| (Min, Max) | (0.0, 44.3) | (0.0, 24.2) | |
| p-value[4] | | | <0.0001 |

| | | | |
|---|---|---|---|
| ***Note***: [1]. The LS Mean treatment difference and p-value for cumulative IV iron intake is created via an ANCOVA model with treatment as the fixed effect. Between-treatment differences are calculated as the LS Mean (KRX-0502) - LS Mean (control). ***Note***: [2]. Average Daily IV iron intake based on the Cumulative IV iron intake to week 52 is calculated as the total Cumulative IV iron intake divided be the total number of days on study drug. ***Note***: [3]. The Method 1 to Handle Overlapping Doses is the following: For the overlapping doses will be prorated based on days to only include a dose for the period of time on study drug during the Safety Assessment Period. ***Note***: [4]. In the case where basic assumptions are not met for ANCOVA, the Wilcoxon Rank Sum Test is used to calculate the p-value, and the CI and LS Mean removed. | | | |

Summary of Cumulative EPO (ESA) Administered to Week 52 by Treatment, Full Analysis Population, Method 1 to Handle Overlapping Doses - shown in Table 20:

**Table 20:**

| **Statistics** | **XRX-0502 in Safety Assessment Period (N=288)** | **Control in Safety Assessment Period (N=146)** | **Treatment Differences[1]** |
|---|---|---|---|
| Average Daily EPO (ESA) intake based on the Cumulative EPO (ESA) intake to week 52 (Visit 4 - 21) [2,3] | | | |
| n | 280 | 141 | |
| Mean (SD) | 1077.67 (1291.384) | 1309.85 (1342.258) | |
| Median | 724.24 | 993.46 | |
| (Min, Max) | (0.0, 11015.0) | (0.0, 8171.9) | |
| p-value[4] | | | 0.0322 |

| | | | |
|---|---|---|---|
| ***Note***: [1]. The LS Mean treatment difference and p-value for cumulative EPO (ESA) intake is created via an ANCOVA model with treatment as the fixed effect. Between-treatment differences are calculated as the LS Mean (KRX-0502) - LS Mean (control). ***Note***: [2]. Average Daily IV iron intake based on the Cumulative EPO (ESA) intake to week 52 is calculated as the total Cumulative EPO (ESA) intake divided be the total number of days on study drug. ***Note***: [3]. The Method 1 to Handle Overlapping Doses is the following: For the overlapping doses will be prorated based on days to only include a dose for the period of time on study drug during the Safety Assessment Period. ***Note***: [4]. In the case where basic assumptions are not met for ANCOVA, the Wilcoxon Rank Sum Test is used to calculate the p-value, and the CI and LS Mean removed. | | | |

### Example 2

### A Study of KRX-0502 (Ferric Citrate) in Managing Serum Phosphorus and Iron Deficiency in Anemic Subjects with Stage III to V Chronic Kidney Disease Not on Dialysis

A phase 2, proof of concept, multicenter, randomized, placebo-controlled, open-label clinical trial is performed.

The study lasts approximately five to seven months, with approximately eight to 12 weeks being allocated for subject screening, two weeks for washing subjects out of their current phosphate binders (if taking them), and 12 weeks allocated for treatment with study drug, which is either the ferric citrate disclosed herein, or placebo. For purposes of this Example, the ferric citrate disclosed herein is referred to as KRX-0502 (ferric citrate).

The objectives of the study are to determine the efficacy and safety of KRX-0502 (ferric citrate) in managing serum phosphorus and iron deficiency in anemic subjects with non-dialysis dependent Stage III to V chronic kidney disease (CKD).

Up to approximately 200 subjects are screened to randomize approximately 140 subjects. Eligible subjects are randomized in a 1:1 ratio to either KRX-0502 (ferric citrate) or placebo. There are approximately 70 subjects randomized per treatment arm. The dropout rate during the two-week washout and 12-week treatment periods is approximately 20% and therefore approximately 110 subjects complete 12 weeks of treatment with study drug (KRX-0502 (ferric citrate) or placebo). There are approximately 55 subjects completing 12 weeks of treatment with study drug (KRX-0502 (ferric citrate) or placebo).

The trial consists of three periods: screening, two-week washout, and 12-week treatment periods. It takes approximately eight to 12 weeks to screen approximately 200 subjects at approximately 10 to 15 sites. The two-week washout period is only for subjects currently taking a phosphate binder.

The trial enrolls two different types of anemic Stage III to V CKD subjects. They are as follows: 1) Subjects with a serum phosphorus ≥ 4.5 mg/dL and < 6.0 mg/dL who have failed a low phosphate diet and have not been initiated on any phosphate binder (de novo subjects) and have a documented history of anemia; or 2) Subjects who are currently taking phosphate binders to manage their serum phosphorus and have a documented history of anemia. De novo subjects do not enter a washout period and subjects currently taking phosphate binders enter a two-week washout period. Following two weeks of washout, these subjects have a serum phosphorus ≥ 4.5 mg/dL and < 6.0 mg/dL in order to enter the 12-week treatment period.

Enrollment is not stratified for de novo subjects vs. subjects currently taking phosphate binders.

### Study Design/Methodology

This trial is a three-period clinical trial consisting of a screening period, a two-week washout period, and a 12-week treatment period. After a subject is determined to be eligible for enrollment, the subject is randomized to either KRX-0502 (ferric citrate) or placebo. Subjects are randomized in a 1:1 ratio to either KRX-0502 (ferric citrate) or placebo.

Subjects currently taking a phosphate binder are entered into a two-week washout period and, following the completion of the two-week washout period, are randomized to either KRX-0502 (ferric citrate) or placebo. Eligible subjects not taking a phosphate binder immediately start on study drug (KRX-0502 (ferric citrate) or placebo). There is no washout period in this subject population. All subjects have a serum phosphorus ≥ 4.5 mg/dL in order to enter the 12-week treatment period.

After starting treatment with study drug (KRX-0502 (ferric citrate) or placebo), subjects are titrated to therapeutic goal (serum phosphorus between 3.0 to 4.0 mg/dL). If a subject has a serum phosphorus ≥ 6.0 mg/dL for at least two visits in a row during the 12-week treatment period, the subject is considered a treatment failure, stops study drug and exits the study.

The use of IV iron and erythropoietin stimulating agents (ESAs) is not permitted during the two-week washout and 12-week treatment periods. If a subject's hemoglobin level (Hgb) is < 9.0 g/dL during the two-week washout, the subject is a screen failure. If a subject's Hgb is < 9.0 g/dL for at least two visits in a row during the 12-week treatment period, the subject is considered a treatment failure, stops study drug and exits the study.

Serum phosphorus, serum calcium, serum creatinine (used to estimate glomerular filtration rate), intact fibroblast growth factor 23 (FGF23), intact parathyroid hormone (iPTH) and several hematological parameters (ferritin, TSAT, unsaturated iron binding capacity (UIBC), TIBC, serum iron, hematocrit (HCT) and Hgb) are determined at screening, during the washout period, prior to the administration of study drug (KRX-0502 (ferric citrate) or placebo) at Visit 4 (Week 0), and weekly during the 12-week treatment period.

Urinary phosphorus is determined prior to the administration of study drug (KRX-0502 (ferric citrate) or placebo) at Visit 4 (Week 0), at Visit 7 (Week 4) and Visit 9 (Week 8) during the 12-week treatment period and at the end of the 12-week treatment period (Visit 11, Week 12).

The inclusion criteria for this trial are as follows:
1. Males and non-pregnant, non-lactating females;
2. Age > 18 years;
3. Stage III to V CKD subjects not on dialysis who have failed a low phosphate diet to control serum phosphorus and: (i) are currently taking a phosphate binder to manage their serum phosphorus and have a serum phosphorus at screening > 2.5 mg/dL and < 6.0 mg/dL, or (ii) are not taking a phosphate binder and have a serum phosphorus level at screening ≥ 4.5 mg/dL and < 6.0 mg/dL;
4. Documented history of anemia;
5. Serum ferritin < 200 ng/mL and TSAT 20%;
6. Hemoglobin > 9.5 g/dL and < 11.5 g/dL;
7. Glomerular filtration rate (GFR) < 60 mL/min;
8. If currently on a phosphate binder, willing to be discontinued from current phosphate binder(s), enter a washout period and be randomized to either KRX-0502 (ferric citrate) or placebo; and
9. Willing and able to give informed consent.
The exclusion criteria for this trial are as follows:
1. Parathyroidectomy within six months prior to Screening Visit (Visit 0);
2. Symptomatic gastrointestinal bleeding within three months prior to Screening Visit (Visit 0) and inflammatory bowel disease;
3. On dialysis;
4. IV iron administered within 60 days prior to randomization (Visit 4, Week 0);
5. Blood transfusion within 60 days prior to randomization (Visit 4, Week 0);
6. Kidney transplant or start of dialysis expected within three (3) months of randomization (Visit 4, Week 0);
7. Causes of anemia other than iron deficiency;
8. Serum parathyroid hormone >1000 pg/ml;
9. History of multiple drug allergies;
10. History of malignancy in the last five years (treated cervical or skin cancer may be permitted, upon approval);
11. Previous intolerance to oral ferric citrate;
12. Absolute requirement for oral iron therapy;
13. Absolute requirement for Vitamin C; however, multivitamins (i.e., Centrum, Nephrocaps, Renaphro, etc.) are allowed;
14. Absolute requirement for calcium-, magnesium-, or aluminum-containing drugs with meals;
15. Psychiatric disorder that interferes with the subject's ability to comply with the study protocol;
16. Planned surgery or hospitalization during the study (scheduled outpatient access surgery allowed);
17. Any other medical condition that renders the subject unable to or unlikely to complete the study or that would interfere with optimal participation in the study or produce significant risk to the subject;
18. Receipt of any investigational drug within 30 days of randomization (Visit 4, Week 0); and
19. Inability to cooperate with study personnel or history of noncompliance.

### Study Drug Administration

KRX-0502 (ferric citrate) is supplied as 1-gram caplets of ferric citrate containing approximately 210 mg of ferric iron to those subjects randomized to ferric citrate.

Matching placebo is supplied to those subjects randomized to placebo.

All subjects are initiated on study drug with a fixed dose of KRX-0502 (ferric citrate) of 3 caplets per day (approximately 3 grams of ferric citrate as approximately 630 mg of ferric iron) or placebo (approximately 3 matching caplets per day). The target level for serum phosphorus is 3.0 to 4.0 mg/dL. Subjects are titrated as follows:
1. If serum phosphorus is at target (3.0 to 4.0 mg/dL), no adjustment in dose is required.
2. If serum phosphorus is < 3.0 mg/dL, the dose of KRX-0502 (ferric citrate) or placebo is decreased by 1 caplet per day and the subject's serum phosphorus is re-checked within seven days.
3. If the serum phosphorus is > 4.0 mg/dL, the dose of KRX-0502 (ferric citrate) or placebo is increased by 1 caplet per day and the subject's serum phosphorus is re-checked within seven days.

The maximum number of KRX-0502 (ferric citrate) or placebo caplets per day is 12, or 12 g/day of ferric citrate. If a subject has a serum phosphorus ≥ 6.0 mg/dL for at least two visits in a row during the 12-week treatment period, the subject is considered a treatment failure, stops study drug and exits the study.

If a subject's Hgb is < 9.0 g/dL during the two-week washout, the subject is a screen failure. If a subject's Hgb is < 9.0 g/dL for at least two visits in a row during the 12-week treatment period, the subject is considered a treatment failure, stops study drug and exits the study.

Subjects take KRX-0502 (ferric citrate) or placebo orally with meals or snacks or within one hour after their meals or snacks. Subjects are instructed not to take KRX-0502 (ferric citrate) or placebo if greater than one hour has passed since the ingestion of their meals or snacks.

### Statistical Considerations: Efficacy

Change in serum phosphorus, ferritin and TSAT levels from baseline to end of treatment after 12 weeks are the primary endpoints.

This study demonstrates that KRX-0502 (ferric citrate) is statistically superior to placebo in managing serum phosphorus and iron deficiency in anemic Stage III to V CKD subjects, not on dialysis, requiring phosphate binders from baseline (Visit 4, Week 0) to endpoint (Visit 11, Week 12).

Change in calcium x phosphorus product, serum calcium, estimated glomerular filtration rate (eGFR), urinary phosphorus, bicarbonate levels, serum iron, UIBC, TIBC, iPTH, and intact fibroblast growth factor 23 (FGF23) from baseline (Visit 4, Week 0) to the end of treatment (Visit 11, Week 12) are also assessed as secondary endpoints.

### Statistical Considerations: Sample Size

Up to approximately 200 subjects are screened to randomize approximately 140 subjects. Eligible subjects are randomized in a 1:1 ratio to either KRX-0502 (ferric citrate) or placebo. There are approximately 70 subjects randomized per treatment arm. The dropout rate during the two-week washout and 12-week treatment periods is approximately 20% and therefore approximately 110 subjects complete 12 weeks of treatment with study drug (KRX-0502 (ferric citrate) or placebo). There are approximately 55 subjects completing 12 weeks of treatment with study drug (KRX-0502 (ferric citrate) or placebo).

The ending serum phosphorus at Visit 11 (Week 12) is approximately 4.3 mg/dL in the KRX-0502 (ferric citrate) group and 4.6 mg/dL in the placebo-treated group. The common standard deviation is approximately 0.5 mg/dL. Based on these parameters, the trial has at least 80% power to detect a difference between the two groups (alpha = 0.05, two sided).

The ending ferritin level at Visit 11 (Week 12) is approximately 300 ng/mL in the KRX-0502 (ferric citrate) group and 150 ng/mL in the placebo-treated group. The common standard deviation is approximately 75 ng/mL. Based on these parameters, the trial has at least 80% power to detect a difference between the two groups (alpha = 0.05, two sided).

The ending TSAT level at Visit 11 (Week 12) is approximately 25% in the KRX-0502 (ferric citrate) group and 17% in the placebo-treated group. The common standard deviation is approximately 5%. Based on these parameters, the trial has at least 80% power to detect a difference between the two groups (alpha = 0.05, two sided).

## Claims

1. A ferric citrate tablet for use in a method for increasing or maintaining the hemoglobin concentration in a non-dialysis chronic kidney disease (CKD) human patient with anemia, wherein the method comprises orally administering one or more tablets to the patient per day, wherein each tablet comprises 1 g of ferric citrate.

2. The ferric citrate tablet for use according to claim 1, wherein the administration of the one or more ferric citrate tablets results in a mean increase in hemoglobin concentration of greater than 0.4 g/dL in the patient.

3. The ferric citrate tablet for use according to claim 1 or 2, wherein the need for intravenous iron supplementation is reduced in the patient.

4. The ferric citrate tablet for use according to claim 1 or 2, wherein the need for erythropoiesis-stimulating agents is reduced in the patient.

5. The ferric citrate tablet for use according to any one of claims 1 to 4, wherein one to twelve ferric citrate tablets are administered to the patient per day.

6. The ferric citrate tablet for use according to any one of claims 1 to 5, wherein each tablet comprises approximately 80% to approximately 90% by weight of ferric citrate, approximately 70% to approximately 92% by weight of ferric citrate, approximately 80% to approximately 92% by weight of ferric citrate, approximately 85% to approximately 92% by weight of ferric citrate, or approximately 90% to approximately 92% by weight of ferric citrate.

7. The ferric citrate tablet for use according to any one of claims 1 to 6, wherein each tablet comprises approximately 4.5% to approximately 30% by weight of pregelatinized starch.

8. The ferric citrate tablet for use according to any one of claims 1 to 7, wherein each tablet comprises approximately 0.5% to approximately 3% by weight of a lubricant.

9. The ferric citrate tablet for use according to claim 8, wherein the lubricant is calcium stearate.

10. The ferric citrate tablet for use according to any one of claims 1 to 9, wherein the water content measured by LOD % is less than 10% water w/w.

11. The ferric citrate tablet for use according to any one of claims 1 to 10, wherein at least 80% of the ferric citrate is dissolved in less than or equal to 60 minutes as measured by test method USP <711>.

12. The ferric citrate tablet for use according to any one of claims 1 to 11, wherein each tablet consists of
a) a core consisting of 80.0 to 90.0 % w/w ferric citrate, 8.0 to 15.0 % w/w pregelatinized starch, and 1.0 to 3.0 % w/w calcium stearate; and
b) a coating.

13. The ferric citrate tablet for use according to any one of claims 1 to 12, wherein the administration of the one or more ferric citrate tablets per day results in a mean increase in hemoglobin concentration of greater than 0.5 g/dL or of 0.4 to 1 g/dL.

14. The ferric citrate tablet for use according to any one of claims 1 to 13, wherein the ferric citrate is a complex of iron(III) and citric acid with a molar ratio of iron (III) to citric acid from 1:0.69 to 1:0.87.

15. The ferric citrate tablet for use according to any one of claims 1 to 14, wherein the anemia is iron deficiency anemia.

## Patentansprüche

1. Eisencitrattablette zur Verwendung in einem Verfahren, um die Hämoglobinkonzentration in einem menschlichen Patienten mit nichtdialytischer chronischer Nierenerkrankung (CKD) mit Anämie zu erhöhen oder aufrechtzuerhalten, wobei das Verfahren orale Verabreichung einer oder mehrerer Tabletten an den Patienten pro Tag umfasst, wobei jede Tablette 1 g Eisencitrat umfasst.

2. Eisencitrattablette zur Verwendung nach Anspruch 1, wobei die Verabreichung der einen oder mehreren Eisencitrattabletten in einer mittleren Erhöhung der Hämoglobinkonzentration von mehr als 0,4g/dL bei dem Patienten resultiert.

3. Eisencitrattablette zur Verwendung nach Anspruch 1 oder 2, wobei der Bedarf an intravenöser Eisensupplementation bei dem Patienten reduziert ist.

4. Eisencitrattablette zur Verwendung nach Anspruch 1 oder 2, wobei der Bedarf an Erythropoiese-stimulierenden Mitteln bei dem Patienten reduziert ist.

5. Eisencitrattablette zur Verwendung nach einem der Ansprüche 1 bis 4, wobei eine bis zwölf Eisencitrattabletten an den Patienten pro Tag verabreicht werden.

6. Eisencitrattablette zur Verwendung nach einem der Ansprüche 1 bis 5, wobei jede Tablette etwa 80 bis etwa 90 Gew.-% Eisencitrat, etwa 70 bis etwa 92 Gew.-% Eisencitrat, etwa 80% bis etwa 92 Gew.% Eisencitrat, etwa 85 bis etwa 92 Gew.-% Eisencitrat oder etwa 90 bis etwa 92 Gew.-% Eisencitrat umfasst.

7. Eisencitrattablette zur Verwendung nach einem der Ansprüche 1 bis 6, wobei jede Tablette etwa 4,5 bis etwa 30 Gew.-% vorgelatinierte Stärke umfasst.

8. Eisencitrattablette zur Verwendung nach einem der Ansprüche 1 bis 7, wobei jede Tablette etwa 0,5 bis etwa 3 Gew.-% Gleitmittel umfasst.

9. Eisencitrattablette zur Verwendung nach Anspruch 8, wobei das Gleitmittel Kalziumstearat ist.

10. Eisencitrattablette zur Verwendung nach einem der Ansprüche 1 bis 9, wobei der Wassergehalt nach LOD % gemessen weniger als 10% Wasser w/w ist.

11. Eisencitrattablette zur Verwendung nach einem der Ansprüche 1 bis 10, wobei mindestens 80% des Eisencitrats in weniger als oder gleich 60 Minuten, nach Testverfahren USP <711> gemessen, aufgelöst sind.

12. Eisencitrattablette zur Verwendung nach einem der Ansprüche 1 bis 11, wobei jede Tablette besteht aus
a) einem Kern, der aus 80,0 bis 90,0% w/w Eisencitrat, 8,0 bis 15,0% w/w vorgelatinierter Stärke und 1,0 bis 3,0% w/w Kalziumstearat besteht; und
b) einer Beschichtung.

13. Eisencitrattablette zur Verwendung nach einem der Ansprüche 1 bis 12, wobei die Verabreichung der einen oder mehreren Eisencitrattabletten pro Tag in einer mittleren Erhöhung der Hämoglobinkonzentration von mehr als 0,5g/dL oder von 0,4 bis 1g/dL resultiert.

14. Eisencitrattablette zur Verwendung nach einem der Ansprüche 1 bis 13, wobei das Eisencitrat ein Komplex von Eisen (III) und Zitronensäure mit einem Molverhältnis von Eisen (III) zu Zitronensäure von 1:0,69 bis 1:0,87 ist.

15. Eisencitrattablette zur Verwendung nach einem der Ansprüche 1 bis 14, wobei die Anämie Eisenmangelanämie ist.

## Revendications

1. Comprimé de citrate ferrique pour une utilisation dans un procédé pour augmenter ou maintenir la concentration en hémoglobine chez un patient humain souffrant d'une maladie rénale chronique (CKD) sans dialyse atteint d'anémie, dans lequel le procédé comprend l'administration orale d'un ou plusieurs comprimés au patient par jour, dans lequel chaque comprimé comprend 1 g de citrate ferrique.

2. Comprimé de citrate ferrique pour une utilisation selon la revendication 1, dans lequel l'administration des un ou plusieurs comprimés de citrate ferrique a pour résultat une augmentation moyenne de concentration en hémoglobine de plus de 0,4 g/dL chez le patient.

3. Comprimé de citrate ferrique pour une utilisation selon la revendication 1 ou 2, dans lequel le besoin en complément de fer par voie intraveineuse est réduit chez le patient.

4. Comprimé de citrate ferrique pour une utilisation selon la revendication 1 ou 2, dans lequel le besoin en agents stimulant l'érythropoïèse est réduit chez le patient.

5. Comprimé de citrate ferrique pour une utilisation selon l'une quelconque des revendications 1 à 4, dans lequel un à douze comprimés de citrate ferrique sont administrés au patient par jour.

6. Comprimé de citrate ferrique pour une utilisation selon l'une quelconque des revendications 1 à 5, dans lequel chaque comprimé comprend approximativement 80 % à approximativement 90 % en poids de citrate ferrique, approximativement 70 % à approximativement 92 % en poids de citrate ferrique, approximativement 80 % à approximativement 92 % en poids de citrate ferrique, approximativement 85 % à approximativement 92 % en poids de citrate ferrique, ou approximativement 90 % à approximativement 92 % en poids de citrate ferrique.

7. Comprimé de citrate ferrique pour utilisation selon l'une quelconque des revendications 1 à 6, dans lequel chaque comprimé comprend approximativement 4,5 % à approximativement 30 % en poids d'amidon prégélatinisé.

8. Comprimé de citrate ferrique pour une utilisation selon l'une quelconque des revendications 1 à 7, dans lequel chaque comprimé comprend approximativement 0,5 % à approximativement 3 % en poids d'un lubrifiant.

9. Comprimé de citrate ferrique pour une utilisation selon la revendication 8, dans lequel le lubrifiant est du stéarate de calcium.

10. Comprimé de citrate ferrique pour une utilisation selon l'une quelconque des revendications 1 à 9, dans lequel la teneur en eau mesurée par la LOD en % est inférieure à 10 % d'eau p/p.

11. Comprimé de citrate ferrique pour une utilisation selon l'une quelconque des revendications 1 à 10, dans lequel au moins 80 % du citrate ferrique est dissous en moins de 60 minutes, ou en 60 minutes, comme mesuré par le procédé d'essai USP <711>.

12. Comprimé de citrate ferrique pour une utilisation selon l'une quelconque des revendications 1 à 11, dans lequel chaque comprimé consiste en
a) un noyau constitué de 80,0 à 90,0 % p/p de citrate ferrique, de 8,0 à 15,0 % p/p d'amidon prégélatinisé, et de 1,0 à 3,0 % p/p de stéarate de calcium ; et
b) un revêtement.

13. Comprimé de citrate ferrique pour une utilisation selon l'une quelconque des revendications 1 à 12, dans lequel l'administration des un ou plusieurs comprimés de citrate ferrique par jour a pour résultat une augmentation moyenne de concentration en hémoglobine de plus de 0,5 g/dL ou de 0,4 à 1 g/dL.

14. Comprimé de citrate ferrique pour une utilisation selon l'une quelconque des revendications 1 à 13, dans lequel le citrate ferrique est un complexe de fer (III) et d'acide citrique avec un rapport molaire entre le fer (III) et l'acide citrique de 1:0,69 à 1:0,87.

15. Comprimé de citrate ferrique pour une utilisation selon l'une quelconque des revendications 1 à 14, dans lequel l'anémie est une anémie ferriprive.
